# EUROPEAN PATENT APPLICATION

(11) **EP 3 978 498 A1**
(43) Date of publication of application: **06.04.2022**
(21) Application number: 20813761.2
(22) Date of filing: 22.05.2020
(51) Int. Cl.: C07D 487/04, A61P 35/00, A61K 31/4985

(54) **SUBSTITUTED FUSED BICYCLIC DERIVATIVE, PREPARATION METHOD THEREFOR, AND APPLICATION THEREOF IN MEDICINES**

(30) Priority: 24.05.2019 CN 201910437300
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); WANG, Bin, Shanghai 200245 (CN); BAI, Dongdong, Shanghai 200245 (CN); HE, Feng, Shanghai 200245 (CN); TAO, Weikang, Shanghai 200245 (CN)
(74) Representative: Potter Clarkson
(86) International application number: PCT/CN2020/091695
(87) International publication number: WO 2020/238776

(57) **Abstract**

The present application relates to a substituted fused bicyclic derivative, a preparation method therefor, and an application thereof in medicines. Specifically, the present application relates to a novel substituted fused bicyclic derivative as represented by formula (I), a preparation method therefor, a pharmaceutical composition containing the derivative, an application thereof as a therapeutic agent, in particular, as an ERK inhibitor, and an application thereof in preparation of medicines for treatment and/or prevention of cancer, inflammation, or other proliferative diseases, wherein the definitions of the substituents in formula (I) are the same as those in the description.

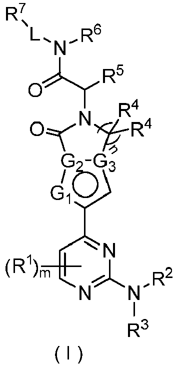

## Description

### TECHNICAL FIELD

The present disclosure belongs to the field of medicine, and relates to a substituted fused bicyclic derivative, preparation method therefor, and application thereof in medicines. In particular, the present disclosure relates to a substituted fused bicyclic derivative of formula (I), a method for preparing the same, a pharmaceutical composition comprising the same, a use thereof as an ERK inhibitor for treating ERK-mediated diseases and disorders or inhibiting the MAPK-ERK signaling pathway.

### BACKGROUND

The proliferation, differentiation, metabolism, and apoptosis of normal cells are strictly regulated by cell signal transduction pathways in the body. Mitogen-activated protein kinase (MAPK) plays an extremely important role in the signal transduction pathways, and extracellular signal regulated kinase (ERK) is a member of the MAPK family. Through the RAS-RAF-MEK-ERK step, the exogenous stimulating signal is transmitted to the ERK, and the activated ERK transfers to the nucleus to regulate the activity of transcription factors, thereby regulating the biological functions such as cell proliferation, differentiation and apoptosis, or to involve in the regulation of cell morphology and the redistribution of cytoskeleton by phosphorylation of cytoskeletal components in the cytoplasm.

Gene mutations in RAS and RAF cause the continuous activation of the MAPK-ERK signaling pathway, which promotes malignant transformation and abnormal proliferation of cells, and eventually produces tumors (Roberts PJ et al., Oncogene, 2007, 26(22), 3291-3310). The combination of a MEK inhibitor and a B-RAF inhibitor can further enhance the effect of the B-RAF inhibitor on inhibiting tumor growth, and can significantly improve the disease-free progression and overall survival rate of melanoma patients with BRAFV600E and V600K mutations (Frederick DT et al., Clinical Cancer Research, 2013.19(5), 1225-1231). Although the combination of B-RAF/MEK inhibitors can inhibit tumors, their efficacy is short-lived. Within 2-18 months, most patients will develop drug resistance and tumors will further deteriorate. The mechanism of resistance to B-RAF/MEK inhibitors is very complex, and is mostly directly related to the reactivation of the ERK signaling pathway (Smalley I et al., Cancer Discovery, 2018, 8(2), 140-142). Therefore, the development of new ERK inhibitors is not only effective for patients with mutations in the MAPK signaling pathway, but also for patients with resistance to B-RAF/MEK inhibitors.

B-RAF/MEK inhibitors not only inhibit tumor growth, but also regulate the immune microenvironment of tumors. B-RAF/MEK inhibitors can enhance the expression of tumor-specific antigens, improve the recognition and killing of tumors by antigen-specific T cells, and promote the migration and infiltration of immune cells. In animal models, after treatment with B-RAF/MEK inhibitors, the expression of PD-L1 in tumor tissues is enhanced. When combined with checkpoint molecule antibodies (such as PD-1 antibody, CTLA4 antibody), it is more effective in inhibiting tumor growth than B-RAF/MEK inhibitors alone (Boni A et al., Cancer Research, 2010, 70(13), 5213-5219). Studies have shown that ERK inhibitors are similar to B-RAF/MEK inhibitors, and their combination with checkpoint antibodies can regulate the tumor microenvironment, improve the function of cytotoxic T cells, and achieve the effect of inhibiting tumor growth.

A number of ERK inhibitor compounds have been developed. Among them, BVD-523 developed by BioMed Valley Discoveries is in the phase II clinical trial, MK-8353 developed by Merck and Astex-029 developed by Astex are in the phase I clinical trial. Related patent applications include WO1999061440A1, WO2001056557A2, WO2001056993A2, WO2001057022A2, WO2002022601A1, WO2012118850A1, WO2013018733A1, WO2014179154A2, WO2015103133A1, WO2016192063A1, WO2017180817A1 and WO2018049127A1.

### SUMMARY OF THE INVENTION

The object of the present disclosure is to provide a compound of formula (I) or a tautomer, mesomer, racemate, enantiomer, diastereomer thereof, or mixture thereof, or a pharmaceutically acceptable salt thereof: 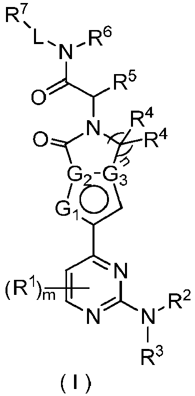 wherein:
G₁, G₂ and G₃ are identical or different and are each independently selected from the group consisting of CH, C and N;
L is a bond or an alkylene, wherein the alkylene is optionally further substituted by one or more substituents selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
each R¹ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
each R⁴ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁵ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁶ is selected from the group consisting of hydrogen atom, halogen, alkyl, alkoxy, haloalkyl, haloalkoxy, hydroxy and hydroxyalkyl;
R⁷ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, haloalkyl, haloalkoxy and aminoalkyl;
n is 1, 2 or 3; and
m is 0, 1 or 2.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (1-1) or (1-2) or a stereisomer, tautomer, mesomer, racemate, enantiomer, diastereomer thereof, or mixture thereof, or a pharmaceutically acceptable salt thereof: 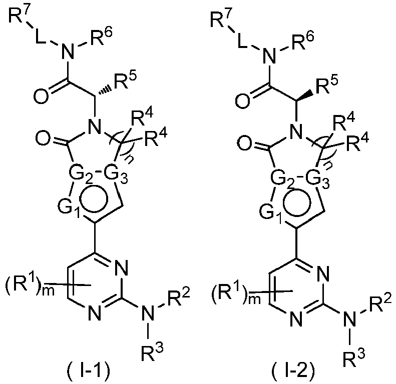 wherein: G₁, G₂, G₃, L, m, n, and R¹ to R⁷ are as defined in the compound of formula (I); preferably, R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; and G₁, G₂, G₃, L, m, n, R¹ to R⁴, R⁶ and R⁷ are as defined in the compound of formula (I).

In some embodiments of the present disclosure, in the compound of formula (I) or the tautomer, mesomer, racemate, enantiomer, diastereomer thereof, or mixture thereof, or the pharmaceutically acceptable salt thereof, n is 1 or 2.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (II-1), (II-2), (II-3) or (II-4) or a stereisomer, tautomer, mesomer, racemate, enantiomer, diastereomer thereof, or mixture thereof, or a pharmaceutically acceptable salt thereof: 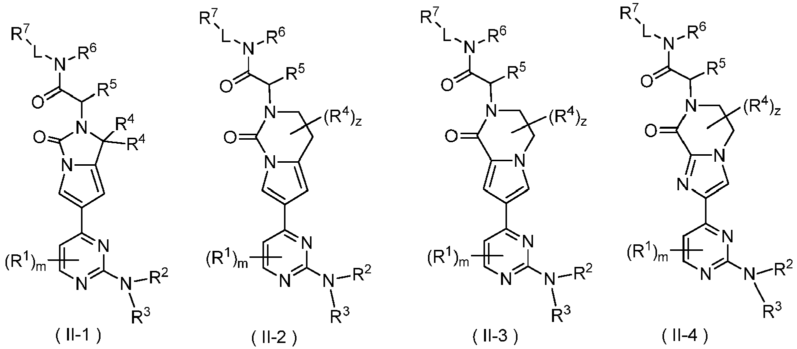 wherein:
z is 0, 1, 2, 3 or 4; and L, m and R¹ to R⁷ are as defined in the compound of formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (II-11), (II-21), (II-31) or (11-41) or a stereisomer, tautomer, mesomer, racemate, enantiomer, diastereomer thereof, or mixture thereof, or a pharmaceutically acceptable salt thereof: 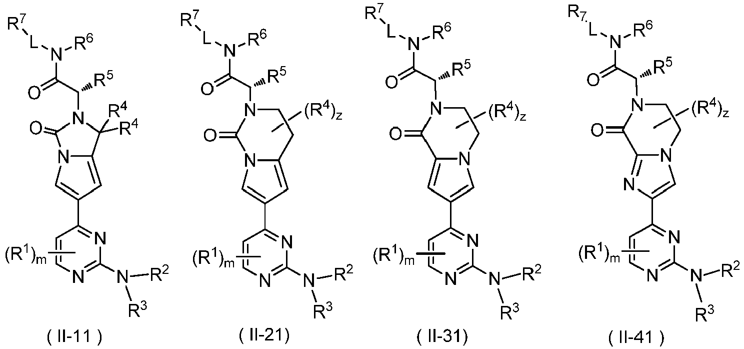 wherein:
z is 0, 1, 2, 3 or 4; and L, m and R¹ to R⁷ are as defined in the compound of formula (I); preferably, R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; z is 0, 1, 2, 3 or 4; and L, m, R¹ to R⁴, R⁶ and R⁷ are as defined in the compound of formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (II-12), (II-22), (II-32) or (11-42) or a stereisomer, tautomer, mesomer, racemate, enantiomer, diastereomer thereof, or mixture thereof, or a pharmaceutically acceptable salt thereof: 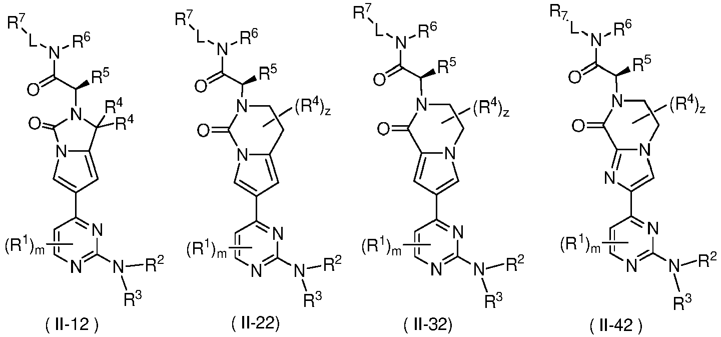 wherein:
z is 0, 1, 2, 3 or 4; and L, m and R¹ to R⁷ are as defined in the compound of formula (I); preferably, R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; z is 0, 1, 2, 3 or 4; and L, m, R¹ to R⁴, R⁶ and R⁷ are as defined in the compound of formula (I).

In some embodiments of the present disclosure, in the compound of formula (I) or the stereisomer, tautomer, mesomer, racemate, enantiomer, diastereomer thereof, or mixture thereof, or the pharmaceutically acceptable salt thereof, L is an alkylene, wherein the alkylene is optionally further substituted by one or more substituents selected from the group consisting of alkyl, alkoxy, oxo, halogen, amino, cyano, nitro, hydroxy, hydroxyalkyl and aminoalkyl, preferably, L is -CH(R⁸)-; R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, L is an alkylene, wherein the alkylene is optionally further substituted by one or more substituents selected from the group consisting of alkyl, alkoxy, oxo, halogen, amino, cyano, nitro, hydroxy, hydroxyalkyl and aminoalkyl, preferably, further substituted by hydroxyalkyl.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (III-1), (III-2), (III-3) or (III-4) or a stereisomer, tautomer, mesomer, racemate, enantiomer, diastereomer thereof, or mixture thereof, or a pharmaceutically acceptable salt thereof: 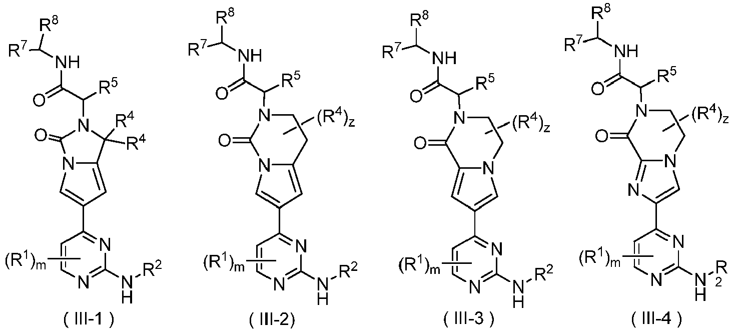 wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
z is 0, 1, 2, 3 or 4; and
R¹, R², R⁴, R⁵, R⁷ and m are as defined in the compound of formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (III-11), (III-21), (III-31) or (III-41) or a stereisomer, tautomer, mesomer, racemate, enantiomer, diastereomer thereof, or mixture thereof, or a pharmaceutically acceptable salt thereof: 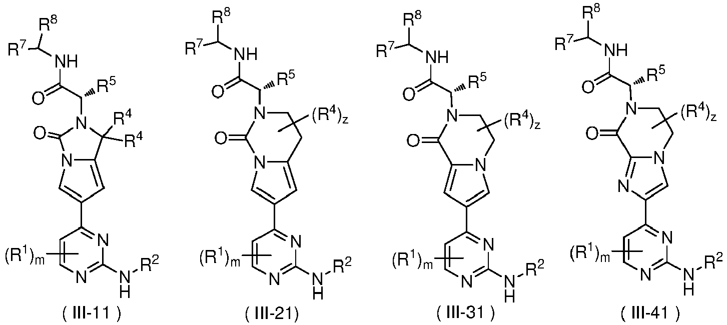 wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
z is 0, 1, 2, 3 or 4; and
R¹, R², R⁴, R⁵, R⁷ and m are as defined in the compound of formula (I); preferably, R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; z is 0, 1, 2, 3 or 4; and R¹, R², R⁴, R⁷ and m are as defined in the compound of formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (III-12), (III-22), (III-32) or (III-42) or a stereisomer, tautomer, mesomer, racemate, enantiomer, diastereomer thereof, or mixture thereof, or a pharmaceutically acceptable salt thereof: 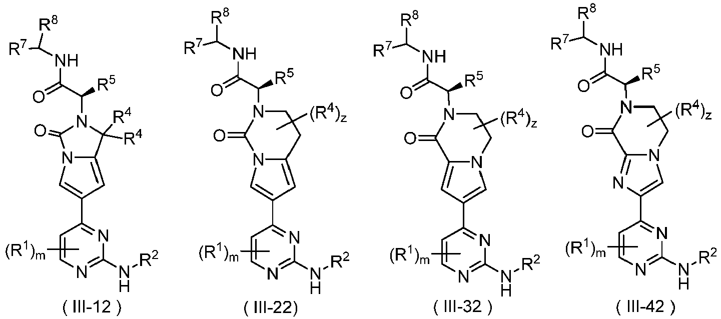 wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
z is 0, 1, 2, 3 or 4; and
R¹, R², R⁴, R⁵, R⁷ and m are as defined in the compound of formula (I); preferably, R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; z is 0, 1, 2, 3 or 4; and R¹, R², R⁴, R⁷ and m are as defined in the compound of formula (I).

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁷ is an aryl or heteroaryl, wherein the aryl and heteroaryl are each optionally further substituted by one or more substituents selected from the group consisting of alkyl, alkoxy, oxo, halogen, amino, cyano, nitro, hydroxy and hydroxyalkyl; preferably, R⁷ is an aryl, wherein the aryl is optionally further substituted by one or more substituents selected from the group consisting of alkyl, alkoxy, oxo, halogen, amino, cyano, nitro, hydroxy and hydroxyalkyl; more preferably, R⁷ is a phenyl, wherein the phenyl is optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁷ is an aryl, wherein the aryl is optionally further substituted by one or more substituents selected from the group consisting of alkyl, alkoxy, oxo, halogen, amino, cyano, nitro, hydroxy and hydroxyalkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁸ is selected from the group consisting of alkyl, alkoxy, oxo, halogen, amino, cyano, nitro, hydroxy, hydroxyalkyl and aminoalkyl; preferably hydroxyalkyl and aminoalkyl; and more preferably C₁₋₆ hydroxyalkyl or aminoC₁₋₆ alkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁵ is selected from the group consisting of hydrogen atom, halogen, alkyl, alkoxy, haloalkyl, haloalkoxy, hydroxy and hydroxyalkyl, preferably selected from the group consisting of hydrogen atom, halogen and alkyl, more preferably C₁₋₆ alkyl, and most preferably methyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁵ is selected from the group consisting of halogen, alkyl, alkoxy, haloalkyl, haloalkoxy, hydroxy and hydroxyalkyl, preferably selected from the group consisting of halogen and alkyl, more preferably C₁₋₆ alkyl, and most preferably methyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R² is selected from the group consisting of C₁₋₆ alkyl, 3 to 8 membered heterocyclyl and 5 to 10 membered heteroaryl, wherein the C₁₋₆ alkyl, 3 to 8 membered heterocyclyl and 5 to 10 membered heteroaryl are each optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R² is a heterocyclyl, wherein the heterocyclyl is optionally further substituted by one or more substituents selected from the group consisting of alkyl, alkoxy, oxo, halogen, amino, cyano, nitro, hydroxy and hydroxyalkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R² is a heteroaryl, wherein the heteroaryl is optionally further substituted by one or more substituents selected from the group consisting of alkyl, alkoxy, oxo, halogen, amino, cyano, nitro, hydroxy and hydroxyalkyl.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R¹ is selected from the group consisting of hydrogen atom, halogen, alkyl, alkoxy, haloalkyl, haloalkoxy, hydroxy and hydroxyalkyl, preferably selected from the group consisting of hydrogen atom, halogen and alkyl, more preferably selected from the group consisting of hydrogen atom, halogen and C₁₋₆ alkyl, and most preferably halogen.

In some embodiments of the present disclosure, in the compound of formula (I) or the stereisomer, tautomer, mesomer, racemate, enantiomer, diastereomer thereof, or mixture thereof, or the pharmaceutically acceptable salt thereof, R⁴ is selected from the group consisting of hydrogen atom, halogen, alkyl, alkoxy, haloalkyl, haloalkoxy, hydroxy and hydroxyalkyl, preferably hydrogen atom or alkyl, and more preferably hydrogen atom or C₁₋₆ alkyl.

Typical compounds of formula (I) include, but are not limited to:

| Example No. | Structure and name of the compound |
|---|---|
| 1-P1/1-P2 | 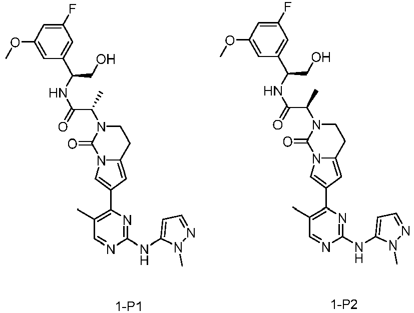 |
| | (*S*)*-N-*((*S*)-1-(3-Fluoro-5-methoxyphenyl)-2-hydroxyethyl)-2-(6-(5-methy 1-2-((1-methyl-1*H*-pyrazol-5-yl)amino)pyrimidin-4-yl)-1-oxo-3,4-dihydro pyrrolo[1,2-*c*]pyrimidin-2(1*H*)-yl)propanamide **1-P1** (*R*)-*N*-((*S*)-1-(3-Fluoro-5-methoxyphenyl)-2-hydroxyethyl)-2-(6-(5-methy 1-2-((1-methyl-1*H*-pyrazol-5-yl)amino)pyrimidin-4-yl)-1-oxo-3,4-dihydro pyrrolo[1,2-*c*]pyrimidin-2(1*H*)-yl)propanamide **1-P2** |
| 2-P1/2-P2 | 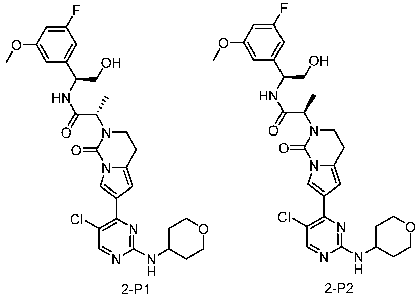 |
| | (*S*)-2-(6-(5-Chloro-2-((tetrahydro-2*H*-pyran-4-yl)amino)pyrimidin-4-yl)-1 -oxo-3,4-dihydropyrrolo[1,2-*c*]pyrimidin-2(1*H*)-yl)-*N*-((*S*)-1-(3-fluoro-5-methoxyphenyl)-2-hydroxyethyl)propanamide **2-P1** (*R*)-2-(6-(5-Chloro-2-((tetrahydro-2*H*-pyran-4-yl)amino)pyrimidin-4-yl)-1-oxo-3,4-dihydropyrrolo[1,2-*c*]pyrimidin-2(1*H*)-yl)-*N*-((*S*)-1-(3-fluoro-5 -methoxyphenyl)-2-hydroxyethyl)propanamide **2-P2** |
| 3-P1 3-P2 | 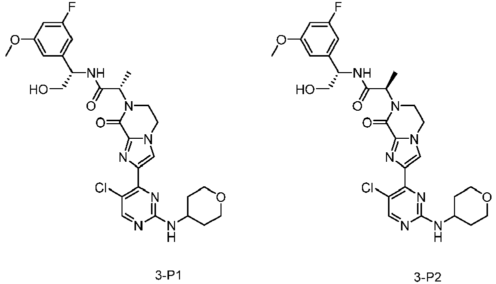 |
| | (*S*)-2-(2-(5-Chloro-2-((tetrahydro-2*H*-pyran-4-yl)amino)pyrimidin-4-yl)-8 -oxo-5,6-dihydroimidazo[1,2-*a*]pyrazin-7(8*H*)-yl)-*N*-((*S*)-1-(3-fluoro-5-m ethoxyphenyl)-2-hydroxyethyl)propanamide **3-P1** (*R*)-2-(2-(5-Chloro-2-((tetrahydro-2*H*-pyran-4-yl)amino)pyrimidin-4-yl)-8-oxo-5,6-dihydroimidazo[1,2-*a*]pyrazin-7(8*H*)-yl)-*N*-((*S*)-1-(3-fluoro-5-methoxyphenyl)-2-hydroxyethyl)propanamide **3-P2** |
| 4-P1/4-P2 | 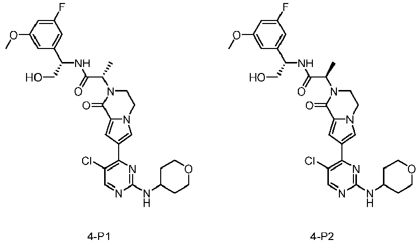 |
| | (*S*)-2-(7-(5-Chloro-2-((tetrahydro-2*H*-pyran-4-yl)amino)pyrimidin-4-yl)-1 -oxo-3,4-dihydropyrrolo[1,2-*a*]pyrazin-2(1*H*)-yl)-*N*-((*S*)-1-(3-fluoro-5-me thoxyphenyl)-2-hydroxyethyl)propanamide **4-P1** (*R*)-2-(7-(5-Chloro-2-((tetrahydro-2*H*-pyran-4-yl)amino)pyrimidin-4-yl)-1-oxo-3,4-dihydropyrrolo[1,2-*a*]pyrazin-2(1*H*)-yl)-*N*-((*S*)-1-(3-fluoro-5-m ethoxyphenyl)-2-hydroxyethyl)propanamide **4-P2** |
| 5 | 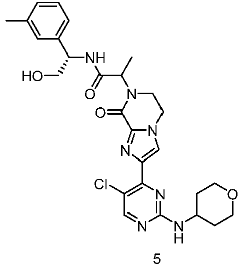 |
| | 2-(2-(5-Chloro-2-((tetrahydro-2*H*-pyran-4-yl)amino)pyrimidin-4-yl)-8-ox o-5,6-dihydroimidazo[1,2-*a*]pyrazin-7(8*H*)-yl)-*N*-((*S*)-2-hydroxy-1-(*m*-tol yl)ethyl)propanamide **5** |
| 6-P1/6-P2 | 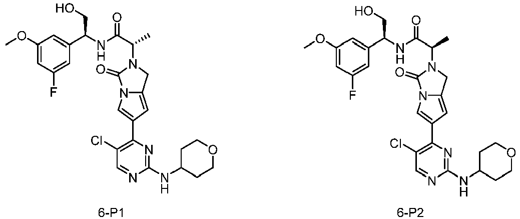 |
| | (*S*)-2-(6-(5-Chloro-2-((tetrahydro-2*H*-pyran-4-yl)amino)pyrimidin-4-yl)-3 -oxo-1*H*-pyrrolo[1,2-*c*]imidazol-2(3*H*)-yl)-*N*-((*S*)-1-(3-fluoro-5-methoxyp henyl)-2-hydroxyethyl)propanamide **6-P1** (*R*)-2-(6-(5-Chloro-2-((tetrahydro-2*H*-pyran-4-yl)amino)pyrimidin-4-yl)-3-oxo-1*H*-pyrrolo[1,2-*c*]imidazol-2(3*H*)-yl)-*N*-((*S*)-1-(3-fluoro-5-methoxy phenyl)-2-hydroxyethyl)propanamide **6-P2** |
| 7-P1/7-P2 | 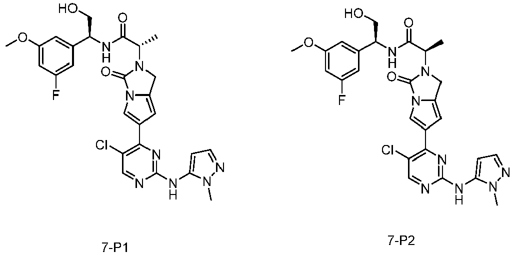 |
| | (*S*)-2-(6-(5-Chloro-2-((1-methyl-1*H*-pyrazol-5-yl)amino)pyrimidin-4-yl)-3-oxo-1*H*-pyrrolo[1,2-*c*]imidazol-2(3*H*)-yl)-*N*-((*S*)-1-(3-fluoro-5-methoxy phenyl)-2-hydroxyethyl)propanamide **7-P1** (*R*)-2-(6-(5-Chloro-2-((1-methyl-1*H*-pyrazol-5-yl)amino)pyrimidin-4-yl)-3-oxo-1*H*-pyrrolo[1,2-*c*]imidazol-2(3*H*)-yl)-*N*-((*S*)-1-(3-fluoro-5-methoxy phenyl)-2-hydroxyethyl)propanamide **7-P2** |
| 8-P1/8-P2 | 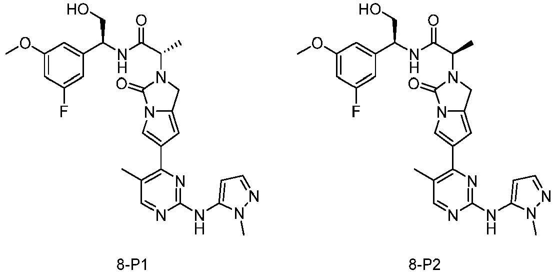 |
| | *(S*)-*N*-((*S*)-1-(3-Fluoro-5-methoxyphenyl)-2-hydroxyethyl)-2-(6-(5-methy 1-2-((1-methyl-1*H*-pyrazol-5-yl)amino)pyrimidin-4-yl)-3-oxo-1*H*-pyrrolo[ 1,2-*c*]imidazol-2(3*H*)-yl)propanamide **8-P1** (*R*)-*N*-((*S*)-1-(3-Fluoro-5-methoxyphenyl)-2-hydroxyethyl)-2-(6-(5-methy 1-2-((1-methyl-1*H*-pyrazol-5-yl)amino)pyrimidin-4-yl)-3-oxo-1*H*-pyrrolo[ 1,2-*c*]imidazol-2(3*H*)-yl)propanamide **8-P2** |
| 9-P1/9-P2 | 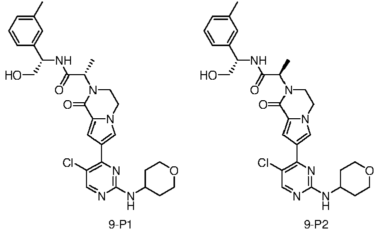 |
| | (*S*)-2-(7-(5-Chloro-2-((tetrahydro-2*H*-pyran-4-yl)amino)pyrimidin-4-yl)-1 -oxo-3,4-dihydropyrrolo[1,2-*a*]pyrazin-2(1*H*)-yl)-*N*-((*S*)-2-hydroxy-1-(*m-*tolyl)ethyl)propanamide **9-P1** (*R*)-2-(7-(5-Chloro-2-((tetrahydro-2*H*-pyran-4-yl)amino)pyrimidin-4-yl)-1-oxo-3,4-dihydropyrrolo[1,2-*a*]pyrazin-2(1*H*)-yl)-*N*-((*S*)-2-hydroxy-1-(m -tolyl)ethyl)propanamide **9-P2** |
| 10-P1/10-P2 | 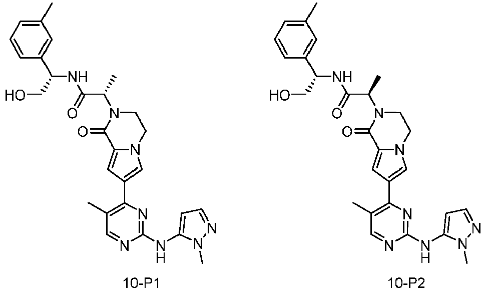 |
| | (*S*)-*N*-((*S*)-2-Hydroxy-1-(*m*-tolyl)ethyl)-2-(7-(5-methyl-2-((1-methyl-1H-pyrazol-5-yl)amino)pyrimidin-4-yl)-1-oxo-3,4-dihydropyrrolo[1,2-*a*]pyra zin-2(1*H*)-yl)propanamide **10-P1** (*R*)-*N*-((*S*)-2-Hydroxy-1-(*m*-tolyl)ethyl)-2-(7-(5-methyl-2-((1-methyl-1*H-*pyrazol-5-yl)amino)pyrimidin-4-yl)-1-oxo-3,4-dihydropyrrolo[1,2-*a*]pyra zin-2(1*H*)-yl)propanamide **10-P2** |
| 11-P1/11-P2 | 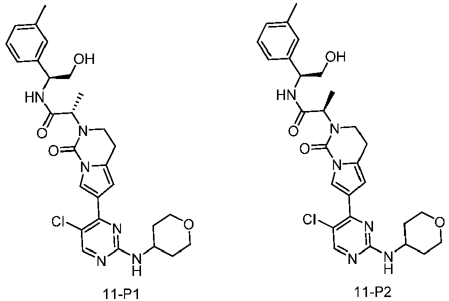 |
| | (*S*)-2-(6-(5-Chloro-2-((tetrahydro-2*H*-pyran-4-yl)amino)pyrimidin-4-yl)-1 -oxo-3,4-dihydropyrrolo[1,2-*c*]pyrimidin-2(1*H*)-yl)-*N*-((*S*)-2-hydroxy-1-( *m*-tolyl)ethyl)propanamide **11-P1** (*R*)-2-(6-(5-Chloro-2-((tetrahydro-2*H*-pyran-4-yl)amino)pyrimidin-4-yl)-1-oxo-3,4-dihydropyrrolo[1,2-*c*]pyrimidin-2(1*H*)-yl)-*N*-((*S*)-2-hydroxy-1-(*m*-tolyl)ethyl)propanamide **11-P2** |
| 12-P1/12-P2 | 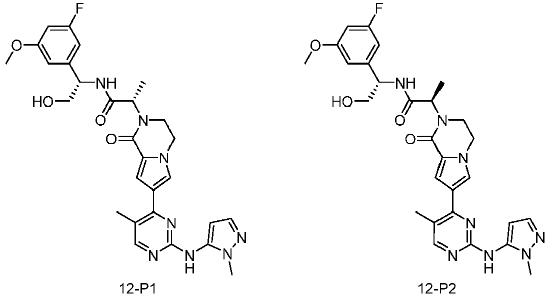 |
| | (*S*)-*N*-((*S*)-1-(3-Fluoro-5-methoxyphenyl)-2-hydroxyethyl)-2-(7-(5-methy 1-2-((1-methyl-1*H*-pyrazol-5-yl)amino)pyrimidin-4-yl)-1-oxo-3,4-dihydro pyrrolo[1,2-*a*]pyrazin-2(1*H*)-yl)propanamide **12-P1** |
| | (*R*)-*N*-((*S*)-1-(3-Fluoro-5-methoxyphenyl)-2-hydroxyethyl)-2-(7-(5-methy 1-2-((1-methyl-1*H*-pyrazol-5-yl)amino)pyrimidin-4-yl)-1-oxo-3,4-dihydro pyrrolo[1,2-*a*]pyrazin-2(1*H*)-yl)propanamide **12-P2** |
| 13-P1/13-P2 | 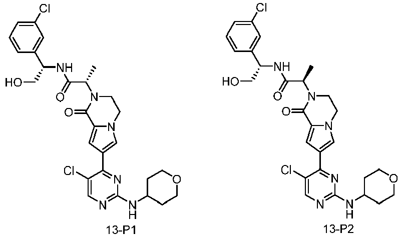 |
| | (*S*)-2-(7-(5-Chloro-2-((tetrahydro-2*H*-pyran-4-yl)amino)pyrimidin-4-yl)-1 -oxo-3,4-dihydropyrrolo[1,2-*a*]pyrazin-2(1*H*)-yl)-*N*-((*S*)-1-(3-chlorophen yl)-2-hydroxyethyl)propanamide **13-P1** (*R*)-2-(7-(5-Chloro-2-((tetrahydro-2*H*-pyran-4-yl)amino)pyrimidin-4-yl)-1-oxo-3,4-dihydropyrrolo[1,2-*a*]pyrazin-2(1*H*)-yl)-*N*-((*S*)-1-(3-chlorophe nyl)-2-hydroxyethyl)propanamide **13-P2** |
| 14-P1/14-P2 | 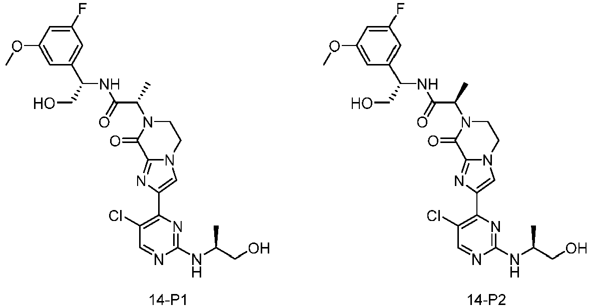 |
| | (*S*)-2-(2-(5-Chloro-2-(((S)-1-hydroxypropan-2-yl)amino)pyrimidin-4-yl)-8-oxo-5,6-dihydroimidazo[1,2-*a*]pyrazin-7(8*H*)-yl)-*N*-((*S*)-1-(3-fluoro-5-methoxyphenyl)-2-hydroxyethyl)propanamide **14-P1** (*R*)-2-(2-(5-Chloro-2-(((*S*)-1-hydroxypropan-2-yl)amino)pyrimidin-4-yl)-8-oxo-5,6-dihydroimidazo[1,2-*a*]pyrazin-7(8*H*)-yl)-*N*-((*S*)-1-(3-fluoro-5-methoxyphenyl)-2-hydroxyethyl)propanamide **14-P2** |
| 15 | 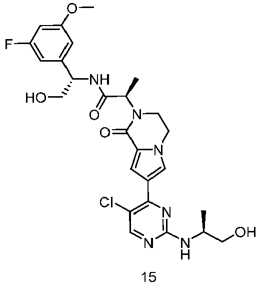 |
| | (*R*)-2-(7-(5-Chloro-2-(((*S*)-1-hydroxypropan-2-yl)amino)pyrimidin-4-yl)-1-oxo-3,4-dihydropyrrolo[1,2-*a*]pyrazin-2(1*H*)-yl)-*N*-((*S*)-1-(3-fluoro-5-methoxyphenyl)-2-hydroxyethyl)propanamide **15** |
| 16-P1/16-P2 | 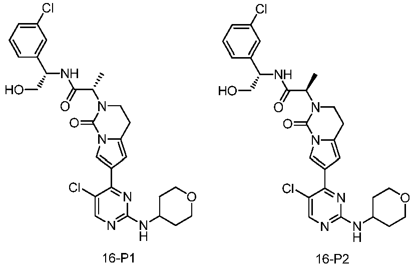 |
| | (*S*)-2-(6-(5-Chloro-2-((tetrahydro-2*H*-pyran-4-yl)amino)pyrimidin-4-yl)-1 -oxo-3,4-dihydropyrrolo[1,2-*c*]pyrimidin-2(1*H*)-yl)-*N*-((*S*)-1-(3-chlorophe nyl)-2-hydroxyethyl)propanamide **16-P1** (*R*)-2-(6-(5-Chloro-2-((tetrahydro-2*H*-pyran-4-yl)amino)pyrimidin-4-yl)-1-oxo-3,4-dihydropyrrolo[1,2-*c*]pyrimidin-2(1*H*)-yl)-*N-*((*S*)-1-(3-chlorop henyl)-2-hydroxyethyl)propanamide **16-P2** |
| 17-P1/17-P2 | 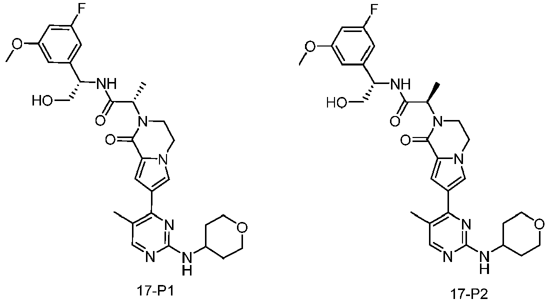 |
| | (*S*)-*N*-((*S*)-1-(3-Fluoro-5-methoxyphenyl)-2-hydroxyethyl)-2-(7-(5-methy 1-2-((tetrahydro-2*H*-pyran-4-yl)amino)pyrimidin-4-yl)-1-oxo-3,4-dihydro pyrrolo[1,2-*a*]pyrazin-2(1*H*)-yl)propanamide **17-P1** (*R*)-*N*-((*S*)-1-(3-Fluoro-5-methoxyphenyl)-2-hydroxyethyl)-2-(7-(5-methy 1-2-((tetrahydro-2*H*-pyran-4-yl)amino)pyrimidin-4-yl)-1-oxo-3,4-dihydro pyrrolo[1,2-*a*]pyrazin-2(1*H*)-yl)propanamide **17-P2** |
| 18-P1/18-P2 | 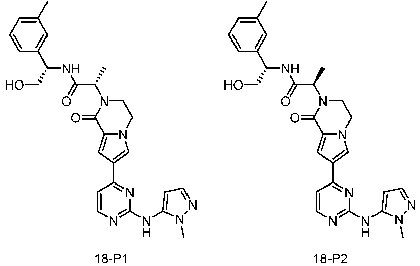 |
| | (*S*)-*N*-((*S*)-2-Hydroxy-1-(*m*-tolyl)ethyl)-2-(7-(2-((1-methyl-1*H*-pyrazol-5-yl)amino)pyrimidin-4-yl)-1-oxo-3,4-dihydropyrrolo[1,2-*a*]pyrazin-2(1*H*)-yl)propanamide **18-P1** (*R*)-*N*-((*S*)-2-Hydroxy-1-(*m*-tolyl)ethyl)-2-(7-(2-((1-methyl-1*H*-pyrazol-5-yl)amino)pyrimidin-4-yl)-1-oxo-3,4-dihydropyrrolo[1,2-*a*]pyrazin-2(1*H*)-yl)propanamide 18-P2 |
| 19-P1/19-P2 | 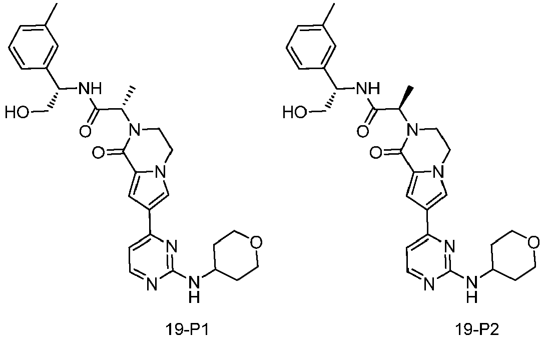 |
| | (*S*)-*N*-((*S*)-2-Hydroxy-1-(*m*-tolyl)ethyl)-2-(1-oxo-7-(2-((tetrahydro-2*H*-py ran-4-yl)amino)pyrimidin-4-yl)-3,4-dihydropyrrolo[1,2-*a*]pyrazin-2(1*H*)-yl)propanamide **19-P1** (*R*)-*N*-((*S*)-2-Hydroxy-1-(*m*-tolyl)ethyl)-2-(1-oxo-7-(2-((tetrahydro-2*H*-py ran-4-yl)amino)pyrimidin-4-yl)-3,4-dihydropyrrolo[1,2-*a*]pyrazin-2(1*H*)-yl)propanamide **19-P2** |

or a tautomer, mesomer, racemate, enantiomer, diastereomer thereof, or mixture thereof, or a pharmaceutically acceptable salt thereof.

In another aspect, the present disclosure relates to a compound of formula (IA) or a tautomer, mesomer, racemate, enantiomer, diastereomer thereof, or mixture thereof, or a pharmaceutically acceptable salt thereof: 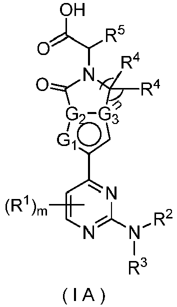 wherein:
G₁, G₂ and G₃ are identical or different and are each independently selected from the group consisting of CH, C and N;
each R¹ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
each R⁴ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁵ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
n is 1, 2 or 3; and
m is 0, 1 or 2.

Typical compounds of formula (I) or the tautomers, mesomers, racemates, enantiomers, diastereomers thereof, or mixtures thereof, or the pharmaceutically acceptable salts thereof include, but are not limited to:

| Example No. | Structure and name of the compound |
|---|---|
| 1l | 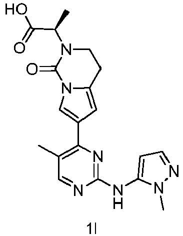 |
| | (*R*)-2-(6-(5-Methyl-2-((1-methyl-1*H*-pyrazol-5-yl)amino)pyrimidin-4-yl)-1-oxo-3,4-dihydropyrrolo[1,2-*c*]pyrimidin-2(1*H*)-yl)propanoic acid 11 |
| 2e | 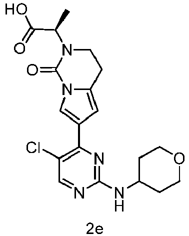 |
| | (*R*)-2-(6-(5-Chloro-2-((tetrahydro-2*H*-pyran-4-yl)amino)pyrimidin-4-yl)-1-oxo-3,4-dihydropyrrolo[1,2-*c*]pyrimidin-2(1*H*)-yl)propanoic acid **2e** |
| 3h | 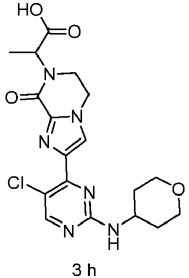 |
| | 2-(2-(5-Chloro-2-((tetrahydro-2*H*-pyran-4-yl)amino)pyrimidin-4-yl)-8-oxo-5,6-dihydroimidazo[1,2-*a*]pyrazin-7(8*H*)-yl)propanoic acid **3h** |
| 4j | 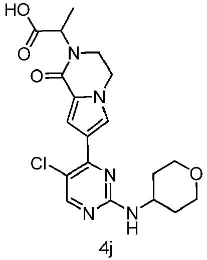 |
| | 2-(7-(5-Chloro-2-((tetrahydro-2*H*-pyran-4-yl)amino)pyrimidin-4-yl)-1-oxo-3,4-dihydropyrrolo[1,2-*a*]pyrazin-2(1*H*)-yl)propanoic acid **4j** |
| 6f | 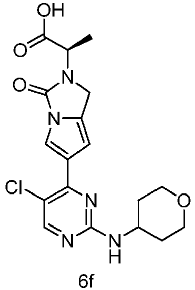 |
| | (*R*)-2-(6-(5-Chloro-2-((tetrahydro-2*H*-pyran-4-yl)amino)pyrimidin-4-yl)-3-oxo-1*H*-pyrrolo[1,2-*c*]imidazol-2(3*H*)-yl)propanoic acid **6f** |
| 7d | 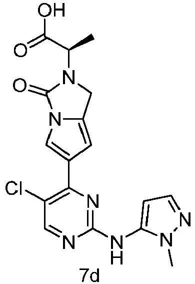 |
| | (*R*)-2-(6-(5-Chloro-2-((1-methyl-1*H*-pyrazol-5-yl)amino)pyrimidin-4-yl)-3-oxo-1*H*-pyrrolo[1,2-*c*]imidazol-2(3*H*)-yl)propanoic acid **7d** |
| 9g | 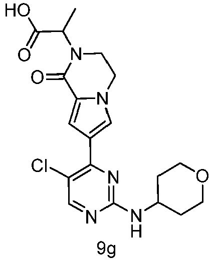 |
| | 2-(7-(5-Chloro-2-((tetrahydro-2*H*-pyran-4-yl)amino)pyrimidin-4-yl)-1-oxo-3,4-dihydropyrrolo[1,2-*a*]pyrazin-2(1*H*)-yl)propanoic acid **9g** |
| 10h | 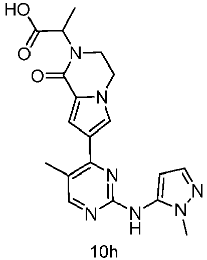 |
| | 2-(7-(5-Methyl-2-((1-methyl-1*H*-pyrazol-5-yl)amino)pyrimidin-4-yl)-1-oxo-3,4-dihydropyrrolo[1,2-*a*]pyrazin-2(1*H*)-yl)propanoic acid **10h** |
| | 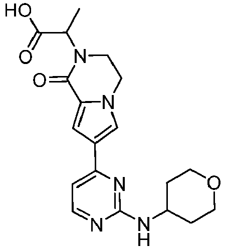 |
| | 2-(7-(2-((Tetrahydro-2*H*-pyran-4-yl)amino)pyrimidin-4-yl)-1-oxo-3,4-dihyd ropyrrolo[1,2-*a*]pyrazin-2(1*H*)-yl)propanoic acid |

In another aspect, the present disclosure relates to a method for preparing the compound of formula (I) or the tautomer, mesomer, racemate, enantiomer, diastereomer thereof, or mixture thereof, or the pharmaceutically acceptable salt thereof, comprising a step of: 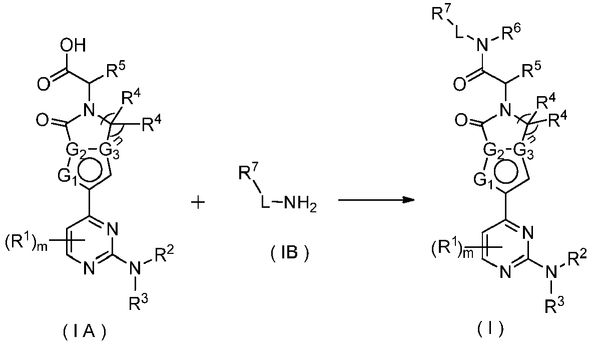 subjecting a compound of formula (IA) and a compound of formula (IB) to a condensation reaction under an alkaline condition to obtain the compound of formula (I) or the tautomer, mesomer, racemate, enantiomer, diastereomer thereof, or mixture thereof, or the pharmaceutically acceptable salt thereof, wherein the alkaline condition is preferably *N,N-*diisopropylethylamine; R⁶ is a hydrogen atom; and G₁, G₂, G₃, R¹ to R⁵, R⁷, L, m and n are as defined in the compound of formula (I).

Typical compounds of formula (IB) include, but are not limited to: 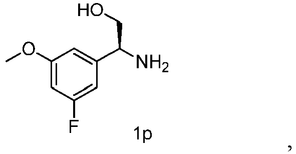 (*S*)-2-amino-2-(*m*-tolyl)ethanol and (*S*)-2-amino-2-(*m*-chlorophenyl)ethanol

In another aspect, the present disclosure relates to a pharmaceutical composition comprising the compound of formula (I) or formula (II) or formula (III), or the tautomer, mesomer, racemate, enantiomer, diastereomer thereof, or mixture thereof, or the pharmaceutically acceptable salt thereof, and one or more pharmaceutically acceptable carriers, diluents or excipients.

The present disclosure further relates to a use of the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same in the preparation of a medicament for inhibiting ERK

The present disclosure further relates to a use of the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same in the preparation of a medicament for treating or preventing cancer, inflammation, or other proliferative diseases, preferably cancer, wherein the cancer is selected from the group consisting of melanoma, liver cancer, kidney cancer, lung cancer (such as non-small cell lung cancer or small cell lung cancer), nasopharyngeal cancer, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The present disclosure further relates to a method for inhibiting ERK comprising a step of administering to a subject in need thereof the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same.

The present disclosure further relates to a method for treating or preventing ERK-mediated diseases comprising a step of administering to a subject in need thereof the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same.

The present disclosure further relates to a method for treating or preventing cancer, inflammation, or other proliferative diseases, preferably cancer, comprising a step of administering to a subject in need thereof the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same, wherein the cancer is selected from the group consisting of melanoma, liver cancer, kidney cancer, lung cancer (such as non-small cell lung cancer or small cell lung cancer), nasopharyngeal cancer, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The present disclosure further relates to the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same, for use as a medicament.

The present disclosure further relates to the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same, for use as an ERK inhibitor.

The present disclosure further relates to the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same, for use in treating or preventing ERK-mediated diseases.

The present disclosure further relates to the compound of formula (I) or formula (II) or formula (III) or the tautomer, mesomer, racemate, enantiomer, diastereomer thereof, or mixture thereof, or the pharmaceutically acceptable salt thereof, or the pharmaceutical composition comprising the same, for use in treating or preventing cancer, inflammation, or other proliferative diseases, preferably cancer, wherein the cancer is selected from the group consisting of melanoma, liver cancer, kidney cancer, lung cancer (such as non-small cell lung cancer or small cell lung cancer), nasopharyngeal cancer, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma (such as multiple myeloma), astrocytoma and glioma.

The active compound can be prepared in a form suitable for administration by any appropriate route, and the active compound is preferably in a unit dose form, or in a form in which the patient can self-administer in a single dose. The unit dosage form of the compound or composition of the present disclosure can be tablet, capsule, cachet, bottled syrup, powder, granule, lozenge, suppository, reconstituted powder or liquid formulation.

The dosage of the compound or composition used in the treatment method of the present disclosure will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present disclosure can also comprise one or more auxiliaries including a filler (diluent), binder, wetting agent, disintegrant, excipient and the like. Depending on the administration mode, the composition can comprise 0.1 to 99% by weight of the active compound.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents, binders and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier, an oil medium.

An aqueous suspension comprises an active ingredient in admixture with excipients suitable for the manufacture of an aqueous suspension. Such excipients are suspending agents, dispersants or wetting agents. The aqueous suspension can also comprise one or more preservatives, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The active ingredient in admixture with the dispersants or wetting agents, suspending agents or one or more preservatives can be prepared as dispersible powders or granules suitable for the preparation of an aqueous suspension by adding water. Suitable dispersants or wetting agents and suspending agents are exemplified by those already mentioned above. Additional excipients, such as sweeteners, flavoring agents and colorants, can also be added. These compositions are preserved by adding an antioxidant such as ascorbic acid.

The pharmaceutical composition of the present disclosure can also be in the form of an oil-in-water emulsion. The oil phase can be a vegetable oil, or a mineral oil, or a mixture thereof. Suitable emulsifying agents can be naturally occurring phospholipid. The emulsion can also contain a sweetening agent, flavoring agent, preservative and antioxidant. Such formulations can also contain a demulcent, a preservative, a coloring agent and an antioxidant.

The pharmaceutical composition of the present disclosure can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection. Alternatively, the solution and micro-emulsion are preferably administrated in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition of the present disclosure can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium. For this purpose, any blended fixed oil can be used. In addition, fatty acids can also be used to prepare injections.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### TERM SECTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n-octyl,* 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of H atom, D atom, halogen, alkyl, alkoxy, haloalkyl, hydroxy, hydroxyalkyl, cyano, amino, nitro, cycloalkyl, heterocyclyl, aryl and heteroaryl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of H atom, D atom, halogen, alkyl, alkoxy, haloalkyl, hydroxy, hydroxyalkyl, cyano, amino, nitro, cycloalkyl, heterocyclyl, aryl and heteroaryl.

The term "alkylene" refers to a saturated linear or branched aliphatic hydrocarbon group having two residues derived from the removal of two hydrogen atoms from the same carbon atom or two different carbon atoms of the parent alkane. It is a linear or branched alkylene having 1 to 20 carbon atoms, preferably 1 to 12 carbon atoms, and more preferably 1 to 6 carbon atoms. Non-limiting examples of alkylene include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-) and the like. The alkylene can be substituted or unsubstituted. When substituted, the substituent group(s) can be substituted at any available connection point. The substituent group(s) is preferably one or more groups independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkenyl" refers to an alkyl containing carbon-carbon double bond(s) in the molecule, wherein the definition of the alkyl is as described above. The alkenyl can be substituted or unsubstituted. When substituted, the substituent group(s) is preferably one or more groups independently selected from the group consisting of hydrogen atom, alkyl, alkoxy, halogen, haloalkyl, hydroxy, hydroxyalkyl, cyano, amino, nitro, cycloalkyl, heterocyclyl, aryl and heteroaryl.

The term "alkynyl" refers to an alkyl containing carbon-carbon triple bond(s) in the molecule, wherein the definition of the alkyl is as described above. The alkenyl can be substituted or unsubstituted. When substituted, the substituent group(s) is preferably one or more groups independently selected from the group consisting of hydrogen atom, alkyl, alkoxy, halogen, haloalkyl, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent group having 3 to 20 carbon atoms, preferably 3 to 12 (for example 3, 4, 5, 6, 7, 8, 9, 10, 11 and 12) carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered (for example 7, 8, 9 or 10 membered) spiro cycloalkyl. According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

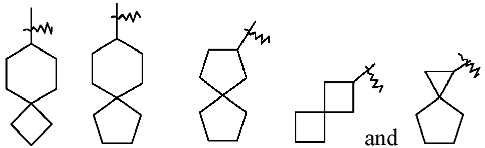

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered (for example 7, 8, 9 or 10 membered) fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

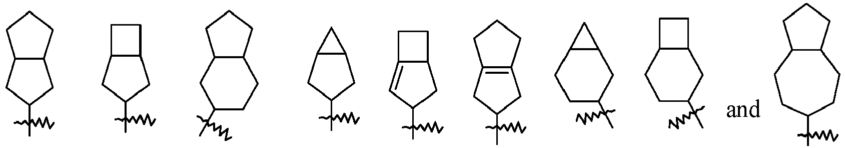

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered (for example 7, 8, 9 or 10 membered) bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

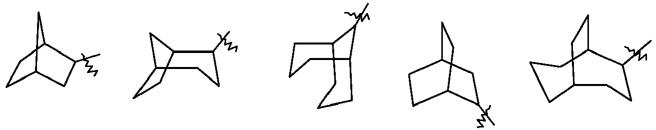

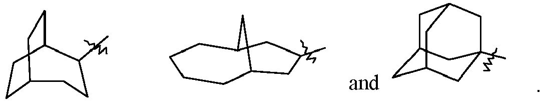

The cycloalkyl (including monocyclic cycloalkyl, spiro cycloalkyl, fused cycloalkyl and bridged cycloalkyl) ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl. The cycloalkyl can be substituted or unsubstituted. When substituted, the substituent group(s) can be substituted at any available connection point. The substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "heterocyclyl" refers to a 3 to 20 membered saturated or partially unsaturated monocyclic or polycyclic hydrocarbon substituent group, wherein one or more ring atoms are heteroatoms selected from the group consisting of N, O, S, S(O) and S(O)₂, but excluding -O-O-, -O-S- or -S-S- in the ring, with the remaining ring atoms being carbon atoms. Preferably, the heterocyclyl has 3 to 12 (for example 3, 4, 5, 6, 7, 8, 9, 10, 11 and 12) ring atoms wherein 1 to 4 (for example 1, 2, 3 and 4) atoms are heteroatoms; more preferably, 3 to 8 ring atoms wherein 1 to 3 atoms are heteroatoms; more preferably, 3 to 6 ring atoms wherein 1 to 3 atoms are heteroatoms; and most preferably 5 or 6 ring atoms wherein 1 to 3 atoms are heteroatoms. Non-limiting examples of monocyclic heterocyclyl include pyrrolidinyl, tetrahydropyranyl, 1,2,3,6-tetrahydropyridyl, piperidinyl, piperazinyl, morpholinyl, thiomorpholinyl, homopiperazinyl and the like. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O, S, S(O) and S(O)₂, with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 10 membered (for example 7, 8, 9 or 10 membered) spiro heterocyclyl. According to the number of the spiro atoms shared between the rings, the spiro heterocyclyl is divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

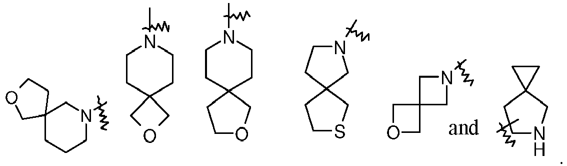

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O, S, S(O) and S(O)₂, with the remaining ring atoms being carbon atoms. The fused heterocyclyl is preferably a 6 to 14 membered fused heterocyclyl, and more preferably a 7 to 10 membered (for example 7, 8, 9 or 10 membered) fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

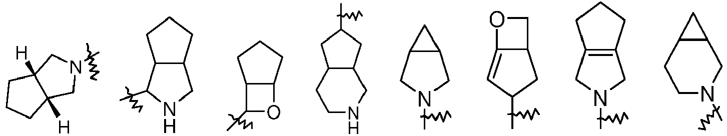

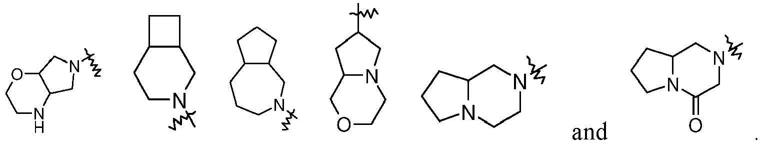

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O, S, S(O) and S(O)₂, with the remaining ring atoms being carbon atoms. The bridged heterocyclyl is preferably a 6 to 14 membered bridged heterocyclyl, and more preferably a 7 to 10 membered (for example 7, 8, 9 or 10 membered) bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

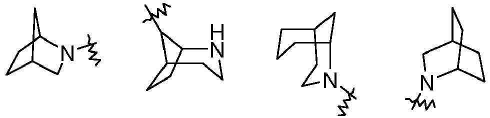

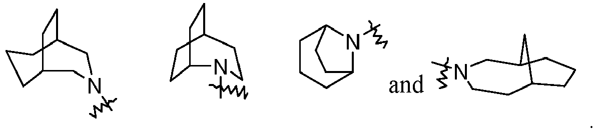

The heterocyclyl (including monocyclic heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl, and non-limiting examples thereof include: 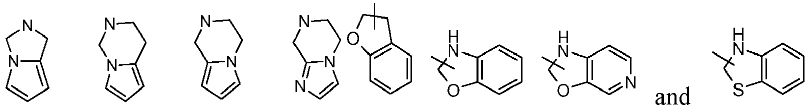 and the like.

The heterocyclyl (including monocyclic heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl, and non-limiting examples thereof include:

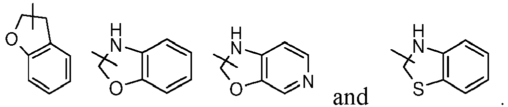

The heterocyclyl can be substituted or unsubstituted. When substituted, the substituent group(s) can be substituted at any available connection point. The substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl ring can be fused to the ring of heteroaryl, heterocyclyl or cycloalkyl, wherein the ring bound to the parent structure is aryl ring, and non-limiting examples thereof include:

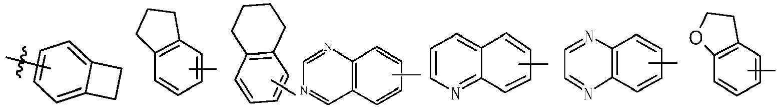

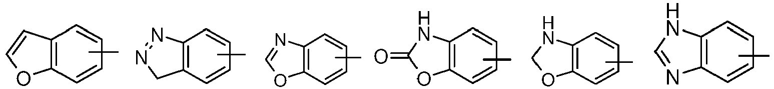

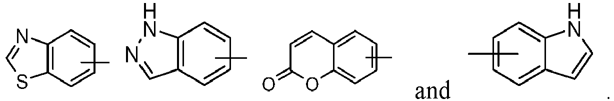

The aryl can be substituted or unsubstituted. When substituted, the substituent group(s) can be substituted at any available connection point. The substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl, more preferably a 5 or 6 membered heteroaryl; for example, furyl, thienyl, pyridyl, pyrrolyl, N-alkylpyrrolyl, pyrimidinyl, pyrazinyl, pyridazinyl, imidazolyl, pyrazolyl, triazolyl, tetrazolyland the like, preferably pyridazinyl and pyridinyl, and more preferably pyridazinyl. The heteroaryl ring can be fused to the ring of aryl, heterocyclyl or cycloalkyl, wherein the ring bound to the parent structure is heteroaryl ring, and non-limiting examples thereof include:

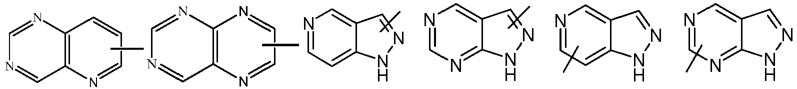

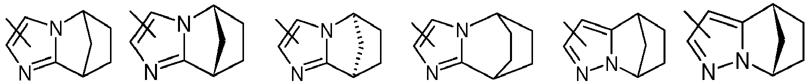

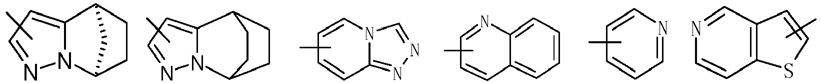

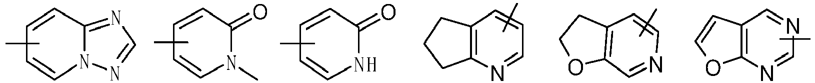

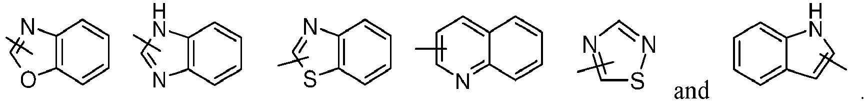

The heteroaryl can be optionally substituted or unsubstituted. When substituted, the substituent group(s) can be substituted at any available connection point. The substituent group(s) is preferably one or more groups independently selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "cycloalkyloxy" refers to a cycloalkyl-O- group, wherein the cycloalkyl is as defined above.

The term "haloalkyl" refers to an alkyl group substituted by one or more halogens, wherein the alkyl is as defined above.

The term "deuterated alkyl" refers to an alkyl group substituted by one or more deuterium atoms, wherein the alkyl is as defined above.

The term "hydroxy" refers to an -OH group.

The term "hydroxyalkyl" refers to an alkyl group substituted by hydroxy(s), wherein the alkyl is as defined above.

The term "halogen" refers to fluorine, chlorine, bromine or iodine.

The term "hydroxy" refers to an -OH group.

The term "amino" refers to a -NH₂ group.

The term "cyano" refers to a -CN group.

The term "nitro" refers to a -NO₂ group.

The term "carbonyl" refers to a C=O group.

The term "carboxy" refers to a -C(O)OH group.

The term "alkoxycarbonyl" refers to a -C(O)O(alkyl), -C(O)O(cycloalkyl), -OC(O)(alkyl) or -OC(O)(cycloalkyl) group, wherein the alkyl and cycloalkyl are as defined above.

The present disclosure also comprises the compounds of formula (I) in various deuterated forms. Each of the available hydrogen atoms attached to the carbon atom can be independently replaced with a deuterium atom. Those skilled in the art can synthesize a compound of formula (I) in a deuterated form with reference to the relevant literatures. The compound of formula (I) in deuterated form can be prepared by employing commercially available deuterated raw materials, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

The compound of the present disclosure can also comprise isotopic derivatives thereof. The term "isotopic derivatives" refers to compounds that differ in structure only in the presence of one or more isotopically enriched atoms. For example, a compound having the structure of the present disclosure except replacing hydrogen with "deuterium" or "tritium", or replacing fluorine with an ¹⁸F-fluorine labeling (¹⁸F isotope), or replacing carbon with ¹¹C-, ¹³C-, or ¹⁴C-enriched carbon (¹¹C-, ¹³C-, or ¹⁴C-carbon labeling; ¹¹C-, ¹³C-, or ¹⁴C-isotope) is within the scope of the present disclosure. Such compounds can be used, for example, as analytical tools or probes in biological assays, or as tracers for in vivo diagnostic imaging of disease, or as tracers for pharmacodynamics, pharmacokinetics or receptor studies. Deuterated compounds can generally retain activity comparable to non-deuterated compounds, and when deuterated at certain specific sites, the resulting compounds can achieve better metabolic stability, thereby obtaining certain therapeutic advantages (such as increased in vivo half-life or reduced dosage requirements).

For drugs or pharmacologically active agents, the term "therapeutically effective amount" refers to a sufficient amount of a drug or agent that is non-toxic but can achieve the desired effect. The determination of the effective amount varies from person to person, depending on the age and general condition of the recipient, and also on the specific active substance. The appropriate effective amount in a case can be determined by the person skilled in the art according to routine experiments.

The present disclosure provides novel ERK inhibitors, and finds that compounds with such structures have strong inhibitory activity and high selectivity, and compounds with such structures have good pharmacokinetic absorption.

### Synthesis Method of the Compound of the Present Disclosure

In order to achieve the object of the present disclosure, the present disclosure applies the following technical solutions.

### Scheme I

A method for preparing the compound of formula (I) or the tautomer, mesomer, racemate, enantiomer, diastereomer thereof, or mixture thereof, or the pharmaceutically acceptable salt thereof according to the present disclosure, comprising the following step of: 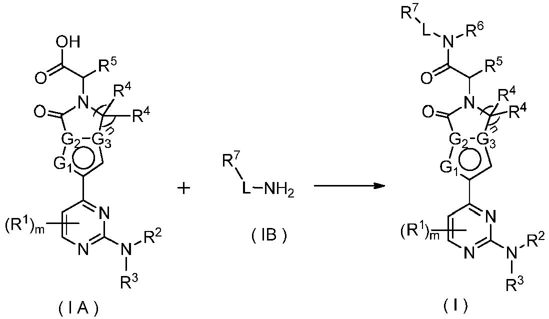 subjecting intermediate 1 of a compound of formula (IA) and intermediate 2 of a compound of formula (IB) to a condensation reaction under an alkaline condition to obtain the compound of formula (I), wherein: R¹ to R⁷, L, m and n are as defined in the compound of formula (I).

### Other Schemes

In other schemes of the preparation of the compound of formula (II-1), (II-2), (II-3), (II-4), (III-1), (III-2), (III-3) or (III-4), intermediate 1 of the compound of formula (IA) in Scheme 1 is replaced with the compound of formula (II-1A), (II-2A), (II-3A), (II-4A)), (III-1A), (III-2A), (III-3A) or (III-4A); intermediate 2 of the compounds of formula (IB) are also replaced accordingly, as shown in the following table:

| Intermediate 1 (replacement of formula IA) | Intermediate 2 (optional replacement of formula IB) | Prepared product |
|---|---|---|
| 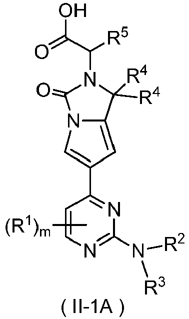 | 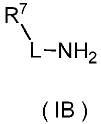 | 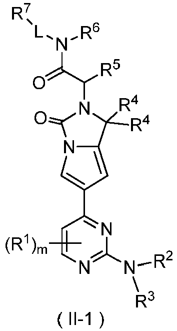 |
| 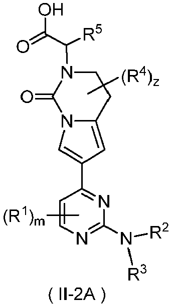 | | 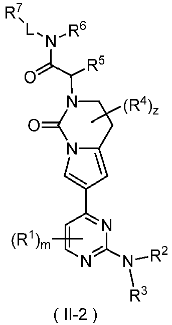 |
| 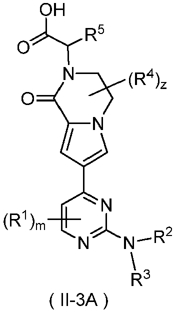 | | 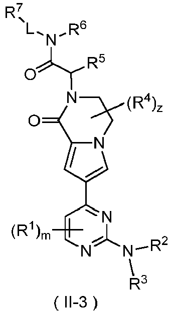 |
| 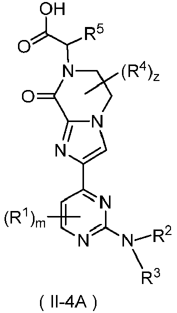 | | 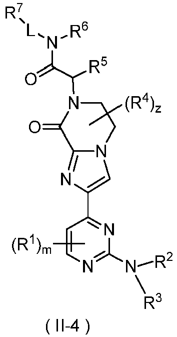 |
| 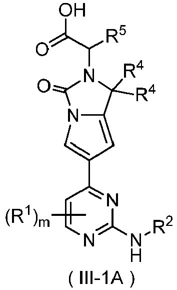 | 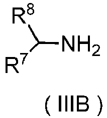 | 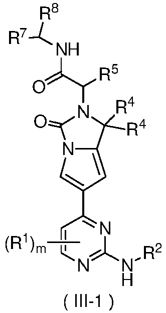 |
| 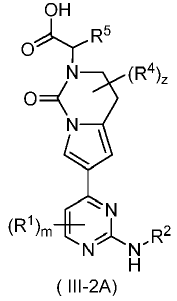 | | 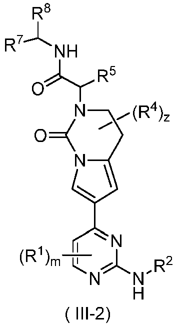 |
| 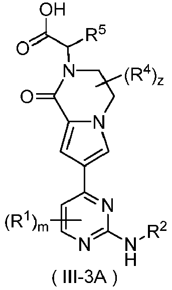 | | 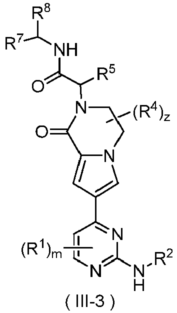 |
| 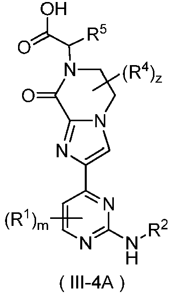 | | 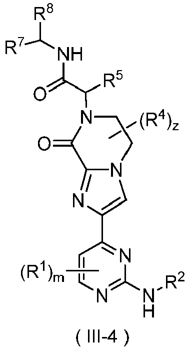 |

The reaction conditions are the same as in Scheme I, that is, a condensation reaction occurs under an alkaline condition to prepare the product.

The reagent that provides an alkaline condition in the above reactions includes organic bases and inorganic bases. The organic bases include, but are not limited to, triethylamine, *N,N-*diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide. The reagent that provides an alkaline condition is preferably *N,N-*diisopropylethylamine.

The above reactions are carried out in a solvent. The solvent used includes, but is not limited to, acetic acid, methanol, ethanol, *n*-butanol, toluene, tetrahydrofuran, dichloromethane, petroleum ether, ethyl acetate, *n*-hexane, dimethyl sulfoxide, 1,4-dioxane, ethylene glycol dimethyl ether, water, *N,N*-dimethylformamide and mixtures thereof, preferably *N,N-*dimethylformamide.

The condensing agent in the above condensation reactions includes, but is not limited to, 1-(3-dimethylaminopropyl)-3-ethylcarbodiimide hydrochloride, *N,N*'-dicyclohexylcarbodiimide, *N,N*'-diisopropylcarbodiimide, *O*-(benzotriazol-1-yl)-*N,N,N',N*' -tetramethyluronium tetrafluoroborate, 1-hydroxybenzotriazole, 1-hydroxy-7-azobenzotriazole, *O*-(benzotriazol-1-yl)-*N,N,N*'*,N*'-tetramethyluronium hexafluorophosphate, 2-(7-azabenzotriazol-1-yl)-*N,N,N*'*,N*'-tetramethyluronium hexafluorophosphate, 2-(7-oxobenzotriazol-1-yl)-*N,N,N*'*,N*'-tetramethyluronium hexafluorophosphate, benzotriazol-1-yloxytris(dimethylamino)phosphonium hexafluorophosphate and benzotriazol-1-yl-oxytripyrrolidinylphosphonium hexafluorophosphate, and preferably 2-(7-azabenzotriazol-1-yl)-*N,N*,*N*',*N*'-tetramethyluronium hexafluorophosphate.

The details of one or more embodiments of the present disclosure are set forth in the above specification. Although any methods and materials similar or identical to those described herein can be used to perform or test the present disclosure, the preferred methods and materials are described below. Through the specification and claims, other features, purposes and advantages of the present disclosure will be apparent. In the specification and claims, unless the context clearly indicates otherwise, the singular form includes the plural referent. Unless otherwise defined, all technical and scientific terms used herein have the general meanings as understood by the person of ordinary skill in the art to which the present disclosure belongs. All patents, patent applications and publications cited in the specification are incorporated by reference. The following examples are provided to more fully illustrate the preferred embodiments of the present disclosure. These examples should not be construed as limiting the scope of the present disclosure in any way, and the scope of the present disclosure is defined by the claims.

### DETAILED DESCRIPTION

The present disclosure will be further described with reference to the following examples, but the examples should not be considered as limiting the scope of the present disclosure.

### EXAMPLES

The structures of the compounds were identified by nuclear magnetic resonance (NMR) and/or mass spectrometry (MS). NMR shifts (δ) are given in 10⁻⁶ (ppm). NMR is determined by a Bruker AVANCE-400 machine. The solvents for determination are deuterated-dimethyl sulfoxide (DMSO-*d*₆), deuterated-chloroform (CDCl₃) and deuterated-methanol (CD₃OD), and the internal standard is tetramethylsilane (TMS). MS was determined by an Agilent 1200 /1290 DAD- 6110/6120 Quadrupole MS liquid chromatograph/mass spectrometer (manufacturer: Agilent, MS model: 6110/6120 Quadrupole MS), waters ACQuity UPLC-QD/SQD (manufacturer: waters, MS model: waters ACQuity Qda Detector/waters SQ Detector), THERMO Ultimate 3000-Q Exactive (manufacturer: THERMO, MS model: THERMO Q Exactive).

High performance liquid chromatography (HPLC) was determined on an Agilent HPLC 1200DAD, Agilent HPLC 1200VWD and Waters HPLC e2695-2489 high pressure liquid chromatograph.

Chiral HPLC was determined on an Agilent 1260 DAD high performance liquid chromatograph.

Preparative high performance liquid chromatography was carried out on Waters 2545-2767, Waters 2767-SQ Detecor2, Shimadzu LC-20AP and Gilson GX-281 preparative chromatographs.

Chiral preparation was carried out on a Shimadzu LC-20AP preparative chromatograph.

CombiFlash rapid preparation instrument used was Combiflash Rf200 (TELEDYNE ISCO).

Yantai Huanghai HSGF254 or Qingdao GF254 silica gel plate was used as the thin layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for silica gel column chromatography.

The average kinase inhibition rates and IC₅₀ values were determined by a NovoStar microplate reader (BMG Co., Germany).

The known starting materials of the present disclosure can be prepared by the known methods in the art, or can be purchased from ABCR GmbH & Co. KG, Acros Organics, Aldrich Chemical Company, Accela ChemBio Inc., Dari Chemical Company etc.

Unless otherwise stated, the reactions can be carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction was performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, and C: petroleum ether/ethyl acetate system. The ratio of the volume of the solvent was adjusted according to the polarity of the compounds, and a small quantity of alkaline reagent such as triethylamine or acidic reagent such as acetic acid could also be added for adjustment.

### Example 1

### (S)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-1-oxo-3,4-dihydropyrrolo[1,2-c]pyrimidin-2(1 H)-yl)propanamide 1-P1

### (R)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-1-oxo-3,4-dihydropyrrolo[1,2-c]pyrimidin-2(1 H)-yl)propanamide 1-P2

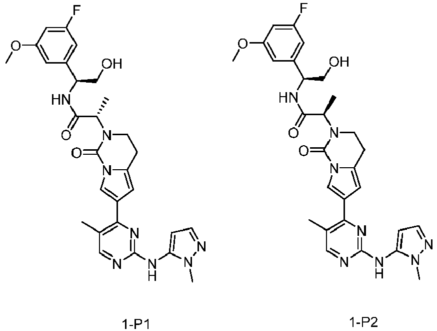

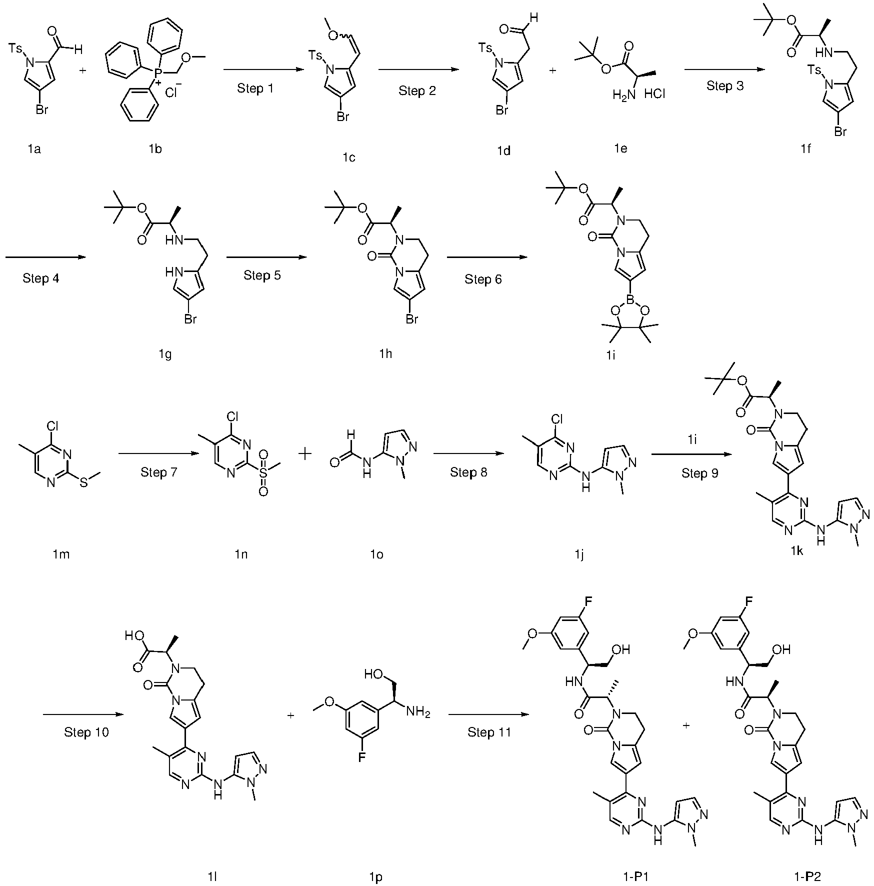

### Step 1

### 4-Bromo-2-(2-methoxyvinyl)-1-tosyl-1H-pyrrole 1c

(Methoxymethyl)triphenylphosphonium chloride **1b** (12.5 g, 36.6 mmol) was dissolved in 150 mL of tetrahydrofuran, and the solution was cooled to 0°C. Potassium *tert*-butoxide (4.1 g, 36.6 mmol) was added, and the reaction solution was stirred for 0.5 hours. 4-Bromo-1-tosyl-1*H*-pyrrole-2-carbaldehyde **1a** (4.0 g, 612.19 mmol, Journal of Organic Chemistry, 2006, vol. 71, # 11, p. 4092-4102) was added, and the reaction solution was stirred for 14 hours. 100 mL of water was added, the reaction solution was concentrated under reduced pressure, and the residues were purified by column chromatography with eluent system C to obtain the title compound **1c** (5.2 g), yield: 79.8%.

MS m/z (ESI): 356.0 [M+1].

### Step 2

### 2-(4-Bromo-1-tosyl-1H-pyrrol-2-yl)acetaldehyde 1d

Compound **1c** (5.2 g, 14.6 mmol) was dissolved in 20 mL of tetrahydrofuran, followed by the addition of 15 mL of concentrated hydrochloric acid, and the reaction solution was stirred for 3 hours. Saturated sodium bicarbonate was added to adjust the pH to 7. The reaction solution was extracted with ethyl acetate (150 mL×2), and the organic phase was concentrated to obtain the title crude compound **1d** (4.9 g), which was directly used in the next step without purification.

MS m/z (ESI): 342.1 [M+1].

### Step 3

### tert-Butyl (R)-2-((2-(4-bromo-1-tosyl-1H-pyrrol-2-yl)ethyl)amino)propionate If

*tert*-Butyl D-alaninate hydrochloride **1e** (3.11 g, 21.5 mmol, Shanghai Bide Pharmatech Ltd.) was dissolved in 25 mL of methanol, followed by the addition of anhydrous potassium carbonate (4.76 g, 34.4 mmol), and the mixture was stirred for 0.5 hours and filtered to remove insoluble matter. Compound **1d** (4.9 g, 14.3 mmol) was added, and the reaction solution was cooled to 0°C. Sodium cyanoborohydride (1.35 g, 21.5 mmol) was added, and the reaction solution was stirred for 14 hours. 100 mL of water was added, and the reaction solution was extracted with ethyl acetate (150 mL×2), dried with anhydrous sodium sulfate, concentrated under reduced pressure, and purifed by column chromatography with eluent system B to obtain the title compound **If** (2.2 g), yield: 33%.

MS m/z (ESI): 471.1 [M+1].

### Step 4

### tert-Butyl (R)-2-((2-(4-bromo-1H-pyrrol-2-yl)ethyl)amino)propionate 1g

Compound **1f** (2.2 g, 4.67 mmol) was dissolved in 10 mL of tetrahydrofuran, followed by the addition of 15 mL of tetrabutylammonium fluoride in tetrahydrofuran (1 M), and the reaction solution was stirred at 65°C for 1 hour. 50 mL of saturated sodium bicarbonate solution was added, and the reaction solution was extracted with ethyl acetate (100 mL×2). The organic phase was concentrated, and the residues were purified by column chromatography with eluent system B to obtain the title compound **1g** (700 mg), yield: 47.2%.

MS m/z (ESI): 317.2 [M+1].

### Step 5

### tert-Butyl (R)-2-(6-bromo-1-oxo-3,4-dihydropyrrolo[1,2-c]pyrimidin-2(1H)-yl)propanoate 1h

Compound **1g** (700 mg, 2.21 mmol) was dissolved in 10 mL of tetrahydrofuran, followed by the addition of *N,N*'-carbonyldiimidazole (1.07 g, 6.62 mmol), and the mixture was stirred for 0.5 hours. Sodium hydride (60%, 254 mg, 6.62 mmol) was added, and the reaction solution was stirred for 14 hours. Water was added, the reaction solution was concentrated under reduced pressure, and the residues were purified by thin layer chromatography with developing solvent system C to obtain the title compound **1h** (680 mg), yield: 89.7%.

MS m/z (ESI): 343.2[M+1].

### Step 6

### tert-Butyl (R)-2-(1-oxo-6-(4,4,5,5-tetramethyl-1,3,2-dioxaborolan-2-yl)-3,4-dihydropyrrolo[1,2-c] pyrimidin-2(1H)-yl)propanoate 1i

Under an argon atmosphere, compound **1h** (680 mg, 1.98 mmol) was dissolved in 30 mL of 1,4-dioxane, followed by the addition of [1,1'-bis(diphenylphosphino)ferrocene]palladium (II) dichloride (290 mg, 0.40 mmol) and potassium acetate (389 mg, 3.96 mmol) successively. The reaction solution was stirred at 70°C for 2 hours, cooled, and filtered through Celite. The filtrate was concentrated, and the residues were purified by thin layer chromatography with developing solvent system C to obtain the title compound **1i** (550 mg), yield: 71.1%.

MS m/z (ESI): 391.2 [M+1].

### Step 7

### 4-Chloro-5-methyl-2-(methylsulfonyl)pyrimidine 1n

4-Cloro-5-methyl-2-(methylthio)pyrimidine **1m** (500 mg, 2.86 mmol, Shanghai Bide Pharmatech Ltd.) in 10 mL of dichloromethane, followed by the addition of *m*-chloroperoxybenzoic acid (1.270 g, 6.3 mmol), and the reaction solution was stirred for 2 hours. The reaction solution was washed with a saturated solution of sodium thiosulfate and a saturated solution of sodium chloride, dried with anhydrous sodium sulfate, and filtered. The filtrate was concentrated under reduced pressure to obtain the crude title compound **1n** (445 mg), which was directly used in the next step without purification.

MS m/z (ESI): 207.2 [M+1].

### Step 8

### 4-Chloro-S-methyl-N-(1-methyl-1H-pyrazol-5-yl)pyrimidin-2-amine 1j

*N*-(1-Methyl -1*H*-pyrazol-5-yl)formamide **1o** (270 mg, 2.15 mmol, prepared by the method disclosed in the patent application "WO201780979, the specification, page 88, Example 2") was dissolved in *N,N*-dimethylformamide, followed by the addition of sodium hydride (60%, 250 mg, 6.5 mmol) at 0°C, and the mixture was stirred for 0.5 hours. The crude compound **1n** (445 mg, 2.15 mmol) was added, and the reaction solution was further reacted for 2 hours. 20 mL of water was added, and the reaction solution was extracted with ethyl acetate (20 mL×3). The organic phases were combined and concentrated under reduced pressure, and the resulting residues were purified by thin layer chromatography with developing solvent system C to obtain the title compound **1j** (240 mg), yield: 49.7%.

MS m/z (ESI): 224.3 [M+1].

### Step 9

### tert-Butyl (R)-2-(6-(5-methyl-2-((1-methyl-1H-pyrazol-5-yl)amino)pyrimidin-4-yl)-1-oxo-3,4-dih ydropyrrolo[1,2-c]pyrimidin-2(1H)-yl)propanoate 1k

Compound **1i** (400 mg, 1.02 mmol) was dissolved in 6 mL of dioxane and 1 mL of water under an argon atmosphere, followed by the addition of compound **1j** (252 mg, 1.12 mmol), cesium carbonate (668 mg, 2.05 mmol) and [1,1'-bis(diphenylphosphino)ferrocene]palladium (II) dichloride (150 mg, 0.20 mmol) successively. The reaction solution was stirred at 80°C for 14 hours, cooled, and filtered through Celite. The filtrate was concentrated, and the residues were purified by column chromatography with eluent system A to obtain the title compound **1k** (340 mg), yield: 73.1%.

MS m/z (ESI): 452.2[M+1].

### Step 10

### (R)-2-(6-(5-Methyl-2-((1-methyl-1H-pyrazol-5-yl)amino)pyrimidin-4-yl)-1-oxo-3,4-dih ydropyrrolo[1,2-c]pyrimidin-2(1H)-yl)propanoic acid 1l

Compound **1k** (340 mg, 0.75 mmol) was dissolved in 3 mL of dichloromethane, followed by the dropwise addition of 1 mL of trifluoroacetic acid. After the addition was completed, the reaction solution was stirred for 1 hour. The organic phase is concentrated to obtain the title compound **1l** (297 mg), yield: 99%.

MS m/z (ESI): 396.2 [M+1].

### Step 11

### (S)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-1-oxo-3,4-dihydropyrrolo[1,2-c]pyrimidin-2(1 H)-yl)propanamide 1-P1

### (R)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-1-oxo-3,4-dihydropyrrolo[1,2-c]pyrimidin-2(1 H)-yl)propanamide 1-P2

Compund **1l** (297 mg, 0.75 mmol) and compound **1p** (139 mg, 0.75 mmol, Shanghai Haohong Biomedical Technology Co., Ltd.) were dissolved in 5 mL *N,N*-dimethylformamide, followed by the addition of 2-(7-azabenzotriazol-1-yl)-*N,N*,*N*',*N*'-tetramethyluronium hexafluorophosphate (286 mg, 0.75 mmol) and *N,N*-diisopropylethylamine (194 mg, 1.5 mmol). The reaction solution was stirred for 14 hours, and concentrated under reduced pressure. The residues were purified by preparative liquid chromatography (Instrument model: Gilson 281, chromatographic column: X-Bridge, Prep 30^{∗}150 mm; 5µm; C18, mobile phase: A-water (10 mM ammonium bicarbonate), B-acetonitrile, flow rate: 30 mL/min, column temperature: room temperature) to obtain the title compounds **1-P1** and **1-P2** (30 mg, 30 mg).

### Compound in a single configuration (with shorter retention time) 1-P1

MS m/z (ESI): 563.2 [M+1]
HPLC analysis: retention time: 16.1 minutes, purity: 98.5% (chromatographic column: X-Bridge, Prep 30^{∗}150mm; 5µm; mobile phase: A-water (10 mM ammonium bicarbonate), B-acetonitrile, gradient: A 23%-42%)
¹H NMR (400 MHz, CD₃OD) *δ* 8.18 (d, 1H), 7.84 (s, 1H), 7.96 (s, 1H), 7.42-7.42 (m, 1H) 6.57-6.73 (m, 3H), 6.63-6.32 (m, 1H), 5.17-5.18 (m, 1H), 4.89-4.94 (m, 1H), 3.74-3.81 (m, 10H), 2.95-3.00 (m, 2H), 2.37 (s, 3H), 1.52 (d, 3H).

### Compound in a single configuration (with longer retention time)1-P2

MS m/z (ESI): 563.2 [M+1]
HPLC analysis: retention time: 18.4 minutes, purity: 97.2% (chromatographic column: X-Bridge, Prep 30^{∗}150mm; 5µm; mobile phase: A-water (10 mM ammonium bicarbonate), B-acetonitrile, gradient: A 23%-42%)
MS m/z (ESI): 563.2 [M+1]
¹H NMR (400 MHz, CD₃OD) *δ* 8.22 (s, 1H), 7.96 (s, 1H), 7.58 (s, 1H), 6.59-6.75 (m, 4H), 6.48 (s, 1H), 5.17-5.19 (m, 1H), 4.95-4.96 (m, 1H), 3.72-3.81 (m, 10H), 3.02-3.05 (m, 2H), 2.43 (s, 3H), 1.50 (d, 3H).

### Example 2

### (S)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-fluoro-5-methoxyphenyl)-2-hydroxye thyl)propanamide 2-P1

### (R)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-fluoro-5-methoxyphenyl)-2-hydroxye thyl)propanamide 2-P2

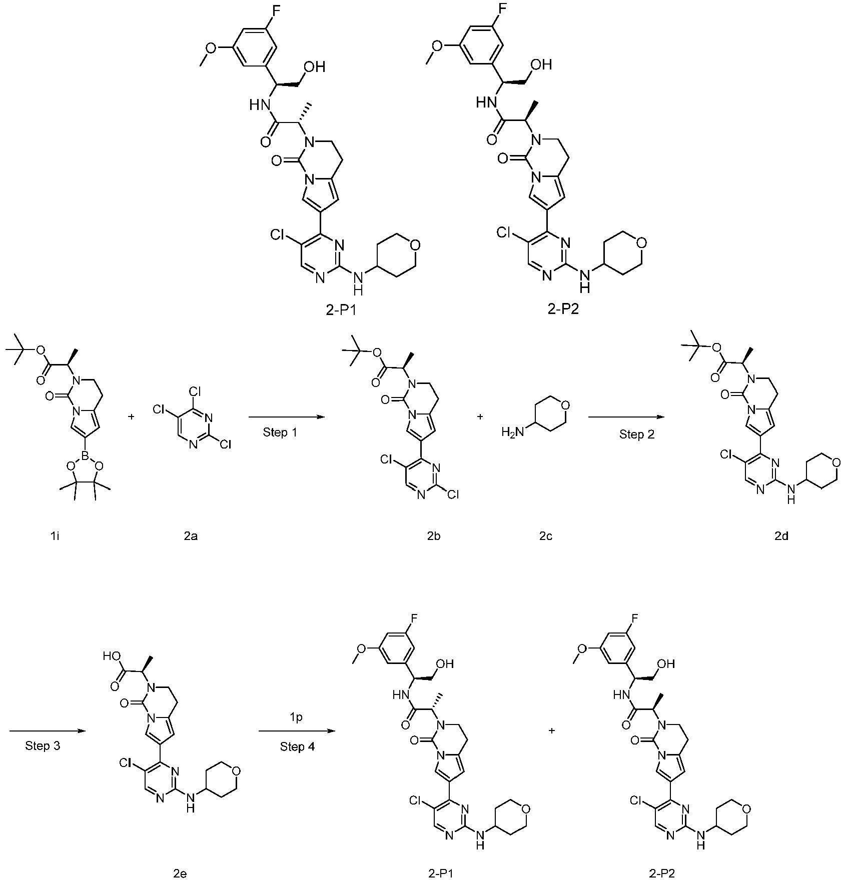

### Step 1

### tert-Butyl (R)-2-(6-(2,5-dichloropyrimidin-4-yl)-1-oxo-3,4-dihydropyrrolo[1,2-c]pyrimidin-2(1H)-yl)propanoate 2b

Compound **1i** (580 mg, 1.48 mmol) was dissolved in 6 mL of dioxane and 1 mL of water under an argon atmosphere, followed by the addition of compound **2a** (327 mg, 1.78 mmol, Shanghai Bide Pharmatech Ltd.), sodium carbonate (315 mg, 2.97 mmol) and [1,1'-bis(diphenylphosphino)ferrocene]palladium (II) dichloride (126 mg, 0.15 mmol) successively. The reaction solution was reacted for 1 hour at 80°C in microwave reactor, cooled, and filtered through Celite. The filtrate was concentrated, and the residues were purified by column chromatography with eluent system A to obtain the title compound **2b** (200 mg), yield: 32.7%.

MS m/z (ESI): 411.2 [M+1].

### Step 2

### tert-Butyl (R)-2-(6-(5-chloro-2-((tetrahydro-2H-pyran-4-yl)amino)pyrimidin-4-yl)-1-oxo-3,4-dihy dropyrrolo[1,2-c]pyrimidin-2(1H)-yl)propanoate 2d

Compound **2b** (200 mg, 0.49 mmol), compound **2c** (148 mg, 1.45 mmol, Shanghai Bide Pharmatech Ltd.) and *N,N*-diisopropylethylamine (126 mg, 0.98 mmol) were dissolved in 10 mL of tetrahydrofuran. The reaction solution was reacted for 4 hours at 100°C in microwave reactor, cooled and concentrated under reduced pressure. The residues were purified by thin layer chromatography with developing solvent system C to obtain the title compound **2d** (100 mg), yield: 43.2%.

MS m/z (ESI): 476.2 [M+1].

### Step 3

### (R)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)propanoic acid 2e

Compound **2d** (100 mg, 0.21 mmol) was dissolved in 3 mL of dichloromethane, followed by the dropwise addition of 1 mL of trifluoroacetic acid. After the addition was completed, the reaction solution was stirred for 1 hour. The organic phase is concentrated to obtain the title compound **2e** (88 mg), yield: 99%.

MS m/z (ESI): 420.1 [M+1].

### Step 4

### (S)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-fluoro-5-methoxyphenyl)-2-hydroxye thyl)propanamide 2-P1

### (R)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-fluoro-5-methoxyphenyl)-2-hydroxye thyl)propanamide 2-P2

Compound **2e** (88 mg, 0.21 mmol) and compound **1p** (38.8 mg, 0.21 mmol) were dissolved in 5 mL of *N,N*-dimethylformamide, followed by the addition of 2-(7-azabenzotriazol-1-yl)-*N,N*,*N*',*N*'-tetramethyluronium hexafluorophosphate (79.7 mg, 0.21 mmol) and *N,N*-diisopropylethylamine (54.2 mg, 0.42 mmol). The reaction solution was stirred for 14 hours, and concentrated under reduced pressure. The residues were purified by preparative liquid chromatography (Instrument model: Gilson 281, chromatographic column: Sharpsil-T, X-Bridge, Prep 30^{∗}150mm; 5µm; C18, mobile phase: A-water (10 mM ammonium bicarbonate), B-acetonitrile, flow rate: 30 mL/min, column temperature: room temperature) to obtain the title compounds **2-P1** and **2-P2** (30 mg, 30 mg).

### Compound in a single configuration (with shorter retention time) 2-P1

MS m/z (ESI): 587.2 [M+1]
HPLC analysis: retention time: 16.1 minutes, purity: 98.2% (chromatographic column: X-Bridge, Prep 30^{∗}150mm; 5µm; mobile phase: A-water (10 mM ammonium bicarbonate), B-acetonitrile, gradient: A 30%-48%)
¹H NMR (400 MHz, CD₃OD) *δ* 8.24 (s, 1H), 8.19 (s, 1H), 6.71-6.75 (m, 3H) 6.56 (d, 1H), 5.16-5.18 (m, 1H), 4.94-4.98 (m, 1H), 3.98-4.01 (m, 3H), 3.74-3.78 (m, 5H), 3.51-3.62 (m, 4H), 2.95-2.99 (m, 2H), 1.99-2.01 (m, 2H), 1.52-1.62 (m, 5H).

### Compound in a single configuration (with longer retention time) 2-P2

MS m/z (ESI): 587.2 [M+1]
HPLC analysis: retention time: 18.2 minutes, purity: 96.8% (chromatographic column: X-Bridge, Prep 30^{∗}150mm; 5µm; mobile phase: A-water (10 mM ammonium bicarbonate), B-acetonitrile, gradient: A 30%-48%)
¹H NMR (400 MHz, CD₃OD) *δ* 8.22 (s, 1H), 8.15 (s, 1H), 6.67-6.74 (m, 3H) 6.56 (d, 1H), 5.14-5.16 (m, 1H), 4.90-4.93 (m, 1H), 3.94-3.97 (m, 3H), 3.56-3.97 (m, 9H), 2.95-3.02 (m, 2H), 1.95-1.99 (m, 2H), 1.45-1.60 (m, 5H).

### Example 3

### (S)-2-(2-(5-Chloro-2-((tetrahydro-2H-pyran-4-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 3-P1

### (R)-2-(2-(5-Chloro-2-((tetrahydro-2H-pyran-4-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 3-P2

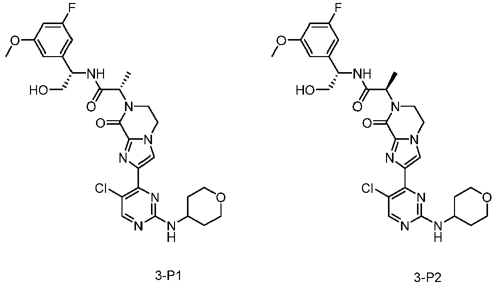

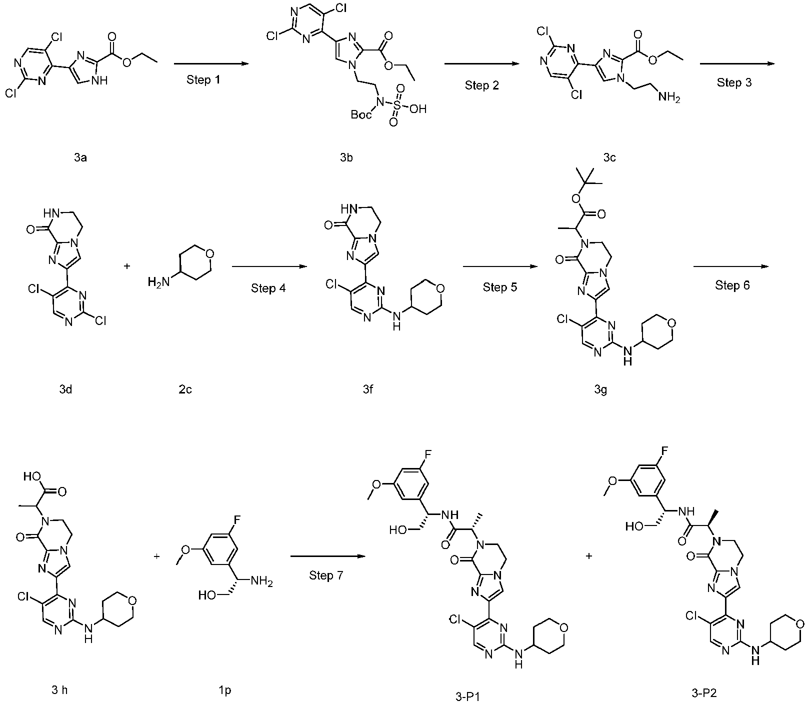

### Step 1

### (tert-Butoxycarbonyl)(2-(4-(2,5-dichloropyrimidin-4-yl)-2-(ethoxycarbonyl)-1H-imidaz ol-1-yl)ethyl)sulfamic acid 3b

Ethyl 4-(2,5-dichloropyrimidin-4-yl)-1*H*-imidazole-2-carboxylate **3a** (830 mg, 2.89 mmol, prepared by the method disclosed in the patent application "WO201780979A1, the specification, page 114, Example 31"), potassium carbonate (1.2 g, 8.68 mmol) and 18-crown-6 (0.153 g, 0.58 mmol) were dissolved in 20 mL of dioxane, followed by the addition of *tert*-butyl 2,2-dioxathiazolidine-3-carboxylate. After the addition was completed, the reaction solution was warmed up to 110°C and reacted for 14 hours. The reaction solution was cooled, filtered, and the filter cake was washed with dichloromethane. The filtrate was collected, and the filtrate was concentrated to dryness by rotary evaporation to obtain the crude title compound **3b** (1.3 g), which was directly used in the next step.

MS m/z (ESI): 510.1 [M+1].

### Step 2

### Ethyl 1-(2-aminoethyl)-4-(2,5-dichloropyrimidin-4-yl)-1H-imidazole-2-carboxylate 3c

The crude compound **3b** (1.3 g, 2.55 mmol) was dissolved in isopropanol hydrochloride solution (5 mol/L) and stirred for 1 hour. The reaction solution was concentrated to dryness by rotary evaporation to obtain the crude title compound **3c** (0.84 g), which was directly used in the next step.

MS m/z (ESI):330.1 [M+1].

### Step 3

### 2-(2,5-Dichloropyrimidin-4-yl)-6,7-dihydroimidazo[1,2-a]pyrazin-8(5H)-one 3d

The crude compound **3c** (0.84 g, 2.55 mmol) was dissolved in a methanol solution of ammonia (7 mol/L) and stirred for 14 hours. The reaction solution was concentrated, and the residues were purified by column chromatography with eluent system A to obtain the title compound **3d** (0.7 g, 2.46 mmol), yield: 59.2%.

MS m/z (ESI): 284.0 [M+1].

### Step 4

### 2-(5-Chloro-2-((tetrahydro-2H-pyran-4-yl)amino)pyrimidin-4-yl)-6,7-dihydroimidazo[1 ,2-a]pyrazin-8(5H)-one 3f

Compound **3d** (0.15 g, 0.53 mmol), compound **2c** (53.40 mg, 0.53 mmol), *N,N*-diisopropylethylamine (0.34 g, 2.64 mmol) and *N,N*-dimethylacetamide (3 mL) were mixed and reacted for 1.5 hours at 110°C in microwave reactor. The filtrate was concentrated, and the resulting residues were purified by thin layer chromatography with developing solvent system A to obtain the title compound **3f** (0.1 g, 0.29 mmol), yield: 54.0%.

MS m/z (ESI): 349.2 [M+1].

### Step 5

### tert-Butyl 2-(2-(5-chloro-2-((tetrahydro-2H-pyran-4-yl)amino)pyrimidin-4-yl)-8-oxo-5,6-dihydroi midazo[1,2-a]pyrazin-7(8H)-yl)propanoate 3g

Compound **3f** (0.1 g, 0.29 mmol) was dissoved in 5 mL of *N,N*-dimethylformamide, and the solution was cooled to 0°C. Sodium hydride (60%, 20 mg, 0.86 mmol) was added, the reaction solution was stirred for 0.5 hours. *tert*-Butyl 2-bromopropionate (89 mg, 0.43 mmol, Shanghai Bide Pharmatech Ltd.) was added, and the reaction solution was stirred for 14 hours. The filtrate was concentrated, and the residues were purified by thin layer chromatography with developing solvent system A to obtain the title compound **3g** (100 mg), yield: 73.1%.

MS m/z (ESI): 477.2 [M+1].

### Step 6

### 2-(2-(5-Chloro-2-((tetrahydro-2H-pyran-4-yl)amino)pyrimidin-4-yl)-8-oxo-5,6-dihydroi midazo[1,2-a]pyrazin-7(8H)-yl)propanoic acid 3h

Compound **3g** (100 mg, 0.21 mmol) was dissolved in 10 mL of dichloromethane, followed by the dropwise addition of 1 mL of trifluoroacetic acid. After the addition was completed, the reaction solution was stirred for 1 hour. The organic phase is concentrated to obtain the title compound **3h** (88 mg), yield: 99.0%.

MS m/z (ESI): 421.1 [M+1].

### Step 7

### (S)-2-(2-(5-Chloro-2-((tetrahydro-2H-pyran-4-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 3-P1

### (R)-2-(2-(5-Chloro-2-((tetrahydro-2H-pyran-4-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 3-P2

Compound **3h** (88 mg, 0.21 mmol) and compound**1p** (32 mg, 0.21 mmol) were dissolved in 5 mL of *N,N*-dimethylformamide, followed by the addition of 2-(7-azabenzotriazol-1-yl)-*N,N*,*N*',*N*'-tetramethyluronium hexafluorophosphate (148 mg, 0.63 mmol) and *N,N*-diisopropylethylamine (81mg, 0.63 mmol). The reaction solution was stirred for 14 hours, and concentrated under reduced pressure. The residues were purified by preparative liquid chromatography (Instrument model: Gilson 281, chromatographic column: X-Bridge, Prep 30^{∗}150 mm; 5µm; C18, mobile phase: A-water (10 mM ammonium bicarbonate), B-acetonitrile, flow rate: 30 mL /min, column temperature: room temperature) to obtain the title compounds **3-P1** and **3-P2** (2 mg, 5 mg).

### Compound in a single configuration (with shorter retention time) 3-P1

MS m/z (ESI): 588.2 [M+1]
HPLC analysis: retention time: 15.5 minutes, purity: 99.1% (chromatographic column: X-Bridge, Prep 30^{∗}150mm; 5µm; mobile phase: A-water (10 mM ammonium bicarbonate), B-acetonitrile, gradient: A 30%-45%)
¹H NMR (400 MHz, CDCl₃): 8.76 (s, 1H), 8.32 (s, 1H), 8.15 (s, 1H), 6.78-6.70 (m, 1H), 6.60(d, 1H), 6.57(d, 1H), 5.43-5.40 (m, 1H), 4.98-4.95 (m, 1H), 4.6-4.3 (m, 2H), 4.2-4.1 (m, 1H), 4.0-3.9 (m, 4H), 3.76 (s, 3H), 3.62-3.45 (m, 3H), 2.37-2.33 (m, 1H), 2.0-1.92 (m, 2H), 1.71-1.52 (m, 2H), 1.45 (d, 3H).

### Compound in a single configuration (with longer retention time) 3-P2 MS m/z (ESI): 588.2 [M+1]

HPLC analysis: retention time: 17.8 minutes, purity: 98.2% (chromatographic column: X-Bridge, Prep 30^{∗}150mm; 5µm; mobile phase: A-water (10 mM ammonium bicarbonate), B-acetonitrile, gradient: A 30%-45%)
¹H NMR (400 MHz, CDCl₃): δ 8.76 (s, 1H), 8.32 (s, 1H), 8.15 (s, 1H), 6.78-6.70 (m, 1H), 6.60 (d, 1H), 6.57 (d, 1H), 5.43-5.40 (m, 1H), 4.98-4.95 (m, 1H), 4.6-4.3 (m, 2H), 4.2-4.1 (m, 1H), 4.0-3.9 (m, 4H), 3.76 (s, 3H), 3.62-3.45 (m, 3H), 2.37-2.33 (m, 1H), 2.0-1.92 (m, 2H), 1.71-1.52 (m, 2H), 1.45 (d, 3H).

### Example 4

### (S)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-fluoro-5-methoxyphenyl)-2-hydroxyeth yl)propanamide 4-P1

### (R)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-fluoro-5-methoxyphenyl)-2-hydroxyeth yl)propanamide 4-P2

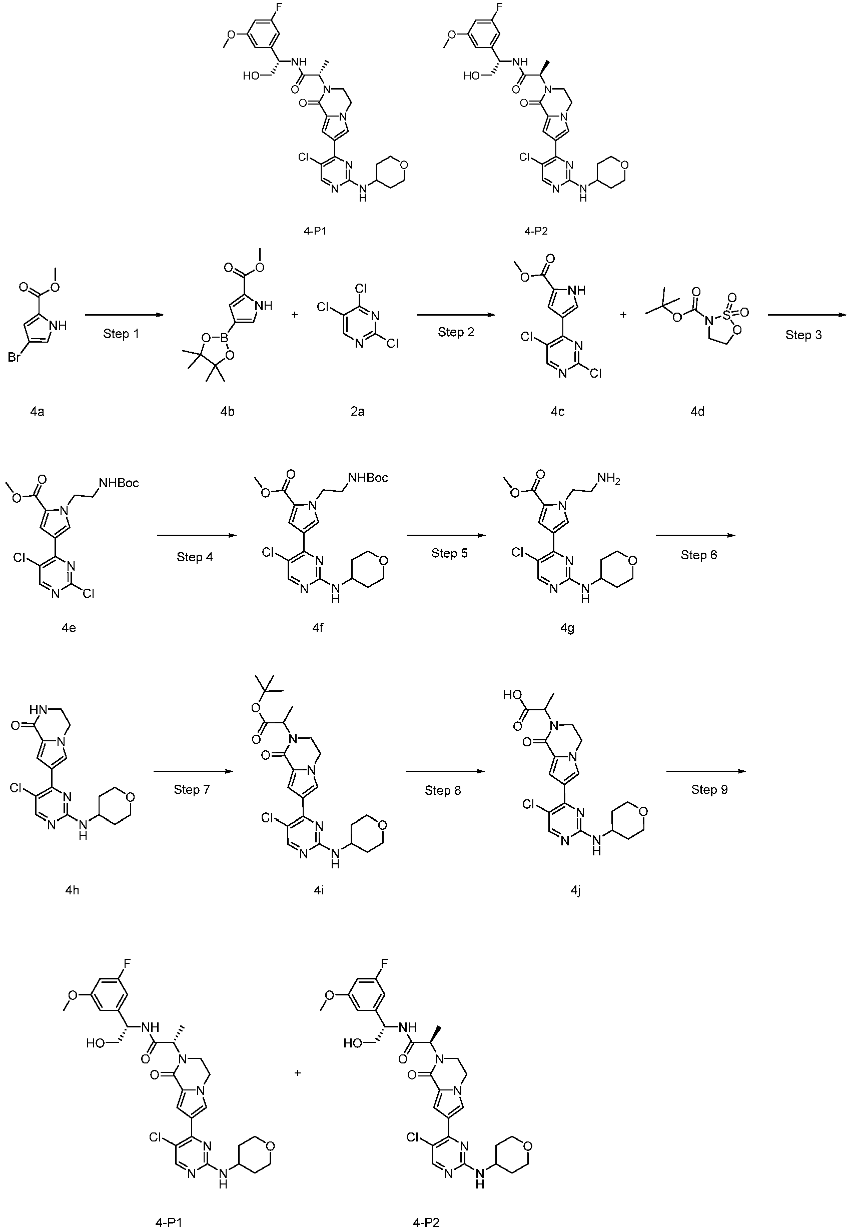

### Step 1

### Methyl 4-(4,4,5,5-tetramethyl-1,3,2-dioxaborolan-2-yl)-1H-pyrrole-2-carboxylate 4b

Methyl 4-bromo-1*H*-pyrrole-2-carboxylate **4a** (15 g, 73.52 mmol, Pharmablock Sciences (Nanjing), Inc.) was dissolved in 300 mL of 1,4-dioxane under an argon atmosphere,, followed by the addition of 4,4,4',4',5,5,5',5'-octamethyl-2,2'-bis(1,3,2-dioxaborolane) (22.41 g, 88.25 mmol, Accela ChemBio (Shanghai) Inc.), potassium acetate (14.13g, 147.03 mmol) and [1,1'-bis(diphenylphosphino)ferrocene]palladium (II) dichloride (8 g, 10.93 mmol) successively. The reaction solution was stirred at 90°C for 16 hours, cooled, and filtered through Celite. The filtrate was concentrated, and the residues were purified by thin layer chromatography with developing solvent system C to obtain the title compound **4b** (10 g), yield: 54.0%.

MS m/z (ESI): 252.2[M+1].

### Step 2

### Methyl 4-(2,5-dichloropyrimidin-4-yl)-1H-pyrrole-2-carboxylate 4c

Compound **4b** (5 g, 19.91 mmol) was dissolved in 100 mL of dioxane and 10 mL of water under an argon atmosphere, followed by the addition of compound **2a** (4 g, 21.81 mmol, Accela ChemBio (Shanghai) Inc.), sodium carbonate (4.22 g, 39.82 mmol) and [1,1'-bis(diphenylphosphino)ferrocene]palladium (II) dichloride (2.48 g, 2.98 mmol) successively. The reaction solution was stirred at 80°C for 3 hours, cooled, and filtered through Celite. The filtrate was concentrated, and the residues were purified by column chromatography with eluent system A to obtain the title compound **4c** (2.6 g), yield: 48.0%.

MS m/z (ESI): 272.1 [M+1].

### Step 3

### Methyl

### 1-(2-((tert-butoxycarbonyl)amino)ethyl)-4-(2,5-dichloropyrimidin-4-yl)-1H-pyrrole-2-c arboxylate 4e

Compound **4c** (2.2 g, 8.9 mmol) was dissolved in 20 mL of dioxane, followed by the addition of compound **4d** (1.8 g, 8.06 mmol, Accela ChemBio (Shanghai) Inc.), potassium carbonate (3.35 g, 24.23 mmol) and 18-crown-6 (427.4 mg, 1.6 mmol). The reaction solution was stirred at 110°C for 14 hours, cooled, and filtered through Celite. The filtrate was concentrated, and the residues were purified by column chromatography with eluent system A to obtain the title compound **4e** (3.2 g), yield: 95.3%.

MS m/z (ESI): 415.1[M+1].

### Step 4

### Methyl

### 1-(2-((tert-butoxycarbonyl)amino)ethyl)-4-(5-chloro-2-((tetrahydro-2H-pyran-4-yl)amin o)pyrimidin-4-yl)-1H-pyrrole-2-carboxylate 4f

Compound **4e** (1.56 g, 7.7 mmol), compound **2c** (1.56 g, 15.4 mmol, Shanghai Bide Pharmatech Ltd.), *N,N*-diisopropylethylamine (0.78g, 7.7 mmol) and *N,N*-dimethylacetamide (3 mL) were mixed and reacted for 1.5 hours at 110°C in microwave reactor. The filtrate was concentrated, and the resulting residues were purified by thin layer chromatography with developing solvent system A to obtain the title compound **4f** (1.7 g), yield: 46%.

MS m/z (ESI): 480.3 [M+1].

### Step 5

### Methyl 1-(2-aminoethyl)-4-(5-chloro-2-((tetrahydro-2H-pyran-4-yl)amino)pyrimidin-4-yl)-1H-pyrrole-2-carboxylate 4g

Compound **4f** (1.7 g, 3.54 mmol) was dissolved in 10 mL of dichloromethane, followed by the dropwise addition of 20 mL of hydrogen chloride in dioxane (4.0 M). After the addition was completed, the reaction solution was stirred for 2 hours, and concentrated under reduced pressure to obtain the crude compound **4g** (1.4 g), which was directly used in the next step without purification.

MS m/z (ESI): 380.2 [M+1].

### Step 6

### 7-(5-Chloro-2-((tetrahydro-2H-pyran-4-yl)amino)pyrimidin-4-yl)-3,4-dihydropyrrolo[1, 2-a]pyrazin-1(2H)-one 4h

The crude compound **4g** (1.4 g, 3.4 mmol) was dissolved in 10 ml of a methanol solution of ammonia (7 mol/L), and stirred for 14 hours. The reaction solution was concentrated under reduced pressure to obtain the crude compound **4h** (1.1 g), which was directly used in the next step without purification.

MS m/z (ESI): 348.1 [M+1].

### Step 7

### tert-Butyl 2-(7-(5-chloro-2-((tetrahydro-2H-pyran-4-yl)amino)pyrimidin-4-yl)-1-oxo-3,4-dihydrop yrrolo[1,2-a]pyrazin-2(1H)-yl)propanoate 4i

Compound **4h** (1.4 g, 3.16 mmol) was dissoved in 20 mL of *N,N*-dimethylformamide, and the solution was cooled to 0°C. Sodium hydride (60%, 411.4 mg, 9.5 mmol) was added, and the reaction solution was stirred for 0.5 hours. *tert*-Butyl 2-bromopropionate (992 mg, 4.74 mmol) was added, and the reaction solution was stirred for 14 hours. The filtrate was concentrated, and the residues were purified by thin layer chromatography with developing solvent system A to obtain the title compound **4i** (1.2 g), yield: 62.6%.

MS m/z (ESI): 476.2 [M+1].

### Step 8

### 2-(7-(5-Chloro-2-((tetrahydro-2H-pyran-4-yl)amino)pyrimidin-4-yl)-1-oxo-3,4-dihydro pyrrolo[1,2-a]pyrazin-2(1H)-yl)propanoic acid 4j

Compound **4i** (1.2 g, 2.52 mmol) was dissolved in 10 mL of dichloromethane, followed by the dropwise addition of 1 mL of trifluoroacetic acid. After the addition was completed, the reaction solution was stirred for 1 hour, and concentrated under reduced pressure to obtain the crude compound **4j** (1.3 g), which was directly used in the next step without purification.

MS m/z (ESI): 420.1 [M+1].

### Step 9

### (S)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-fluoro-5-methoxyphenyl)-2-hydroxyeth yl)propanamide 4-P1

### (R)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-fluoro-5-methoxyphenyl)-2-hydroxyeth yl)propanamide 4-P2

Compound **4j** (17 mg, 31.8 µmol) and **compound1p** (7.1 mg, 38.2 µmol) were dissolved in 5 mL of *N,N*-dimethylformamide, followed by the addition of2-(7-azabenzotriazol-1-yl)-*N,N,N',N'*-tetramethyluronium hexafluorophosphate (11.2 mg, 47.8 µmol) and *N,N*-diisopropylethylamine (12.4 mg, 0.1 mmol), and the reaction solution was stirred for 14 hours. The filtrate was concentrated, and the residues were purified by preparative liquid chromatography (Instrument model: Gilson 281, chromatographic column: X-Bridge, Prep 30^{∗}150 mm; 5µm; C18, mobile phase: A-water (10 mM ammonium bicarbonate), B-acetonitrile, flow rate: 30 mL/min, column temperature: room temperature) to obtain the title compounds **4-P1** and **4-P2** (5 mg, 5 mg), yield: 26.7%, 26.7%.

### Compound in a single configuration (with shorter retention time) 4-P1

MS m/z (ESI): 587.2 [M+1]
HPLC analysis: retention time: 17.6 minutes, purity: 96.8% (chromatographic column: X-Bridge, Prep 30^{∗}150mm; 5µm; mobile phase: A-water (10 mM ammonium bicarbonate), B-acetonitrile, gradient: A 23%-42%)
¹H NMR (400 MHz, CDCl₃): δ 8.21 (s, 1H), 7.74 (d, 1H), 7.67 (d, 1H), 749-7.45 (m, 2H), 6.63-6.61 (m, 2H), 6.04-5.91 (m, 1H), 5.43-5.37 (m, 3H), 5.25-5.22 (m, 1H), 5.03-5.01 (m, 1H), 4.17-4.15 (m, 2H), 4.05-4.02 (m, 2H), 3.92-3.87 (m, 2H), 3.72 (s, 3H), 3.60-3.58 (m, 3H), 2.12-2.03 (m, 4H), 1.45 (d, 3H).

### Compound in a single configuration (with longer retention time) 4-P2

MS m/z (ESI): 587.2 [M+1]
HPLC analysis: retention time: 19.1 minutes, purity: 98.2% (chromatographic column: X-Bridge, Prep 30^{∗}150mm; 5µm; mobile phase: A-water (10 mM ammonium bicarbonate), B-acetonitrile, gradient: A 23%-42%)
¹H NMR (400 MHz, CDCl₃): δ 8.21 (s, 1H), 7.67 (d, 1H), 7.56 (d, 1H), 748-7.45 (m, 2H), 6.71-6.67 (m, 2H), 6.59-6.51 (m, 1H), 5.40-5.37 (m, 3H), 5.20-5.18 (m, 1H), 5.08-5.06 (m, 1H), 4.17-4.10 (m, 2H), 4.05-4.02 (m, 2H), 3.89-3.83 (m, 3H), 3.82 (s, 3H), 3.62-3.57 (m, 2H), 2.32-2.23 (m, 4H), 1.47 (d, 3H).

### Example 5

### 2-(2-(5-Chloro-2-((tetrahydro-2H-pyran-4-yl)amino)pyrimidin-4-yl)-8-oxo-5,6-dihydroi midazo[1,2-a]pyrazin-7(8H)-yl)-N-((S)-2-hydroxy-1-(m-tolyl)ethyl)propanamide 5

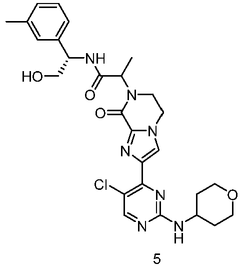

In accordance with the synthetic route in Example 3, the starting compound **1p** in Step 7 was replaced with (*S*)-2-amino-2-(*m*-tolyl)ethanol (Shanghai Bide Pharmatech Ltd.), to obtain the title compound **5** (12 mg).
MS m/z (ESI): 554.2 [M+1].
1H NMR (400 MHz, CDCl₃): δ 8.76 (s, 1H), 8.32 (s, 1H), 8.15 (s, 1H), 6.79 (s, 1 H), 6.60-6.59 (d, 1H), 6.56 (d, 1H), 5.43-5.39 (m, 1H), 4.97-4.94 (m, 1H), 4.6-4.5 (m, 1H), 4.5-4.4 (m, 1H), 4.15-4.05 (m, 1H), 4.05-3.9 (m, 4H), 3.9-3.7 (m, 2H), 3.6-3.4 (m, 2H), 2.05-1.95 (m, 2H), 1.63-1.53 (m, 2H), 1.44 (d, 3H), 1.28 (s, 3H).

### Example 6

### (S)-2-(6-(5-Chloro-2-((tetrahydro-2H-pyran-4-yl)amino)pyrimidin-4-yl)-3-oxo-1H-pyrr olo[1,2-c]imidazol-2(3H)-yl)-N-((S)-1-(3-fluoro-5-methoxyphenyl)-2-hydroxyethyl)pro panamide 6-P1

### (R)-2-(6-(5-Chloro-2-((tetrahydro-2H-pyran-4-yl)amino)pyrimidin-4-yl)-3-oxo-1H-pyrrolo[1,2-c]imidazol-2(3H)-yl)-N-((S)-1-(3-fluoro-5-methoxyphenyl)-2-hydroxyethyl )propanamide 6-P2

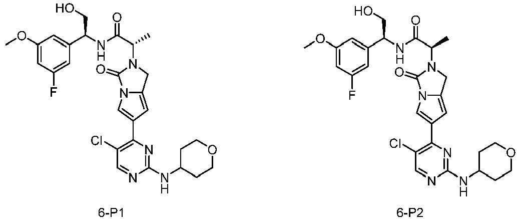

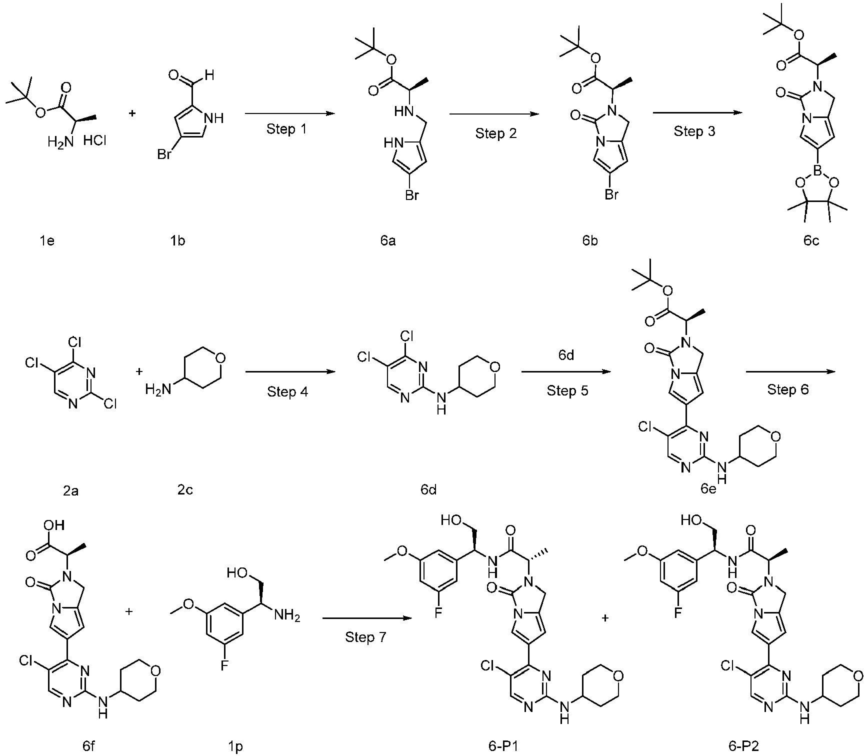

### Step 1

### tert-Butyl ((4-bromo-1H-pyrrol-2-yl)methyl)-D-alaninate 6a

Compound **1e** (417 mg, 2.30 mmol, Shanghai Bide Pharmatech Ltd.) was dissolved in methanol (5 mL), followed by the addition of anhydrous potassium carbonate (476 mg, 3.44 mmol), and the mixture was stirred for 30 minutes and filtered to remove insoluble matter. Compound **1b** (500 mg, 2.87 mmol) was added, and the reaction solution was cooled to 0°C. Sodium borohydride (108 mg, 2.87 mmol) was added, and the reaction solution was stirred for 2 hours. Water was added, and the reaction solution was extracted with ethyl acetate (10 mL×2), dried with anhydrous sodium sulfate, concentrated under reduced pressure, and purifed by column chromatography with eluent system B to obtain the title compound 6a (600 mg), yield: 68.0%.

MS m/z (ESI): 303.1 [M+1].

### Step 2

### tert-Butyl (R)-2-(6-bromo-3-oxo-1H-pyrrolo[1,2-c]imidazol-2(3H)-yl)propanoate 6b

Compound **6a** (800 mg, 2.64 mmol) and *N*,*N*'-carbonyldiimidazole (513 mg, 3.16 mmol) were dissolved in 60 mL of tetrahydrofuran, and the reaction solution was stirred for 0.5 hours. Sodium hydride (60%, 121 mg, 3.16 mmol) was added, and the reaction solution was stirred for 14 hours. Water was added, and the reaction solution was concentrated under reduced pressure. The residues were purified by column chromatography with eluentsystem B to obtain the title compound **6b** (400 mg), yield: 46.0%.

MS m/z (ESI): 329.1 [M+1].

### Step 3

### tert-Butyl (R)-2-(3-oxo-6-(4,4,5,5-tetramethyl-1,3,2-dioxaborolan-2-yl)-1H-pyrrolo[1,2-c]imidazo 1-2(3H)-yl)propanoate 6c

Compound 6b (200 mg, 0.61 mmol) was dissolved in 3 mL of 1,4-dioxane under an argon atmosphere, followed by the addition of 4,4,4',4',5,5,5',5'-octamethyl-2,2'-bis(1,3,2-dioxaborolane) (184 mg, 0.72 mmol), potassium acetate (119 mg, 1.21 mmol) and [1,1'-bis(diphenylphosphino)ferrocene]palladium (II) dichloride (44 mg, 0.06 mmol) successively. The reaction solution was stirred at 90°C for 2 hours, cooled, and filtered through Celite. The filtrate was concentrated, and the residues were purified by thin layer chromatography with developing solvent system C to obtain the title compound **6c** (100 mg), yield: 44.0%.

MS m/z (ESI): 377.2[M+1].

### Step 4

### 4,5-Dichloro-N-(tetrahydro-2H-pyran-4-yl)pyrimidin-2-amine 6d

Compound **2a** (1 g, 5.45 mmol), compound **2c** (551 mg, 5.45 mmol), *N,N-*diisopropylethylamine (2 g, 15.47 mmol) and 2 mL of *N,N-*dimethylacetamide were reacted at 140°C for 0.5 hours. The reaction solution was cooled and concentrated under reduced pressure. The residues were purified by column chromatography with eluent system C to obtain the title compound **6d** (120 mg), yield: 8.8%.

MS m/z (ESI): 248.1[M+1].

### Step 5

### tert-Butyl (R)-2-(6-(5-chloro-2-((tetrahydro-2H-pyran-4-yl)amino)pyrimidin-4-yl)-3-oxo-1H-pyrr olo[1,2-c]imidazol-2(3H)-yl)propanoate 6e

Compound **6c** (100 mg, 0.265 mmol) was dissolved in 50 mL of dioxane under an argon atmosphere, followed by the addition of compound **6d** (60 mg, 0.242 mmol), potassium carbonate (67 mg, 0.485 mmol) and [1,1'-bis(diphenylphosphino)ferrocene]palladium (II) dichloride (18 mg, 0.025 mmol) successively. The reaction solution was reacted for 1.5 hours at 85°C in microwave reactor, cooled, and filtered through Celite. The filtrate was concentrated, and the residues were purified by column chromatography with eluent system A to obtain the title compound **6e** (50 mg), yield: 45.0%.

MS m/z (ESI): 462.2[M+1].

### Step 6

### (R)-2-(6-(5-Chloro-2-((tetrahydro-2H-pyran-4-yl)amino)pyrimidin-4-yl)-3-oxo-1H -pyrrolo[1,2-c]imidazol-2(3H)-yl)propanoic acid 6f

Compound **6e** (60 mg, 0.14 mmol) was dissolved in 3 mL of dichloromethane, followed by the dropwise addition of 0.5 mL of trifluoroacetic acid. After the addition was completed, the reaction solution was stirred for 2 hours, and concentrated under reduced pressure to obtain the crude compound **6f** (52 mg), which was directly used in the next step without purification.

MS m/z (ESI): 406.2[M+1].

### Step 7

### (S)-2-(6-(5-Chloro-2-((tetrahydro-2H-pyran-4-yl)amino)pyrimidin-4-yl)-3-oxo-1H-pyrr olo[1,2-c]imidazol-2(3H)-yl)-N-((S)-1-(3-fluoro-5-methoxyphenyl)-2-hydroxyethyl)pro panamide 6-P1

### (R)-2-(6-(5-Chloro-2-((tetrahydro-2H-pyran-4-yl)amino)pyrimidin-4-yl)-3-oxo-1H-pyrrolo[1,2-c]imidazol-2(3H)-yl)-N-((S)-1-(3-fluoro-5-methoxyphenyl)-2-hydroxyethyl )propanamide 6-P2

The cruded compound **6f** (52 mg, 0.013 mmol) and compound **1p** (35 mg, 0.019 mmol) were dissolved in 5 mL of *N,N*-dimethylformamide, followed by the addition of 2-(7-azabenzotriazol-1-yl)-*N,N,N*'*,N*'-tetramethyluronium hexafluorophosphate (45 mg, 0.19 mmol) and *N,N*-diisopropylethylamine (82 mg, 0.63 mmol). The reaction solution was stirred for 1.5 hours, and concentrated under reduced pressure. The residues were purified by preparative high performance liquid chromatography to obtain the compounds **6-P1** and **6-P2** (12 mg, 12 mg), yield: 16.3%, 16.3%.

### Compound in a single configuration (with shorter retention time) 6-P1

MS m/z (ESI): 573.3[M+1]
HPLC analysis: retention time: 17.1 minutes, purity: 97.9% (chromatographic column: X-Bridge, Prep 30^{∗}150mm; 5µm; mobile phase: A-water (10 mM ammonium bicarbonate), B-acetonitrile, gradient: A 5%-95%)
¹H NMR (400 MHz, CD₃OD): δ 8.20 (s, 1H), 8.04 (s, 1H), 6.90 (s, 1H), 6.73 (s, 1H), 6.68 (d, 1H), 6.55 (d, 1H), 4.78-4.65 (m, 3H), 4.01-3.98 (m, 3H), 3.80-3.72 (m, 6H), 3.59-3.57 (m, 2H), 2.02-1.96 (m, 2H), 1.65-1.55 (m, 5H).

### Compound in a single configuration (with longer retention time) 6-P2

MS m/z (ESI): 573.3[M+1]
HPLC analysis: retention time: 18.5 minutes, purity: 98.2% (chromatographic column: X-Bridge, Prep 30^{∗}150mm; 5µm; mobile phase: A-water (10 mM ammonium bicarbonate), B-acetonitrile, gradient: A 25%-45%)
¹H NMR (400 MHz, CD₃OD): δ 8.19 (s, 1H), 8.05 (s, 1H), 6.90 (s, 1H), 6.74 (s, 1H), 6.64 (d, 1H), 6.59 (d, 1H), 4.73-4.69 (m, 3H), 4.00-3.97 (m, 3H), 3.80-3.72 (m, 6H), 3.58-3.53 (m, 2H), 2.02-1.96 (m, 2H), 1.65-1.55 (m, 5H).

### Example 7

### (S)-2-(6-(5-Chloro-2-((1-methyl-1H-pyrazol-5-yl)amino)pyrimidin-4-yl)-3-oxo-1H-pyrr olo[1,2-c]imidazol-2(3H)-yl)-N-((S)-1-(3-fluoro-5-methoxyphenyl)-2-hydroxyethyl)pro panamide 7-P1

### (R)-2-(6-(5-Chloro-2-((1-methyl-1H-pyrazol-5-yl)amino)pyrimidin-4-yl)-3-oxo-1H-pyrr olo[1,2-c]imidazol-2(3H)-yl)-N-((S)-1-(3-fluoro-5-methoxyphenyl)-2-hydroxyethyl)pro panamide 7-P2

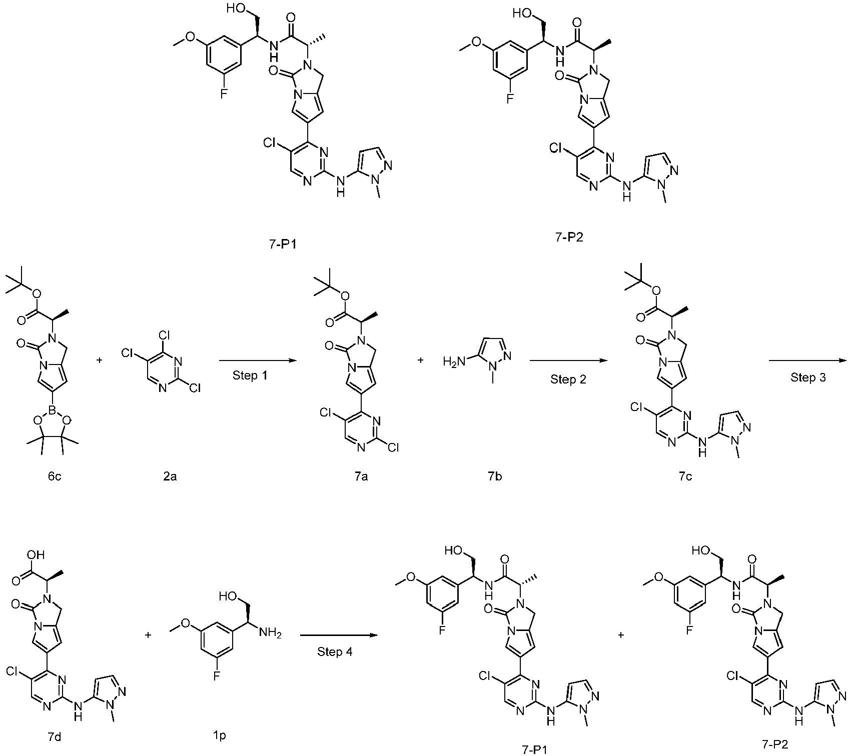

### Step 1

### tert-Butyl (R)-2-(6-(2,5-dichloropyrimidin-4-yl)-3-oxo-1H-pyrrolo[1,2-c]imidazol-2(3H)-yl)propa noate 7a

Compound **6c** (1.2 g, 3.2 mmol )was dissolved in 30 mL of dioxane and 6 mL of water under an argon atmosphere, followed by the addition of compound **2a** (702 mg, 3.83 mmol, Shanghai Bide Pharmatech Ltd.), sodium carbonate (676 mg, 6.37 mmol) and [1,1'-bis(diphenylphosphino)ferrocene]palladium (II) dichloride (233 mg, 0.32 mmol) successively. The reaction solution was reacted at 80°C for 14 hours, cooled, and filtered through Celite. The filtrate was concentrated, and the residues were purified by column chromatography with eluent system A to obtain the title compound **7a** (400mg), yield: 31.5%.

MS m/z (ESI): 397.2 [M+1].

### Step 2

### tert-Butyl (R)-2-(6-(5-chloro-2-((1-methyl-1H-pyrazol-5-yl)amino)pyrimidin-4-yl)-3-oxo-1H-pyrr

### olo[1,2-c]imidazol-2(3H)-yl)propanoate 7c

Compound **7a** (400 mg, 1.01 mmol) was dissolved in 15 mL of 1,4-dioxane under an argon atmosphere, followed by the addition of tris(dibenzylideneacetone)dipalladium (92 mg, 0.1 µmol), 4,5-bis(diphenylphosphino)-9,9-dimethylxanthene (117 mg, 0.2 µmol), cesium carbonate (656 mg, 2.01 mmol) and 1-methyl-5-aminopyrazole **7b** (147 mg, 1.51 mmol, Shanghai Bide Pharmatech Ltd.). The reaction solution was stirred at 100°C for 14 hours, cooled, and filtered through Celite. The filtrate was concentrated, and the residues were purified by thin layer chromatography with developing solvent system A to obtain the title compound **7c** (180 mg), yield: 39.0%.

MS m/z (ESI): 458.2 [M+1].

### Step 3

### (R)-2-(6-(5-Chloro-2-((1-methyl-1H-pyrazol-5-yl)amino)pyrimidin-4-yl)-3-oxo-1 H-pyrrolo[1,2-c]imidazol-2(3H)-yl)propanoic acid 7d

Compound **7c** (180 mg, 0.39 mmol) was dissolved in 3 mL of dichloromethane, followed by the dropwise addition of 0.5 mL of trifluoroacetic acid. After the addition was completed, the reaction solution was stirred for 2 hours, and concentrated under reduced pressure to obtain the crude compound **7d** (157mg), which was directly used in the next step without purification.

MS m/z (ESI): 402.2[M+1].

### Step 4

### (S)-2-(6-(5-Chloro-2-((1-methyl-1H-pyrazol-5-yl)amino)pyrimidin-4-yl)-3-oxo-1H-pyrr olo[1,2-c]imidazol-2(3H)-yl)-N-((S)-1-(3-fluoro-5-methoxyphenyl)-2-hydroxyethyl)pro panamide 7-P1

### (R)-2-(6-(5-Chloro-2-((1-methyl-1H-pyrazol-5-yl)amino)pyrimidin-4-yl)-3-oxo-1H -pyrrolo[1,2-c]imidazol-2(3H)-yl)-N-((S)-1-(3-fluoro-5-methoxyphenyl)-2-hydroxyethy l)propanamide 7-P2

The crude compound **7d** (158 mg, 0.39 mmol) and compound **1p** (73 mg, 0.39 mmol) were dissolved in 5 mL of *N*,*N*-dimethylformamide, followed by the addition of 2-(7-azabenzotriazol-l-yl)-*N,N,N*',*N*'-tetramethyluronium hexafluorophosphate (179 mg, 0.47 mmol) and *N,N*-diisopropylethylamine (152 mg, 1.18 mmol). The reaction solution was stirred for 14 hours, and concentrated under reduced pressure. The residues were purified by preparative high performance liquid chromatography to obtain the compounds **7-P1** and **7-P2** (20 mg, 20 mg), yield: 9%, 9%.

### Compound in a single configuration (with shorter retention time) 7-P1

MS m/z (ESI): 569.1[M+1]
HPLC analysis: retention time: 12.4 minutes, purity: 98.2% (chromatographic column: X-Bridge, Prep 30^{∗}150 mm; 5µm; mobile phase: A-water (10 mM ammonium bicarbonate), B-acetonitrile, gradient: A 30%-46%)
¹H NMR (400 MHz, CD₃OD): δ 8.37 (s, 1H), 8.08 (s, 1H), 7.45 (d, 1H), 6.85 (s, 1H), 6.67-6.66 (m, 2H), 6.56-6.53 (m, 1H), 6.33 (d, 1H), 4.86-4.84 (m, 1H), 4.60 (d, 2H), 3.77-3.72 (m, 9H), 1.61 (d, 3H).

### Compound in a single configuration (with longer retention time) 7-P2

MS m/z (ESI): 569.1[M+1]
HPLC analysis: retention time: 14.5 minutes, purity: 97.2% (chromatographic column: X-Bridge, Prep 30^{∗}150mm; 5µm; mobile phase: A-water (10 mM ammonium bicarbonate), B-acetonitrile, gradient: A 30%-46%)
¹H NMR (400 MHz, CD₃OD): δ 8.37 (s, 1H), 8.10 (s, 1H), 7.44 (d, 1H), 6.75 (s, 1H), 6.68-6.66 (m, 2H), 6.56-6.53 (m, 1H), 6.33 (d, 1H), 4.86-4.84 (m, 1H), 4.60 (d, 2H), 3.80-3.74 (m, 9H), 1.56 (d, 3H).

### Example 8

### (S)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-3-oxo-1H-pyrrolo[1,2-c]imidazol-2(3H)-yl)pro panamide 8-P1

### (R)-N-((S)-1-(3-Fluoro-5-methoxyphenyl)-2-hydroxyethyl)-2-(6-(5-methyl-2-((1-methyl -1H-pyrazol-5-yl)amino)pyrimidin-4-yl)-3-oxo-1H-pyrrolo[1,2-c]imidazol-2(3H)-yl)pro panamide 8-P2

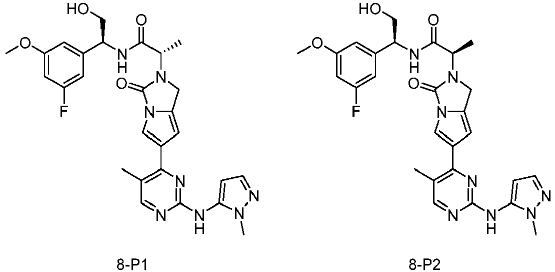

In accordance with the synthetic route in Example 3, the starting compound 2a in Step 4 was replaced with compound **1j** to obtain the compounds **8-P1** and **8-P2** (5 mg, 5 mg).

### Compound in a single configuration (with longer retention time) 8-P1

MS m/z (ESI): 549.2 [M+1]
HPLC analysis: retention time: 13.1 minutes, purity: 98.5% (chromatographic columnX-Bridge, Prep 30^{∗}150 mm; 5µm; mobile phase: A-water (10 mM ammonium bicarbonate), B-acetonitrile, gradient: A 22%-40%)
¹H NMR (400 MHz, CDCl₃): δ 8.10 (s, 1H), 7.69 (s, 1H), 7.58 (s, 1H), 6.85-6.81 (m, 3H), 6.66-6.63 (m, 1H), 6.44 (s, 1H), 5.05-5.02 (m, 1H), 4.75 (d, 1H), 4.56-4.50 (m, 2H), 3.99-3.98 (m, 2H), 3.97 (s, 3H), 3.80 (s, 3H), 2.43 (s, 3H), 1.35 (d, 3H).

### Compound in a single configuration (with longer retention time) 8-P2

MS m/z (ESI): 549.2 [M+1]
HPLC analysis: retention time: 15.5 minutes, purity: 97.9% (chromatographic column: X-Bridge, Prep 30^{∗}150mm; 5µm; mobile phase: A-water (10 mM ammonium bicarbonate), B-acetonitrile, gradient: A22%-40%)
¹H NMR (400 MHz, CD₃OD): *δ* 8.24 (s, 1H), 7.77 (s, 1H), 7.59 (s, 1H), 6.78-6.75 (m, 2H), 6.70-6.59 (m, 2H), 6.49 (s, 1H), 4.95-4.93 (m, 1H), 4.86(d, 1H), 4.73 (d, 1H), 4.58 (d, 1H), 3.81-3.73 (m, 8H), 2.43 (s, 3H), 1.56 (d, 3H).

### Example 9

### (S)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-2-hydroxy-1-(m-tolyl)ethyl)propanamide 9-P1

### (R)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-2-hydroxy-1-(m-tolyl)ethyl)propanamide 9-P2

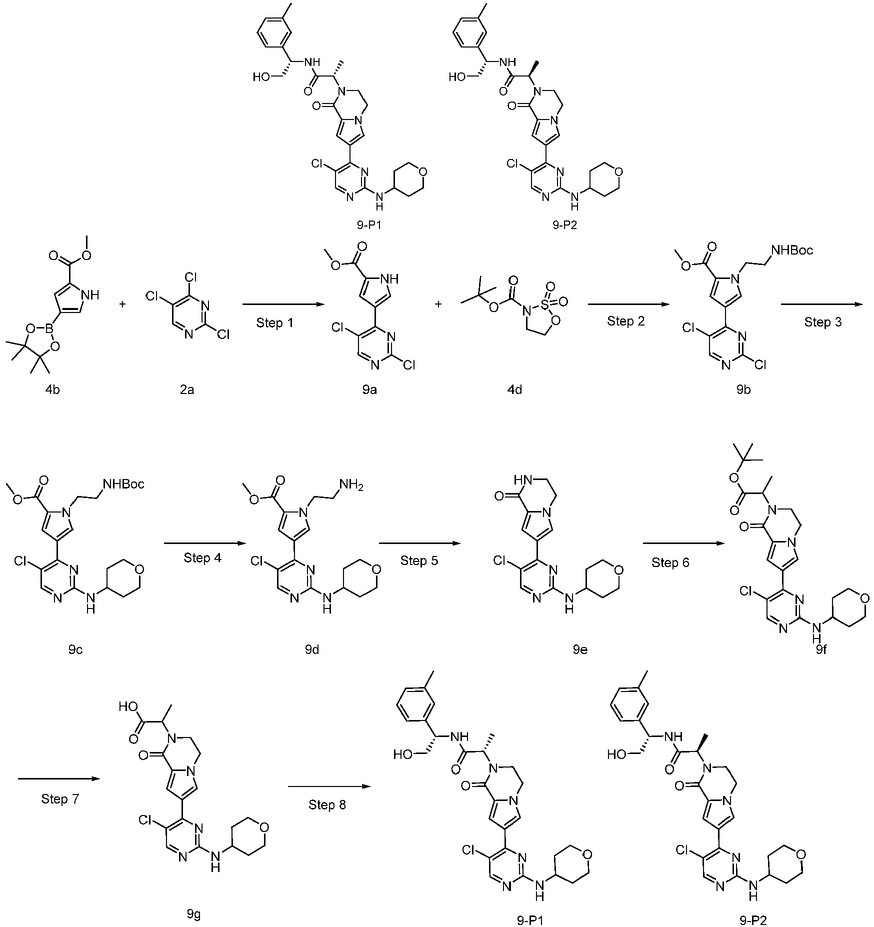

### Step 1

Methyl 4-(2,5-dichloropyrimidin-4-yl)-1*H*-pyrrole-2-carboxylate**9a** Compound 4b (5 g, 19.92 mmol) was dissolved in 100 mL of dioxane and 10 mL of water under an argon atmosphere, followed by the addition of compound 2a (4.38 g, 23.88 mmol), sodium carbonate (3.16 g, 29.81 mmol) and [1,1'-bis(diphenylphosphino)ferrocene]palladium (II) dichloride (2.49 g, 2.99 mmol) successively. The reaction solution was reacted at 80°C for 3 hours, cooled, and filtered through Celite. The filtrate was concentrated, and the residues were purified by column chromatography with eluent system A to obtain the title compound **9a** (3 g), yield: 55.3%.

MS m/z (ESI): 272.0 [M+1].

### Step 2

### Methyl 1-(2-((tert-butoxycarbonyl)amino)ethyl)-4-(2,5-dichloropyrimidin-4-yl)-1H-pyrrole-2-c arboxylate 9b

Compound **9a** (2.2 g, 8.9 mmol) were dissolved in 20 mL of dioxane, followed by the addition of compound **4d** (1.8 g, 8.06 mmol), potassium carbonate (3.35 g, 24.23 mmol) and 18-crown-6 (427.4 mg, 1.6 mmol) successively. The reaction solution was stirred at 110°C for 14 hours, cooled, and filtered through Celite. The filtrate was concentrated, and the residues were purified by column chromatography with eluent system A to obtain the title compound **9b** (3.2 g), yield: 95.3%.

MS m/z (ESI): 415.1 [M+1].

### Step 3

### Methyl 1-(2-((tert-butoxycarbonyl)amino)ethyl)-4-(5-chloro-2-((tetrahydro-2H-pyran-4-yl)amin o)pyrimidin-4-yl)-1H-pyrrole-2-carboxylate 9c

Compound **9b** (1.56 g, 7.7 mmol), compound **2c** (1.56 g, 15.4 mmol), *N,N*-diisopropylethylamine (0.78g, 7.7 mmol) and *N,N-*dimethylacetamide (3 mL) were mixed and reacted for 1.5 hours at 110°C in microwave reactor. The filtrate was concentrated, and the resulting residues were purified by thin layer chromatography with developing solvent system A to obtain the title compound **9c** (1.7 g), yield: 46%.

MS m/z (ESI): 480.3 [M+1].

### Step 4

### Methyl 1-(2-aminoethyl)-4-(5-chloro-2-((tetrahydro-2H-pyran-4-yl)amino)pyrimidin-4-yl)-1H-pyrrole-2-carboxylate 9d

Compound **9c** (1.7 g, 3.54 mmol) was dissolved in 10 mL of dichloromethane, followed by the dropwise addition of 20 mL of hydrogen chloride in dioxane (4.0 M). After the addition was completed, the reaction solution was stirred for 2 hours, and concentrated under reduced pressure to obtain the crude compound **9d** (1.4 g), which was directly used in the next step without purification.

MS m/z (ESI): 380.2 [M+1].

### Step 5

### 7-(5-Chloro-2-((tetrahydro-2H-pyran-4-yl)amino)pyrimidin-4-yl)-3,4-dihydropyrrolo[1, 2-a]pyrazin-1(2H)-one 9e

The crude compound **9d** (1.4 g, 3.4 mmol) was dissolved in 10 mL of a methanol solution of ammonia (7 mol/L), and stirred for 14 hours. The reaction solution was concentrated under reduced pressure to obtain the crude compound **9e** (1.1 g), which was directly used in the next step without purification.

MS m/z (ESI): 348.1 [M+1].

### Step 6

### tert-Butyl 2-(7-(5-chloro-2-((tetrahydro-2H-pyran-4-yl)amino)pyrimidin-4-yl)-1-oxo-3,4-dihydrop yrrolo[1,2-a]pyrazin-2(1H)-yl)propanoate 9f

Compound **9e** (1.4 g, 3.16 mmol) was dissoved in 20 mL of *N,N*-dimethylformamide, and the solution was cooled to 0°C. Sodium hydride (60%, 411.4 mg, 9.5 mmol) was added, the reaction solution was stirred for 0.5 hours. *tert*-Butyl 2-bromopropionate (992 mg, 4.74 mmol) was added, and the reaction solution was stirred for 14 hours. The filtrate was concentrated, and the residues were purified by thin layer chromatography with developing solvent system A to obtain the title compound **9f** (1.2 g), yield: 62.6%.

MS m/z (ESI): 476.2 [M+1].

### Step 7

### 2-(7-(5-Chloro-2-((tetrahydro-2H-pyran-4-yl)amino)pyrimidin-4-yl)-1-oxo-3,4-dihydro pyrrolo[1,2-a]pyrazin-2(1H)-yl)propanoic acid 9g

Compound **9f** (1.2 g, 2.52 mmol) was dissolved in 10 mL of dichloromethane, followed by the dropwise addition of 1 mL of trifluoroacetic acid. After the addition was completed, the reaction solution was stirred for 1 hour, and concentrated under reduced pressure to obtain the crude compound **9g** (1.3 g), which was directly used in the next step without purification.

MS m/z (ESI): 420.1 [M+1].

### Step 8

### (S)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-2-hydroxy-1-(m-tolyl)ethyl)propanamide 9-P1

### (R)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-2-hydroxy-1-(m-tolyl)ethyl)propanamide 9-P2

Compound **9g** (120 mg, 0.25 mmol) and compound (*S*)-2-amino-2-(*m*-tolyl)ethanol (44.2 mg, 0.29 mmol) were dissolved in 5 mL of *N,N*-dimethylformamide, followed by the addition of 2-(7-azabenzotriazol-1-yl)-*N,N,N*',*N*'-tetramethyluronium hexafluorophosphate (79.33 mg, 0.37 mmol) and *N,N*-diisopropylethylamine (72.6 mg, 0.56 mmol). The reaction solution was stirred for 14 hours, and concentrated under reduced pressure. The residues were purified by preparative high performance liquid chromatography to obtain the compounds **9-P1** and **9-P2** (20 mg, 20 mg), yield: 16%, 16%.

### Compound in a single configuration (with shorter retention time) 9-P1

MS m/z (ESI): 553.2[M+1]
HPLC analysis: retention time: 11.1 minutes, purity: 98.2% (chromatographic column: X-Bridge, Prep 30^{∗}150mm; 5µm; mobile phase: A-water (10 mM ammonium bicarbonate), B-acetonitrile, gradient: A 40%-60%)
¹H NMR (400 MHz, CD₃OD): *δ* 8.17 (s, 1H), 7.90 (s, 1H), 7.71 (s, 1H), 7.19-7.04 (m, 4H), 5.35-5.41 (m, 1H), 4.97-4.94 (m, 1H), 4.39-4.29 (m, 2H), 4.02-3.99 (m, 3H), 3.87-3.84 (m, 2H), 3.77-3.74 (m, 2H), 3.65-3.58(m, 2H), 2.25 (s, 3H), 2.03-2.01 (m, 2H), 1.66-1.59 (m, 2H), 1.48 (d, 3H).

### Compound in a single configuration (with longer retention time) 9-P2

MS m/z (ESI): 553.2[M+1]
HPLC analysis: retention time: 12.5 minutes, purity: 97.2% (chromatographic column: X-Bridge, Prep 30^{∗}150mm; 5µm; mobile phase: A-water (10 mM ammonium bicarbonate), B-acetonitrile, gradient: A 40%-60%)
¹H NMR (400 MHz, CD₃OD): *δ* 8.18 (s, 1H), 7.93 (s, 1H), 7.71 (s, 1H), 7.24-7.08 (m, 4H), 5.38-5.33 (m, 1H), 4.98-4.95 (m, 1H), 4.39-4.29 (m, 2H), 4.02-3.99 (m, 3H), 3.87-3.84 (m, 2H), 3.76-3.73 (m, 2H), 3.62-3.56 (m, 2H), 2.35 (s, 3H), 2.03-2.01 (m, 2H), 1.67-1.58 (m, 2H), 1.48 (d, 3H).

### Example 10

### (S)-N-((S)-2-Hydroxy-1-(m-tolyl)ethyl)-2-(7-(5-methyl-2-((1-methyl-1H-pyrazol-5-yl)a mino)pyrimidin-4-yl)-1-oxo-3,4-dihydropyrrolo[1,2-a]pyrazin-2(1H)-yl)propanamide 10-P1

### (R)-N-((S)-2-Hydroxy-1-(m-tolyl)ethyl)-2-(7-(5-methyl-2-((1-methyl-1H-pyrazol-5-yl)a mino)pyrimidin-4-yl)-1-oxo-3,4-dihydropyrrolo[1,2-a]pyrazin-2(1H)-yl)propanamide 10-P2

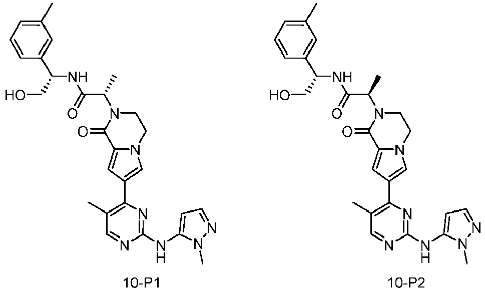

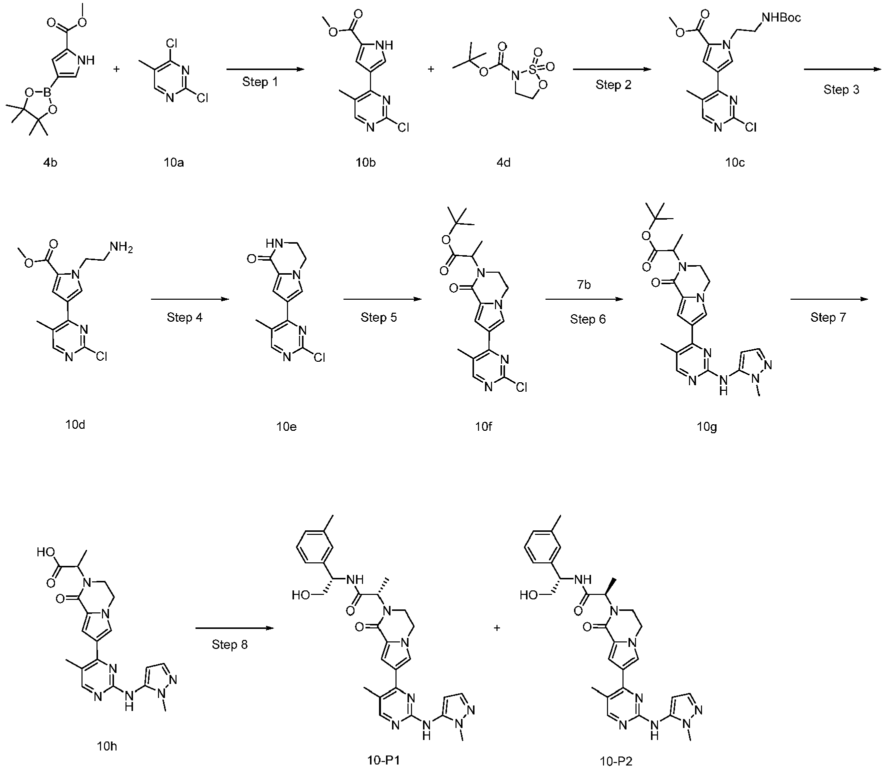

### Step 1

Methyl 4-(2-chloro-5-methylpyrimidin-4-yl)-1*H*-pyrrole-2-carboxylate **10b** Compound **4b** (12 g, 47.79 mmol) was dissolved in 100 mL of dioxane and 10 mL of water under an argon atmosphere, followed by the addition of compound **10a** (8.6 g, 52.51 mmol, Accela ChemBio (Shanghai) Inc.), sodium carbonate (10.1 g, 95.58 mmol) and [1,1'-bis(diphenylphosphino)ferrocene]palladium (II) dichloride (5.85 g, 7.18 mmol) successively. The reaction solution was reacted at 80°C for 3 hours, cooled, and filtered through Celite. The filtrate was concentrated, and the residues were purified by column chromatography with eluent system A to obtain the title compound **10b** (2 g), yield: 16.6%.

MS m/z (ESI): 252.1[M+1],

### Step 2

### Methyl 1-(2-((tert-butoxycarbonyl)amino)ethyl)-4-(2-chloro-5-methylpyrimidin-4-yl)-1H-pyrro le-2-carboxylate 10c

Compound **10b** (1g, 3.97 mmol) were dissolved in 20 mL of dioxane, followed by the addition of compound **4d** (887 mg, 3.97 mmol), potassium carbonate (1.65 g, 11.9 mmol) and 18-crown-6 (210 mg, 0.79 mmol) successively. The reaction solution was stirred at 110°C for 14 hours, cooled, and filtered through Celite. The filtrate was concentrated, and the residues were purified by column chromatography with eluent system A to obtain the title compound **10c** (800 mg), yield: 51%.

MS m/z (ESI): 395.1[M+1].

### Step 3

### Methyl 1-(2-aminoethyl)-4-(2-chloro-5-methylpyrimidin-4-yl)-1H-pyrrole-2-carboxylate 10d

Compound **10c** (800 mg, 2 mmol) was dissolved in 3 mL of dichloromethane, followed by the dropwise addition of 5 mL of hydrogen chloride in dioxane (4.0 M). After the addition was completed, the reaction solution was stirred for 2 hours, and concentrated under reduced pressure to obtain the crude compound **10d** (638 mg), which was directly used in the next step without purification.

MS m/z (ESI): 295.1 [M+1].

### Step 4

### 7-(2-Chloro-5-methylpyrimidin-4-yl)-3,4-dihydropyrrolo[1,2-a]pyrazin-l(2H)-one 10e

The crude compound **10d** (600 mg, 2.04 mmol) was dissolved in 10 mL of a methanol solution of ammonia (7 mol/L), and stirred for 14 hours. The reaction solution was concentrated under reduced pressure to obtain the crude compound **10e** (534 mg), which was directly used in the next step without purification.

MS m/z (ESI): 263.2 [M+1].

### Step 5

### tert-Butyl 2-(7-(2-chloro-5-methylpyrimidin-4-yl)-1-oxo-3,4-dihydropyrrolo[1,2-a]pyrazin-2(1H)-yl)propanoate 10f

Compound **10e** (0.6 g, 2.28 mmol) was dissoved in 5 mL of *N,N*-dimethylformamide, and the solution was cooled to 0°C. Sodium hydride (60%, 262 mg, 6.84 mmol) was added, the reaction solution was stirred for 0.5 hours. *tert*-Butyl 2-bromopropionate (955 mg, 4.57 mmol) was added, and the reaction solution was stirred for 14 hours. The filtrate was concentrated, and the residues were purified by thin layer chromatography with developing solvent system A to obtain the title compound **10f** (600 mg), yield: 67.2%.

MS m/z (ESI): 391.1 [M+1].

### Step 6

### tert-Butyl 2-(7-(5-methyl-2-((1-methyl-1H-pyrazol-5-yl)amino)pyrimidin-4-yl)-1-oxo-3,4-dihydro pyrrolo[1,2-a]pyrazin-2(1H)-yl)propanoate 10g

Compound **10h** (150 mg, 0.38 mmol) was dissolved in 6 mL of dioxane under an argon atmosphere, followed by the addition of compound **7b** (56 mg, 0.58 mmol), cesium carbonate (375 mg, 1.15 mmol), 4,5-bis(diphenylphosphino)-9,9-dimethylxanthene (35 mg, 38.2 µmol) and tris(dibenzylideneacetone)dipalladium (35 mg, 38 µmol) successively. The reaction solution was stirred at 90°C for 14 hours, cooled, and filtered through Celite. The filtrate was concentrated, and the residues were purified by column chromatography with eluent system A to obtain the title compound **10g** (100 mg), yield: 57.7%.

MS m/z (ESI): 452.3 [M+1].

### Step 7

### 2-(7-(5-Methyl-2-((1-methyl-1H-pyrazol-5-yl)amino)pyrimidin-4-yl)-1-oxo-3,4-dihydro pyrrolo[1,2-a]pyrazin-2(1H)-yl)propanoic acid 10h

Compound **10g** (100 mg, 0.21 mmol) was dissolved in 5 mL of dichloromethane, followed by the dropwise addition of 1 mL of trifluoroacetic acid. After the addition was completed, the reaction solution was stirred for 1 hour, and concentrated under reduced pressure to obtain the crude compound **10h** (107 mg), which was directly used in the next step without purification.

MS m/z (ESI): 396.2 [M+1].

### Step 8

### (S)-N-((S)-2-Hydroxy-1-(m-tolyl)ethyl)-2-(7-(5-methyl-2-((1-methyl-1H-pyrazol-5-yl)a mino)pyrimidin-4-yl)-1-oxo-3,4-dihydropyrrolo[1,2-a]pyrazin-2(1H)-yl)propanamide 10-P1

### (R)-N-((S)-2-Hydroxy-1-(m-tolyl)ethyl)-2-(7-(5-methyl-2-((1-methyl-1H-pyrazol-5-yl)a mino)pyrimidin-4-yl)-1-oxo-3,4-dihydropyrrolo[1,2-a]pyrazin-2(1H)-yl)propanamide 10-P2

Compound **10h** (107 mg, 0.21 mmol) and (*S*)-2-amino-2-(*m*-tolyl)ethanol (38 mg, 0.25 mmol) were dissolved in 5 mL of *N,N*-dimethylformamide, followed by the addition of 2-(7-azabenzotriazol-1-yl)-*N,N,N*',*N*'-tetramethyluronium hexafluorophosphate (100 mg, 0.26 mmol) and *N,N-*diisopropylethylamine (81 mg, 0.63 mmol). The reaction solution was stirred for 14 hours, and concentrated under reduced pressure. The residues were purified by preparative liquid chromatography (Instrument model: Gilson 281, chromatographic column: X-Bridge, Prep 30^{∗}150mm; 5µm; C18, mobile phase: A-water (10 mM ammonium bicarbonate), B-acetonitrile, flow rate: 30 mL /min, column temperature: room temperature) to obtain the title compounds **10-P1** and **10-P2** (17 mg, 17 mg), yield: (15%, 15%).

### Compound in a single configuration (with shorter retention time) 10-P1

MS m/z (ESI): 529.3 [M+1]
HPLC analysis: retention time: 11.3 minutes, purity: 98.2% (chromatographic column: X-Bridge, Prep 30^{∗}150mm; 5µm; mobile phase: A-water (10 mM ammonium bicarbonate), B-acetonitrile, gradient: A 25%-39%).
¹H NMR (400 MHz, CD₃OD): *δ* 8.14 (s, 1H), 7.55 (s, 1H), 7.46 (s, 1H), 7.42 (s, 1H), 7.19-7.06 (m, 3H), 7.02 (d, 1H), 6.32 (s, 1H), 5.36 (q, 1H), 5.00-4.93 (m, 1H), 4.24-4.02 (m, 2H), 3.74 (s, 6H), 3.65-3.54 (m, 1H), 2.35 (s, 3H), 2.22 (s, 3H), 1.46 (d, 3H).

### Compound in a single configuration (with longer retention time) 10-P2

MS m/z (ESI): 529.3 [M+1]
HPLC analysis: retention time: 12.5 minutes, purity: 98.5% (chromatographic column: X-Bridge, Prep 30^{∗}150mm; 5µm; mobile phase: A-water (10 mM ammonium bicarbonate), B-acetonitrile, gradient: A 25%-39%).
¹H NMR (400 MHz, CD₃OD): *δ* 8.14 (s, 1H), 7.55 (s, 1H), 7.44 (d, 2H), 7.25-7.04 (m, 4H), 6.33 (s, 1H), 5.33 (q, 1H), 5.00-4.95 (m, 1H), 4.41-4.18 (m, 2H), 3.88-3.68 (m, 7H), 2.34 (d, 6H), 1.43 (d, 3H).

### Example 11

### (S)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-2-hydroxy-1-(m-tolyl)ethyl)propanamide 11-P1

### (R)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-2-hydroxy-1-(m-tolyl)ethyl)propanamide 11-P2

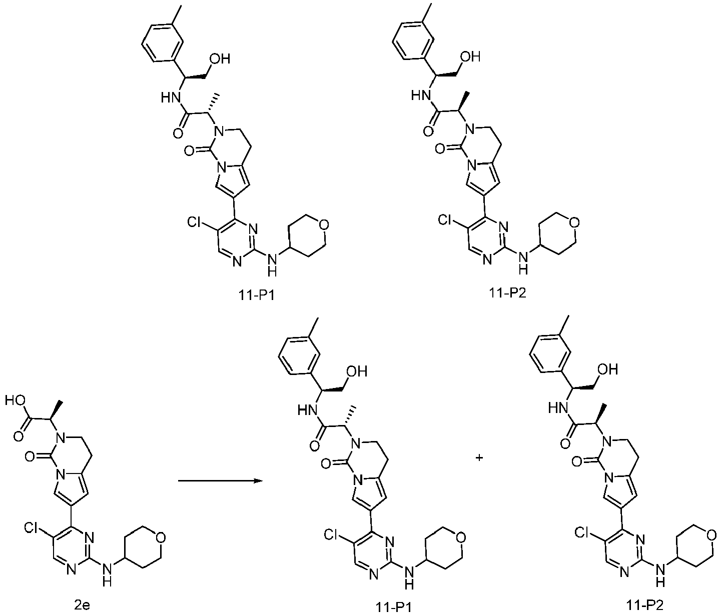

Compound 2e (70 mg, 0.17 mmol) and compound (*S*)-2-amino-2-(*m*-tolyl)ethanol (25 mg, 0.17 mmol) were dissolved in 5 mL of *N,N*-dimethylformamide, followed by the addition of 2-(7-azabenzotriazol-1-yl)-*N,N,N*',*N*'-tetramethyluronium hexafluorophosphate (70 mg, 0.18 mmol) and *N,N*-diisopropylethylamine (64 mg, 0.49 mmol). The reaction solution was stirred for 14 hours, and concentrated under reduced pressure. The residues were purified by preparative liquid chromatography (Instrument model: Gilson 281, chromatographic column: Sharpsil-T, Prep30^{∗}150mm; 5µm; C18, mobile phase: A-water (0.1% trifluoroacetic acid), B-acetonitrile, flow rate: 30 mL /min, column temperature: room temperature) to obtain the title compounds **11-P1** and **11-P2** (15 mg, 15 mg), yield: (16.2%, 16.2%).

### Compound in a single configuration (with shorter retention time) 11-P1

MS m/z (ESI): 553.2 [M+1]
HPLC analysis: retention time: 16.6 minutes, purity: 97.2% (chromatographic column: Sharpsil-T, Prep 30^{∗}150mm; 5µm; mobile phase: A-water (0.1% trifluoroacetic acid), B-acetonitrile, gradient: A31%-49%)
¹H NMR (400 MHz, CD₃OD) *δ* 8.29(s, 1H), 8.22(s, 1H), 7.15-7.19 (m, 4H), 6.77 (s, 1H), 5.19 (d, 1H), 4.91-4.94 (m, 1H), 3.98-4.01 (m, 3H), 3.74-3.77(m, 2H), 3.57-3.59 (m, 4H), 3.51-3.54 (m, 1H), 2.92-2.93 (m, 1H), 2.28 (s, 3H), 1.99-2.02 (m, 2H), 1.61-1.63 (m, 2H), 1.50 (d, 3H).

### Compound in a single configuration (with longer retention time) 11-P2

MS m/z (ESI): 553.2 [M+1]
HPLC analysis: retention time: 17.5 minutes, purity: 96.2% (chromatographic column: Sharpsil-T, Prep 30^{∗}150mm; 5µm; mobile phase: A-water (0.1% trifluoroacetic acid), B-acetonitrile, gradient: A 31%-49%)
MS m/z (ESI): 553.2 [M+1]
¹H NMR (400 MHz, CD₃OD) *δ* 8.19(s, 1H), 8.13(s, 1H), 7.06-7.21 (m, 4H), 6.69 (s, 1H), 5.17 (d, 1H), 4.95-4.97 (m, 1H), 3.75-3.98 (m, 3H), 3.72-3.75 (m, 2H), 3.50-3.59 (m, 4H), 3.08-3.10 (m, 1H), 2.96-2.97 (m, 1H), 2.31 (s, 3H), 1.96-1.99 (m, 2H), 1.59-1.60 (m, 2H), 1.44 (d, 3H).

### Example 12

### (S)-N-((S)-1-(3-Fluoro-5-methoxyphenyl)-2-hydroxyethyl)-2-(7-(5-methyl-2-((1-methyl -1H-pyrazol-5-yl)amino)pyrimidin-4-yl)-1-oxo-3,4-dihydropyrrolo[1,2-a]pyrazin-2(1H) -yl)propanamide 12-P1

### (R)-N-((S)-1-(3-Fluoro-5-methoxyphenyl)-2-hydroxyethyl)-2-(7-(5-methyl-2-((1-methyl -1H-pyrazol-5-yl)amino)pyrimidin-4-yl)-1-oxo-3,4-dihydropyrrolo[1,2-a]pyrazin-2(1H) -yl)propanamide 12-P2

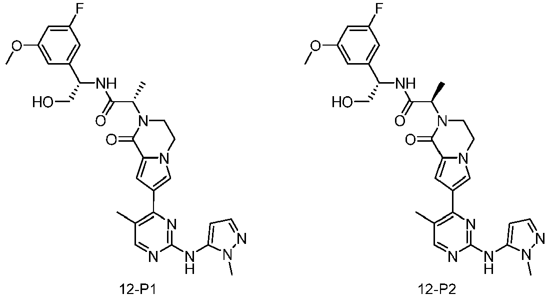

In accordance with the synthetic route in Example **10,** the starting compound (*S*)-2-amino-2-(*m*-tolyl)ethanol in Step 8 was replaced with compound **1p,** to obtain the title compounds **12-P1** and **12-P2** (20 mg, 20 mg).

### Compound in a single configuration (with shorter retention time) 12-P1

MS m/z (ESI): 563.2 [M+1]
HPLC analysis: retention time: 11.01 minutes, purity: 96.2% (chromatographic column: Sharpsil-T, Prep 30^{∗}150mm; 5µm; mobile phase: A-water (0.1% trifluoroacetic acid), B-acetonitrile, gradient: A 21%-39%)
¹H NMR (400 MHz, CD₃OD) *δ* 8.19 (s, 1H), 7.69 (s, 1H), 7.52-7.54 (m, 2H), 6.66-6.72 (m, 2H), 6.54-6.55 (m, 1H), 6.45 (s, 1H), 5.32-5.37 (m, 1H), 4.95-4.96 (m, 1H), 4.25-4.28 (m, 2H), 3.73-3.81 (m, 10H), 2.42 (s, 3H), 1.51(d, 3H).

### Compound in a single configuration (with longer retention time) 12-P2

MS m/z (ESI): 563.2 [M+1]
HPLC analysis: retention time: 16.3 minutes, purity: 98.2% (chromatographic column: Sharpsil-T, Prep 30^{∗}150mm; 5µm; mobile phase: A-water (0.1% trifluoroacetic acid), B-acetonitrile, gradient: A 21%-39%)
¹H NMR (400 MHz, CD₃OD) *δ* 8.20 (s, 1H), 7.76 (s, 1H), 7.63 (s, 1H), 7.55 (s, 1H), 6.65-6.75 (m, 4H), 5.34-5.35 (m, 1H), 4.94-4.95 (m, 1H), 4.33-4.40 (m, 2H), 3.72-3.88 (m, 10H), 2.45 (s, 3H), 1.48 (d, 3H).

### Example 13

### (S)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-chlorophenyl)-2-hydroxyethyl)propana mide 13-P1

### (R)-2-(7-(5-Chloro-2-((tetrahydro-2H-pyran-4-yl)amino)pyrimidin-4-yl)-1-oxo-3,4-dihy dropyrrolo[1,2-a]pyrazin-2(1H)-yl)-N-((S)-1-(3-chlorophenyl)-2-hydroxyethyl)propana mide 13-P2

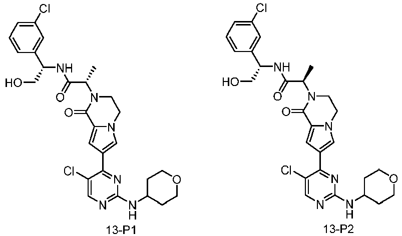

In accordance with the synthetic route in Example 9, the starting compound (*S*)-2-amino-2-(*m*-tolyl)ethanol in Step 8 was replaced with compound (*S*)-2-amino-2-(*m*-chlorophenyl)ethanol (Shanghai Bide Pharmatech Ltd.), to obtain the compounds **13-P1** and **13-P2** (18 mg, 18 mg).

### Compound in a single configuration (with shorter retention time) 13-P1

MS m/z (ESI): 573.1 [M+1]
HPLC analysis: retention time: 12.1 minutes, purity: 96.8% (chromatographic column: X-Bridge, Prep 30^{∗}150mm; 5µm; mobile phase: A-water (10 mM ammonium acetate), B-acetonitrile, gradient: A 29%-44%)
¹H NMR (400 mHz, CDCl₃): *δ* 8.09 (s, 1H), 7.92 (s, 1H), 7.85(s, 1H), 7.25-7.18 (m, 4H), 5.44-5.38 (m, 1H), 5.06-5.02 (m, 1H), 4.20-4.15 (m, 3H), 4.06-4.03 (m, 2H), 3.96-3.94 (m, 1H), 3.89-3.87 (m, 1H), 3.79-3.75 (m, 1H), 3.62-3.57 (m, 3H), 2.06-2.03 (m, 2H), 1.80-1.77 (m, 2H), 1.49 (d, 3H).

### Compound in a single configuration (with longer retention time) 13-P2

MS m/z (ESI): 573.1[M+1]
HPLC analysis: retention time: 13.2 minutes, purity: 97.2% (chromatographic column: X-Bridge, Prep 30^{∗}150mm; 5µm; mobile phase: A-water (10 mM ammonium acetate), B-acetonitrile, gradient: A 29%-44%)
¹H NMR (400 mHz, CD₃OD): *δ* 8.53-8.51 (d, 1H), 8.22 (s, 1H), 8.00 (s, 1H), 7.72 (s, 1H), 7.39 (s, 1H), 7.30-7.28 (m, 2H), 5.37-5.32 (m, 1H), 5.00-4.95 (m, 1H), 4.43-4.37 (m, 1H), 4.36-4.31 (m, 1H), 4.02-3.99 (m, 3H), 3.85-3.82 (m, 2H), 3.79-3.71 (m, 2H), 3.62-3.56 (m, 2H), 2.04-2.01 (m, 2H), 1.69-1.59 (m, 2H), 1.47 (d, 3H).

### Example 14

### (S)-2-(2-(5-Chloro-2-(((S)-1-hydroxypropan-2-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 14-P1

### (R)-2-(2-(5-Chloro-2-(((S)-1-hydroxypropan-2-yl)amino)pyrimidin-4-yl)-8-oxo-5,6 -dihydroimidazo[1,2-a]pyrazin-7(8H)-yl)-N-((S)-1-(3-fluoro-5-methoxyphenyl)-2-hydro xyethyl)propanamide 14-P2

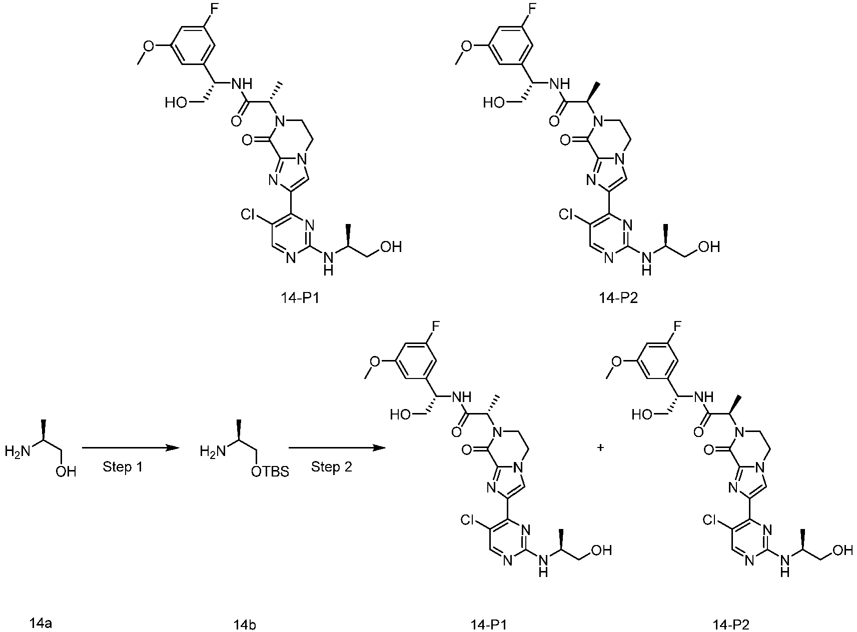

### Step 1

### (S)-1-((tert-Butyldimethylsilyl)oxy)propan-2-amine 14b

Compound **14a** (2 g, 26.6 mmol, Shanghai Haohong Biomedical Technology Co., Ltd.) and imidazole (3.6 g, 52.9 mmol) were dissolved in 80 mL of dichloromethane, followed by the addition of *tert*-butyldimethylchlorosilane (6 g, 39.8 mmol) in an ice bath, and the reaction solution was stirred for 14 hours. Water was added, and the reaction solution was extracted with dichloromethane (80 mL×2). The organic phases were combined, washed with a saturated solution of sodium chloride, dried over anhydrous sodium sulfate, and filtered. The filtrate was concentrated under reduced pressure, and the residues were purified by column chromatography with eluent system C to obtain the title compound **14b** (4.0 g), yield: 79.3%.

MS m/z (ESI): 190.2 [M+1].

### Step 2

### (S)-2-(2-(5-Chloro-2-(((S)-1-hydroxypropan-2-yl)amino)pyrimidin-4-yl)-8-oxo-5,6-dihy droimidazo[1,2-a]pyrazin-7(8H)-yl)-N-((S)-1-(3-fluoro-5-methoxyphenyl)-2-hydroxyet hyl)propanamide 14-P1

### (R)-2-(2-(5-Chloro-2-(((S)-1-hydroxypropan-2-yl)amino)pyrimidin-4-yl)-8-oxo-5,6-dih ydroimidazo[1,2-a]pyrazin-7(8H)-yl)-N-((S)-1-(3-fluoro-5-methoxyphenyl)-2-hydroxye thyl)propanamide 14-P2

In accordance with the synthetic route in Example **3,** the starting compound **2c** in Step 3 was replaced with compound **14b,** to obtain the compounds **14-P1** and **14-P2** (20 mg, 20 mg).

### Compound in a single configuration (with shorter retention time) 14-P1

MS m/z (ESI): 562.2 [M+1]
HPLC analysis: retention time: 15.4 minutes, purity: 96.8% (chromatographic column: X-Bridge, Prep 30^{∗}150mm; 5µm; mobile phase: A-water (10 mM ammonium bicarbonate), B-acetonitrile, gradient: A 25%-45%)
¹H NMR (400 MHz, CD₃OD): δ 8.27 (s, 1H), 8.06 (s, 1H), 6.82(s, 1H),6.81-6.73 (m, 1H), 6.61-6.57 (m, 1H), 5.45-5.43 (m, 1H), 4.99-4.97 (m, 1H), 4.61-4.51 (m,1H), 4.37-4.34 (m, 1H), 4.31-4.15 (m,1H), 3.92-3.89 (m, 2H), 3.79 (s, 3H), 3.78-3.72 (m, 1H), 3.59-3.58 (m, 2H),1.44 (d, 3H), 1.29-1.23 (m, 1H), 1.22 (d, 3H).

### Compound in a single configuration (with longer retention time) 14-P2

MS m/z (ESI): 562.2 [M+1]
HPLC analysis: retention time: 17.2 minutes, purity: 97.2% (chromatographic column: X-Bridge, Prep 30^{∗}150mm; 5µm; mobile phase: A-water (10 mM ammonium bicarbonate), B-acetonitrile, gradient: A 25%-45%).
¹H NMR (400 MHz, CD₃OD): δ 8.27 (s, 1H), 8.06 (s, 1H), 6.82 (s, 1H), 6.81-6.73 (m, 1H), 6.61-6.57 (m, 1H), 5.45-5.43 (m, 1H), 4.99-4.97 (m, 1H), 4.61-4.51 (m, 1H), 4.37-4.34 (m, 1H), 4.31-4.15 (m, 1H), 3.92-3.89 (m, 2H), 3.79 (s, 3H), 3.78-3.72 (m, 1H), 3.59-3.58 (m, 2H), 1.44 (d, 3H), 1.29-1.23 (m, 1H), 1.22 (d, 3H).

### Example 15

### (R)-2-(7-(5-Chloro-2-(((S)-1-hydroxypropan-2-yl)amino)pyrimidin-4-yl)-1-oxo-3,4-dih ydropyrrolo[1,2-a]pyrazin-2(1H)-yl)-N-((S)-1-(3-fluoro-5-methoxyphenyl)-2-hydroxyet hyl)propanamide 15

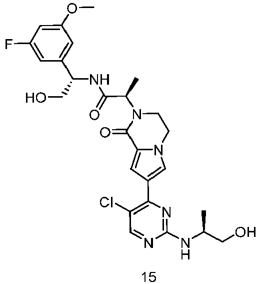

In accordance with the synthetic route in Example **4,** the starting compound **2c** in Step 4 was replaced with compound **14b,** to obtain compounds **15** (20 mg).
MS m/z (ESI): 561.2 [M+1].
¹H NMR (400 MHz, CDCl₃) *δ* 8.20 (s, 1H), 7.60 (s, 1H), 6.71-6.74 (m, 2H), 6.53-6.63 (m, 2H), 5.37-5.40 (m, 1H), 5.07-5.20 (m, 3H), 4.32-4.36 (m, 2H), 4.14-4.16 (m, 2H), 3.69-3.81 (m, 7H), 1.15-1.25 (m, 5H).

### Example 16

### (S)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-chlorophenyl)-2-hydroxyethyl)propa namide 16-P1

### (R)-2-(6-(5-Chloro-2-((tetrahydro-2H-pyran-4-yl)amino)pyrimidin-4-yl)-1-oxo-3,4-dihy dropyrrolo[1,2-c]pyrimidin-2(1H)-yl)-N-((S)-1-(3-chlorophenyl)-2-hydroxyethyl)propa namide 16-P2

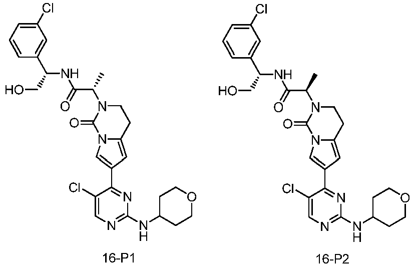

In accordance with the synthetic route in Example **11,** the starting compound (*S*)-2-amino-2-(*m*-tolyl)ethanol in Step 10 was replaced with compound (*S*)-2-amino-2-(*m*-chlorophenyl)ethanol, to obtain the compounds **16-P1** and **16-P2** (30 mg, 30 mg).

### Compound in a single configuration (with shorter retention time) 16-P1

MS m/z (ESI): 573.1 [M+1]
HPLC analysis: retention time: 15.4 minutes, purity: 96.8% (chromatographic column: X-Bridge, Prep 30^{∗}150mm; 5µm; mobile phase: A-water (10 mM ammonium bicarbonate), B-acetonitrile, gradient: A33%-51%).
¹H NMR (400 mHz, CDCl₃): *δ* 8.49 (s, 1H), 8.10 (s, 1H), 7.25 (s, 1H), 7.18 (s, 1H), 7.03 (d, 1H), 6.75 (s, 1H), 5.22-5.17 (m, 1H), 5.07-5.03 (m, 1H), 4.20-4.14 (m, 1H), 4.06-4.03 (m, 2H), 3.99-3.88 (m, 2H), 3.60-3.55 (m, 3H), 3.47-3.44 (m, 1H), 3.03-2.97 (m, 3H), 2.07-2.04 (m, 2H), 1.78-1.76 (m, 2H), 1.53 (d, 3H).

### Compound in a single configuration (with longer retention time) 16-P2

MS m/z (ESI): 573.1 [M+1]
HPLC analysis: retention time: 17.5 minutes, purity: 97.2% (chromatographic column: X-Bridge, Prep 30^{∗}150mm; 5µm; mobile phase: A-water (10 mM ammonium bicarbonate), B-acetonitrile, gradient: A33%-51%).
¹H NMR (400 mHz, CDCl₃): *δ* 9.18 (s, 1H), 8.47 (s, 1H), 8.10 (s, 1H), 7.34-7.30 (m, 3H), 7.22-7.21 (m, 1H), 7.08 (s, 1H), 6.75 (s, 1H), 5.23-5.18 (m, 1H), 5.07 (s, 1H), 4.36 (s, 1H), 4.14 (s, 1H), 4.07-4.04 (m, 2H), 3.93-3.91 (m, 1H), 3.85-3.80 (m, 1H), 3.74-3.69 (m, 1H), 3.67-3.64 (m, 1H), 3.65-3.60 (m, 2H), 3.17-3.12 (m, 1H), 3.06-3.01 (m, 1H), 2.06-2.03 (m, 2H), 1.80-1.78 (m, 2H), 1.54 (d, 3H).

### Example 17

### (S)-N-((S)-1-(3-Fluoro-5-methoxyphenyl)-2-hydroxyethyl)-2-(7-(5-methyl-2-((tetrahydr o-2H-pyran-4-yl)amino)pyrimidin-4-yl)-1-oxo-3,4-dihydropyrrolo[1,2-a]pyrazin-2(1H) -yl)propanamide 17-P1

### (R)-N-((S)-1-(3-Fluoro-5-methoxyphenyl)-2-hydroxyethyl)-2-(7-(5-methyl-2-((tetrahydr o-2H-pyran-4-yl)amino)pyrimidin-4-yl)-1-oxo-3,4-dihydropyrrolo[1,2-a]pyrazin-2(1H) -yl)propanamide 17-P2

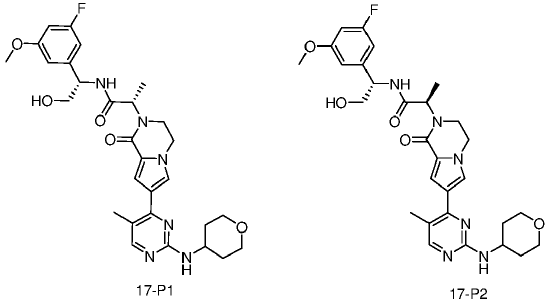

In accordance with the synthetic route in Example **10,** the starting compound **7b** in Step 6 was replaced with compound **2c,** and the starting compound (*S*)-2-amino-2-(*m*-tolyl)ethanol in Step 8 was replaced with compound **1p**, to obtain the title compounds **17-P1** and **17-P2** (6 mg, 6 mg).

### Compound in a single configuration (with shorter retention time) 17-P1

MS m/z (ESI): 567.2 [M+1]
HPLC analysis: retention time: 12.4 minutes, purity: 98.6% (chromatographic column: X-Bridge, Prep 30^{∗}150mm; 5µm; mobile phase: A-water (10 mM ammonium bicarbonate), B-acetonitrile, gradient: A 25%-39%).
¹H NMR (400 MHz, CD₃OD): *δ* 8.01 (s, 1H), 7.57 (s, 1H), 7.47 (s, 1H), 6.78-6.62 (m, 2H), 6.54 (d, 1H), 5.34 (q, 1H), 4.29-4.14 (m, 2H), 4.10-3.89 (m, 3H), 3.88-3.64 (m, 8H), 3.57 (t, 2H), 2.30 (s, 3H), 2.04-1.97 (m, 2H), 1.64-1.53 (m, 2H), 1.49 (d, 3H).

### Compound in a single configuration (with longer retention time) 17-P2

MS m/z (ESI): 567.2 [M+1]
HPLC analysis: retention time: 13.5 minutes, purity: 99.2% (chromatographic column: X-Bridge, Prep 30^{∗}150mm; 5µm; mobile phase: A-water (10 mM ammonium bicarbonate), B-acetonitrile, gradient: A 25%-39%).
¹H NMR (400 MHz, CD₃OD): *δ* 8.02 (s, 1H), 7.59 (s, 1H), 7.48 (s, 1H), 6.75 (s, 1H), 6.69 (d, 1H), 6.59 (d, 1H), 5.34 (q, 1H), 4.45-4.23 (m, 2H), 4.13-3.93 (m, 3H), 3.91-3.66 (m, 8H), 3.58 (t, 2H), 2.31 (s, 3H), 1.99 (s, 2H), 1.65-1.56 (m, 2H), 1.46 (d, 3H).

### Example 18

### (S)-N-((S)-2-Hydroxy-1-(m-tolyl)ethyl)-2-(7-(2-((1-methyl-1H-pyrazo1-5-yl)amino)pyri midin-4-yl)-1-oxo-3,4-dihydropyrrolo[1,2-a]pyrazin-2(1H)-yl)propanamide 18-P1

### (R)-N-((S)-2-Hydroxy-1-(m-tolyl)ethyl)-2-(7-(2-((1-methyl-1H-pyrazol-5-yl)amino)pyri midin-4-yl)-1-oxo-3,4-dihydropyrrolo[1,2-a]pyrazin-2(1H)-yl)propanamide 18-P2

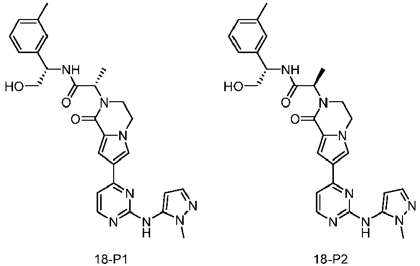

In accordance with the synthetic route in Example **10,** the starting compound **10a** in Step 1 was replaced with 2,4-dichloropyrimidine, to obtain the compounds **18-P1** and **18-P2** (30 mg, 30 mg).

### Compound in a single configuration (with shorter retention time) 18-P1

MS m/z (ESI): 515.3 [M+1]
HPLC analysis: retention time: 13.2 minutes, purity: 96.8% (chromatographic column: X-Bridge, Prep 30^{∗}150mm; 5µm; mobile phase: A-water (10 mM ammonium acetate), B-acetonitrile, gradient: A 20%-36%).
¹H NMR (400 mHz, CDCl₃): *δ* 8.34(s, 1H), 7.49(s, 1H), 7.39 (s, 2H), 7.12-7.09 (m, 2H), 7.03-7.00 (m, 3H), 6.93 (m,1H), 6.70 (s, 1H), 6.33 (s, 1H), 5.43-5.38 (m, 1H), 5.01-5.00 (m, 1H), 4.10-4.05 (m, 1H), 3.97-3.95 (m, 1H), 3.86-3.82 (m, 2H), 3.81 (s, 3H),3.68-3.63 (m, 1H), 3.53-3.48 (m, 1H), 2.24-2.21 (m, 1H), 1.44(d, 3H)

### Compound in a single configuration (with longer retention time) 18-P2

MS m/z (ESI): 515.3 [M+1]
HPLC analysis: retention time: 15.4 minutes, purity: 97.2% (chromatographic column: X-Bridge, Prep 30^{∗}150mm; 5µm; mobile phase: A-water (10 mM ammonium acetate), B-acetonitrile, gradient: A 20%-36%).
¹H NMR (400 mHz, CDCl₃): *δ* 8.29 (s, 1H), 7.76 (s, 1H), 7.51 (s, 1H), 7.24-7.22 (m, 2H), 7.18-7.16 (m, 2H), 7.11-7.10 (m, 2H), 6.79 (s, 1H), 6.33 (s, 1H), 5.40-5.36 (m, 1H), 5.13 (s, 1H), 4.24-4.21 (m, 1H), 4.05-4.01 (m, 1H), 3.91-3.79 (m, 6H), 3.73-3.68 (m, 1H), 2.35 (s, 3H), 2.24-2.22 (m, 1H), 2.06-2.04 (m, 1H).

### Example 19

### (S)-N-((S)-2-Hydroxy-1-(m-tolyl)ethyl)-2-(1-oxo-7-(2-((tetrahydro-2H-pyran-4-yl)amin o)pyrimidin-4-yl)-3,4-dihydropyrrolo[1,2-a]pyrazin-2(1H)-yl)propanamide 19-P1

### (R)-N-((S)-2-Hydroxy-1-(m-tolyl)ethyl)-2-(1-oxo-7-(2-((tetrahydro-2H-pyran-4-yl)amin o)pyrimidin-4-yl)-3,4-dihydropyrrolo[1,2-a]pyrazin-2(1H)-yl)propanamide 19-P2

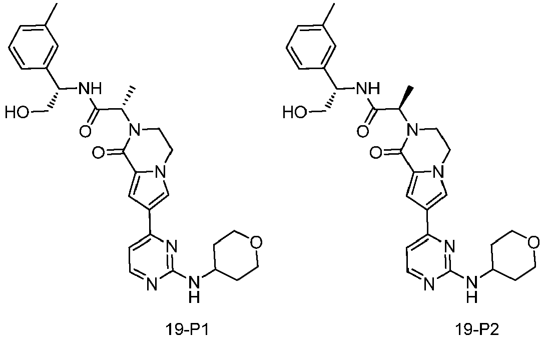

In accordance with the synthetic route in Example **9,** the starting compound **2a** in Step 1 was replaced with 2,4-dichloropyrimidine, to obtain the compounds **19-P1** and **19-P2** (60 mg, 30 mg).

### Compound in a single configuration (with shorter retention time) 19-P1

MS m/z (ESI): 519.2 [M+1]
HPLC analysis: retention time: 11.6 minutes, purity: 98.1% (chromatographic column: X-Bridge, Prep 30^{∗}150mm; 5µm; mobile phase: A-water (10 mM ammonium acetate), B-acetonitrile, gradient: A 25%-39%).
H NMR (400 MHz, CD₃OD): *δ*8.15 (d, 1H), 7.96 (s, 1H), 7.21-7.14 (m, 4H), 7.00 (d, 1H), 6.54 (s, 1H), 5.20 (q, 1H), 4.98-4.97 (m, 1H), 4.01-3.98 (m, 3H), 3.77-3.74 (m, 2H), 3.63-3.45 (m, 4H), 2.91-2.89 (m, 2H), 2.29 (s, 3H), 2.03-2.01 (m, 2H), 1.64-1.60(m, 2H), 1.49 (d, 3H).

### Compound in a single configuration (with longer retention time) 19-P2

MS m/z (ESI): 519.2 [M+1]
HPLC analysis: retention time: 12.7 minutes, purity: 98.5% (chromatographic column: X-Bridge, Prep 30^{∗}150mm; 5µm; mobile phase: A-water (10 mM ammonium acetate), B-acetonitrile, gradient: A 25%-39%).
H NMR (400 MHz, CD₃OD):*δ* 8.16 (d, 1H), 7.96 (s, 1H), 7.20-7.10 (m, 4H), 7.00 (d, 1H), 6.54 (s, 1H), 5.19 (q, 1H), 4.98-4.95 (m, 1H), 4.01-3.98 (m, 3H), 3.76-3.55 (m, 6H), 3.20-2.98 (m, 2H), 2.34 (s, 3H), 2.04-2.01 (m, 2H), 1.64-1.60(m, 2H), 1.47 (d, 3H).

### Biological Assay

### Test example 1: ERK1 enzyme activity test

### 1. Test purpose

The purpose of this experiment is to detect the inhibitory ability of the compounds of the present disclosure on the ERK1 enzyme activity and to evaluate the in vitro activity of the compounds based on the IC₅₀. The ADP-Glo^{™} Kinase Assay Kit was used in this experiment. The substrate was phosphorylated under the action of the enzyme and ADP was produced at the same time. The ADP-Glo reagent was added to remove the unreacted ATP in the reaction system, and the ADP produced by the reaction was detected with kinase detection reagent. In the presence of the compound, the inhibition rate of the compound was calculated by measuring the signal value.

### 2. Experimental method

Preparation of Enzyme and substrate: ERK1 (1879-KS-010, R&D) and substrate (AS-61777, anaspec) were prepared to 0.75 ng/µl and 100µM respectively in a buffer, and then the enzyme solution and the substrate solution were prepared into a mixed solution at a volume ratio of 2:1 for later use. ATP was diluted to 300 µM with buffer. The compound was dissolved in DMSO (dimethyl sulfoxide, Shanghai Titan Scientific Co., Ltd.) to prepare a stock solution with an initial concentration of 20 mM, and then Bravo was used to prepare the compound. Finally, 3 µL of a mixed solution of enzyme and substrate, and 1 µL of different concentrations of the compound (the initial concentration was 50 µM, 4-fold dilution) were added to each well of the 384-well plate, and the plate was incubated at 30°C for 10 minutes, and finally 1 µL of 300 µM ATP solution was added to each well, and the plate was incubated at 30°C for 2 hours. Then 5 µL of ADP-Glo was added, and the plate was incubated at 30°C for 40 minutes. Then 10 µL of kinase detection buffer was added, and the plate was incubated at 30°C for 40 minutes. The 384-well plate was taken out and placed in a microplate reader (BMG labtech, PHERAstar FS), and the chemiluminescence was measured by a microplate reader.

### 3. Data analysis

Microsoft Excel, Graphpad Prism 5 was used to process and analyze the data. The IC₅₀ value of the compound was obtained, and the results are shown in the table below.

| Example No. | IC₅₀ (nM) |
|---|---|
| 1-P2 | 7 |
| 2-P2 | 4.6 |
| 3-P2 | 4.6 |
| 4-P2 | 3.8 |
| 9-P2 | 9 |
| 10-P2 | 8.5 |
| 11-P2 | 17 |
| 12-P2 | 9.4 |
| 17-P2 | 22 |

Conclusion: The compounds of the present disclosure have a significant inhibitory effect on the ERK1 enzyme activity.

### Test example 2: ERK2 enzyme activity test

### 1. Test purpose

The purpose of this experiment is to detect the inhibitory ability of the compounds of the present disclosure on the ERK2 enzyme activity, and to evaluate the in vitro activity of the compounds based on the IC₅₀. The ADP-Glo^{™} Kinase Assay Kit was used in this experiment. The substrate was phosphorylated under the action of the enzyme and ADP was produced at the same time. The ADP-Glo reagent was added to remove the unreacted ATP in the reaction system, and the ADP produced by the reaction was detected with kinase detection reagent. In the presence of the compound, the inhibition rate of the compound was calculated by measuring the signal value.

### 2. Experimental method

Preparation of enzyme and substrate: ERK2 (1879-KS-010, R&D) and substrate (custom peptide, Gill Biochemical) were prepared to 0.75 ng/µl and 1500µM in a buffer (40 mM Tris, 20 mM MgCl₂, 0.1 mg/ml BSA, 50 µM DTT), and then the enzyme solution and the substrate solution were prepared into a mixed solution at a volume ratio of 2:1 for later use. ATP was diluted to 500 µM with buffer. The compound was dissolved in DMSO (dimethyl sulfoxide, Shanghai Titan Scientific Co., Ltd.) to prepare a stock solution with an initial concentration of 20 mM, and then Bravo was used to prepare the compound. Finally, 3 µL of a mixed solution of enzyme and substrate, and 1 µL substrate of different concentrations of the compound (the initial concentration is 50 µM, 4-fold dilution) were added to each well of the 384-well plate, and the plate was incubated at 30°C for 10 minutes, and finally 1 µL 500 µM ATP solution was added to each well, and the plate was incubated at 30°C for 2 hours. Then 5 µL of ADP-Glo was added, and the plate was incubated at 30°C for 40 minutes. Then 10 µL of kinase detection buffer was added, and the plate was incubated at 30°C for 40 minutes. The 384-well plate was taken out and placed in a microplate reader (BMG labtech, PHERAstar FS), and the chemiluminescence was measured by a microplate reader.

### 3. Data analysis

Microsoft Excel, Graphpad Prism 5 was used to process and analyze the data. The IC₅₀ value of the compound was obtained, and the results are shown in the table below.

| Example No. | IC₅₀ (nM) |
|---|---|
| 1-P1 | 811 |
| 1-P2 | 1.5 |
| 2-P1 | 336 |
| 2-P2 | 1.7 |
| 3-P2 | 1.75 |
| 4-P1 | 819 |
| 4-P2 | 1 |
| 5 | 3.6 |
| 6-P1 | 2541 |
| 6-P2 | 7.5 |
| 8-P1 | 2121 |
| 8-P2 | 26 |
| 9-P2 | 1.9 |
| 10-P2 | 1.1 |
| 11-P1 | 75 |
| 11-P2 | 2.1 |
| 12-P1 | 948 |
| 12-P2 | 0.9 |
| 13-P2 | 2.8 |
| 14-P1 | 1527 |
| 14-P2 | 4.9 |
| 15 | 28.7 |
| 16-P2 | 4.2 |
| 17-P2 | 3.1 |
| 18-P2 | 5.5 |
| 19-P2 | 6.7 |

Conclusion: The compounds of the present disclosure have a significant inhibitory effect on the ERK2 enzyme activity.

Test example 3: In Vitro Proliferation Inhibition Test on Colo205 Tumor Cells

### 1. Test purpose

The purpose of this experiment is to test the inhibitory activity of the compounds of the present disclosure on the proliferation of Colo205 cells (CCL-222, ATCC) in vitro. The cells were treated in vitro with different concentrations of the compounds. After 3 days of culture, the cell proliferation was tested with CTG (CellTiter-Glo^{®} Luminescent Cell Viability Assay, Promega, Catalog No. G7573) reagent, and the in vitro activity of the compounds was evaluated according to the IC₅₀ value.

### 2. Experimental method

In the following, the in vitro proliferation inhibition test method of Colo205 cells is taken as an example to illustrate the method for testing the in vitro proliferation inhibitory activity of the compounds of the present disclosure on tumor cells. This method is also applicable to, but not limited to, the in vitro proliferation inhibitory activity test on other tumor cells.

Colo205 cells were digested, centrifuged and then resuspended. The single cell suspension was mixed well, and the density of viable cells was adjusted to 5.0×10⁴ cells/ml with cell culture medium (RPMI1640+2% FBS), and 95µL/well was added to a 96-well cell culture plate. Only 100 µL medium was added to the peripheral wells of the 96-well plate. The culture plate was incubated in an incubator for 24 hours (37°C, 5% CO₂).

The compound was dissolved in DMSO (dimethyl sulfoxide, Shanghai Titan Scientific Co., Ltd.) and prepared as a stock solution with an initial concentration of 20 mM. The initial concentration of the small molecule compound was 2 mM, and then 4-fold diluted into 9 points, and the 10^{th} point was DMSO. Another 96-well plate was taken, and 90 µL of cell culture medium (RPMI1640+2% FBS) was added to each well. Then 10 µL of different concentrations of the test sample was added to each well. The mixture was mixed well, and then 5µL of different concentrations of the test sample was added to the cell culture plate. Each sample has two duplicate holes. The culture plate was incubated in the incubator for 3 days (37°C, 5% CO₂). The 96-well cell culture plate was taken out. 50 µL solution of CTG was added to each well, and the plate was incubated for 10 minutes at room temperature. In a microplate reader (BMG labtech, PHERAstar FS), chemiluminescence was measured with the microplate reader.

### 3. Data analysis

Microsoft Excel, Graphpad Prism 5 was used to process and analyze the data. The example results are shown in the table below.

| Example No. | IC₅₀ (nM) |
|---|---|
| 1-P2 | 17.4 |
| 2-P2 | 15 |
| 4-P2 | 10 |
| 9-P2 | 17.7 |
| 10-P2 | 100 |
| 11-P2 | 26 |
| 12-P2 | 82 |
| 13-P2 | 31 |
| 16-P2 | 54 |
| 17-P2 | 38 |
| 19-P2 | 67 |

Conclusion: The compounds of the present disclosure have a significant inhibitory effect on the proliferation of Colo205 tumor cells.

### Pharmacokinetics Evaluation

### Test Example 4. Pharmacokinetics assay of the compounds of the present disclosure in mice

### 1. Abstract

Mice were used as test animals. The drug concentration in plasma at different time points was determined by LC/MS/MS method after administration of the compounds 1-P2, 2-P2, 4-P2, 6-P2, 9-P2, 10-P2 and 11-P2. The pharmacokinetic behavior of the compounds of the present disclosure was studied and evaluated in mice.

### 2. Test protocol

### 2.1 Test compounds

Compounds 1-P2, 2-P2, 4-P2, 6-P2, 9-P2, 10-P2 and 11-P2.

### 2.2 Test animals

Sixty-three C57 mice (female, equally divided into seven groups) were purchased from Shanghai Jiesijie Laboratory Animal Co., LTD. (Certificate No.: SCXK(Shanghai)2013-0006).

### 2.3 Preparation of the test compound

A certain amount of the test compound was weighed, dissolved in 5% of DMSO by volume and 5% of tween 80, followed by the additon of 90% of normal saline to prepare a 0.1 mg/mL colorless, clear and transparent solution.

### 2.4 Administration

After an overnight fast, C57 mice were intragastrically administered the test compound at an administration dose of 2 mg/kg and an administration volume of 0.2 ml/10 g.

### 3. Process

The mice were intragastrically administered the test compounds. 0.1 ml of blood was taken before the administration and at 0.25, 0.5, 1.0, 2.0, 4.0, 6.0, 8.0, 11.0 and 24.0 hours after the administration. The samples were stored in heparinized tubes, and centrifuged for 10 minutes at 3500 rpm to separate the blood plasma. The plasma samples were stored at -20°C.

The content of the test compound in the plasma of mice after intragastrical administration of the test compound at different concentrations was determined: 25 µL of rat plasma at each time point after the administration was taken, followed by the addition of 50 µL of the internal standard camptothecin solution (100ng/mL) and 200 µL of acetonitrile. The resulting solution was vortex-mixed for 5 minutes, and centrifuged for 10 minutes (4000 rpm). 4 µL of the supernatant was taken from the plasma samples for LC/MS/MS analysis.

### 4. Results of pharmacokinetic parameters

Pharmacokinetic parameters of the compounds of the present disclosure are shown below:

| Compou nd No. | Pharmacokinetics assay in mice | | | | | |
|---|---|---|---|---|---|---|
| | Plasma concentration | Area under curve | Half-life | Residence time | Clearance | Apparent distribution volume |
| | Cmax (ng /mL) | AUC (ng /mL^{∗}h) | T1/2 (h) | MRT (h) | CLz/F (ml/min/k g) | Vz/F (ml/kg) |
| 1-P2 | 501 | 1113 | 1.46 | 1.84 | 29.7 | 3749 |
| 2-P2 | 867 | 994 | 2.05 | 1.08 | 33.5 | 5939 |
| 4-P2 | 905 | 936 | 1.07 | 1.07 | 35.5 | 3291 |
| 6-P2 | 1311 | 1744 | 4.74 | 1.39 | 18.9 | 7741 |
| 9-P2 | 1406 | 1328 | 1.44 | 0.838 | 25.1 | 3135 |
| 11-P2 | 813 | 784 | 0.957 | 1.15 | 42.4 | 3508 |

Conclusion: The compounds of the present disclosure are well absorbed, and have a significant pharmacokinetic advantage.

## Claims

1. A compound of formula (I) or a tautomer, mesomer, racemate, enantiomer, diastereomer thereof, or mixture thereof, or a pharmaceutically acceptable salt thereof: 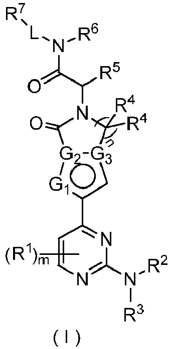 wherein:
G₁, G₂ and G₃ are identical or different and are each independently selected from the group consisting of CH, C and N;
L is a bond or an alkylene, wherein the alkylene is optionally further substituted by one or more substituents selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
each R¹ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
each R⁴ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁵ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁶ is selected from the group consisting of hydrogen atom, halogen, alkyl, alkoxy, haloalkyl, haloalkoxy, hydroxy and hydroxyalkyl;
R⁷ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, haloalkyl, haloalkoxy and aminoalkyl;
n is 1, 2 or 3; and
m is 0, 1 or 2.

2. The compound of formula (I) or the tautomer, mesomer, racemate, enantiomer, diastereomer thereof, or mixture thereof, or the pharmaceutically acceptable salt thereof according to claim 1, being a compound of formula (I-1) or (1-2) or a stereisomer, tautomer, mesomer, racemate, enantiomer, diastereomer thereof, or mixture thereof, or a pharmaceutically acceptable salt thereof: 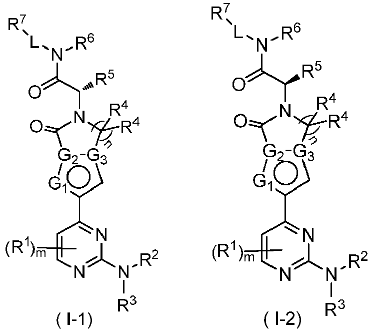 wherein:
R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl; and
G₁, G₂, G₃, L, m, n, R¹ to R⁴, R⁶ and R⁷ are as defined in claim 1.

3. The compound of formula (I) or the tautomer, mesomer, racemate, enantiomer, diastereomer thereof, or mixture thereof, or the pharmaceutically acceptable salt thereof according to claim 1 or 2, wherein n is 1 or 2.

4. The compound of formula (I) or the tautomer, mesomer, racemate, enantiomer, diastereomer thereof, or mixture thereof, or the pharmaceutically acceptable salt thereof according to claim 1, being a compound of formula (II-1), (II-2), (II-3) or (II-4) or a stereisomer, tautomer, mesomer, racemate, enantiomer, diastereomer thereof, or mixture thereof, or a pharmaceutically acceptable salt thereof: 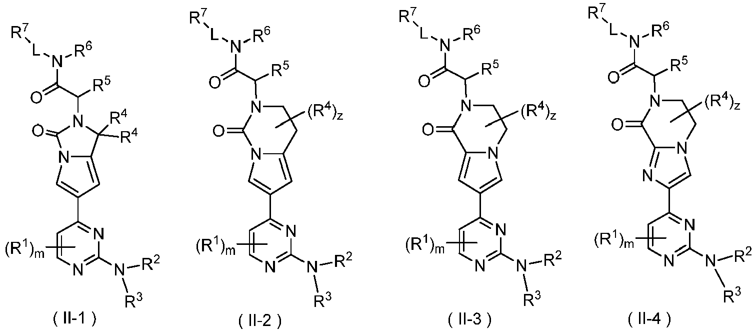 wherein:
z is 0, 1, 2, 3 or 4; and
L, m and R¹ to R⁷ are as defined in claim 1.

5. The compound of formula (I) or the tautomer, mesomer, racemate, enantiomer, diastereomer thereof, or mixture thereof, or the pharmaceutically acceptable salt thereof according to claim 1 or 4, being a compound of formula (II-12), (11-22), (11-32) or (II-42) or a stereisomer, tautomer, mesomer, racemate, enantiomer, diastereomer thereof, or mixture thereof, or a pharmaceutically acceptable salt thereof: 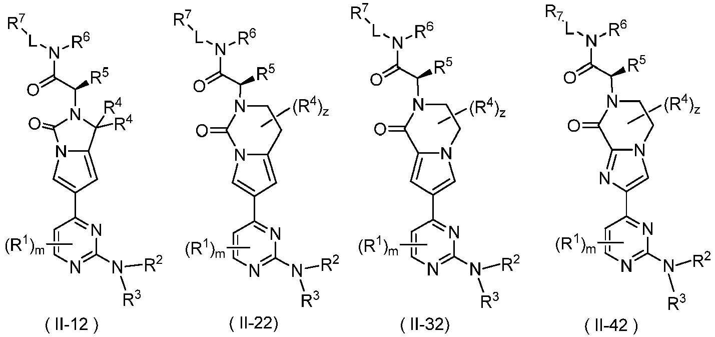 wherein:
R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl;
z is 0, 1, 2, 3 or 4; and
L, m, R¹ to R⁴, R⁶ and R⁷ are as defined in claim 1.

6. The compound of formula (I) or the stereisomer, tautomer, mesomer, racemate, enantiomer, diastereomer thereof, or mixture thereof, or the pharmaceutically acceptable salt thereof according to any one of claims 1 to 5, wherein L is an alkylene, wherein the alkylene is optionally further substituted by one or more substituents selected from the group consisting of alkyl, alkoxy, oxo, halogen, amino, cyano, nitro, hydroxy, hydroxyalkyl, aminoalkyl, cycloalkyl, heterocyclyl, aryl and heteroaryl, preferably, L is -CH(R⁸)-; R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl.

7. The compound of formula (I) or the tautomer, mesomer, racemate, enantiomer, diastereomer thereof, or mixture thereof, or the pharmaceutically acceptable salt thereof according to any one of claims 1, 4 and 6, being a compound of formula (III-1), (III-2), (III-3) or (III-4) or a stereisomer, tautomer, mesomer, racemate, enantiomer, diastereomer thereof, or mixture thereof, or a pharmaceutically acceptable salt thereof: 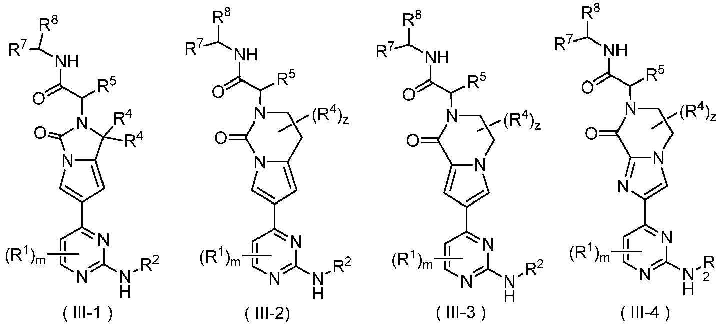 wherein:
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
z is 0, 1, 2, 3 or 4; and
R¹, R², R⁴, R⁵, R⁷ and m are as defined in claim 1.

8. The compound of formula (I) or the tautomer, mesomer, racemate, enantiomer, diastereomer thereof, or mixture thereof, or the pharmaceutically acceptable salt thereof according to any one of claims 1, 4, 6 and 7, being a compound of formula (III-12), (III-22), (III-32) or (III-42) or a stereisomer, tautomer, mesomer, racemate, enantiomer, diastereomer thereof, or mixture thereof, or a pharmaceutically acceptable salt thereof: 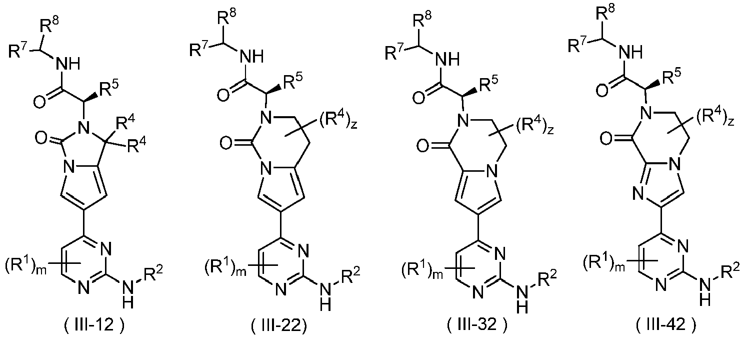 wherein:
R⁵ is selected from the group consisting of halogen, alkyl, alkoxy, haloalkyl, haloalkoxy, hydroxy, hydroxyalkyl, cyano, amino, nitro, cycloalkyl, heterocyclyl, aryl and heteroaryl;
R⁸ is selected from the group consisting of alkyl, alkoxy, oxo, halogen, amino, cyano, nitro, hydroxy, hydroxyalkyl, aminoalkyl, cycloalkyl, heterocyclyl, aryl and heteroaryl;
z is 0, 1, 2, 3 or 4; and
R¹, R², R⁴, R⁷ and m are as defined in claim 1.

9. The compound of formula (I) or the stereisomer, tautomer, mesomer, racemate, enantiomer, diastereomer thereof, or mixture thereof, or the pharmaceutically acceptable salt thereof according to any one of claims 1 to 8, wherein R⁷ is an aryl, wherein the aryl is optionally further substituted by one or more substituents selected from the group consisting of alkyl, alkoxy, oxo, halogen, amino, cyano, nitro, hydroxy and hydroxyalkyl; preferably, R⁷ is a phenyl, wherein the phenyl is optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

10. The compound of formula (I) or the stereisomer, tautomer, mesomer, racemate, enantiomer, diastereomer thereof, or mixture thereof, or the pharmaceutically acceptable salt thereof according to any one of claims 1 to 9, wherein R⁸ is selected from the group consisting of alkyl, alkoxy, oxo, halogen, amino, cyano, nitro, hydroxy, hydroxyalkyl and aminoalkyl; preferably, R⁸ is C₁₋₆ hydroxyalkyl or aminoC₁₋₆ alkyl.

11. The compound of formula (I) or the stereisomer, tautomer, mesomer, racemate, enantiomer, diastereomer thereof, or mixture thereof, or the pharmaceutically acceptable salt thereof according to any one of claims 1 to 10, wherein R⁵ is selected from the group consisting of hydrogen atom, halogen, alkyl, alkoxy, haloalkyl, haloalkoxy, hydroxy and hydroxyalkyl, preferably hydrogen atom, halogen or alkyl, and more preferably C₁₋₆ alkyl.

12. The compound of formula (I) or the stereisomer, tautomer, mesomer, racemate, enantiomer, diastereomer thereof, or mixture thereof, or the pharmaceutically acceptable salt thereof according to any one of claims 1 to 11, wherein R² is selected from the group consisting of C₁₋₆ alkyl, 3 to 8 membered heterocyclyl and 5 to 10 membered heteroaryl, wherein the C₁₋₆ alkyl, 3 to 8 membered heterocyclyl and 5 to 10 membered heteroaryl are each optionally further substituted by one or more substituents selected from the group consisting of C₁₋₆ alkyl, C₁₋₆ alkoxy, oxo, halogen, amino, cyano, nitro, hydroxy and C₁₋₆ hydroxyalkyl.

13. The compound of formula (I) or the stereisomer, tautomer, mesomer, racemate, enantiomer, diastereomer thereof, or mixture thereof, or the pharmaceutically acceptable salt thereof according to any one of claims 1 to 11, wherein R² is a heterocyclyl, wherein the heterocyclyl is optionally further substituted by one or more substituents selected from the group consisting of alkyl, alkoxy, oxo, halogen, amino, cyano, nitro, hydroxy and hydroxyalkyl.

14. The compound of formula (I) or the stereisomer, tautomer, mesomer, racemate, enantiomer, diastereomer thereof, or mixture thereof, or the pharmaceutically acceptable salt thereof according to any one of claims 1 to 11, wherein R² is a heteroaryl, wherein the heteroaryl is optionally further substituted by one or more substituents selected from the group consisting of alkyl, alkoxy, oxo, halogen, amino, cyano, nitro, hydroxy and hydroxyalkyl.

15. The compound of formula (I) or the stereisomer, tautomer, mesomer, racemate, enantiomer, diastereomer thereof, or mixture thereof, or the pharmaceutically acceptable salt thereof according to any one of claims 1 to 14, wherein R¹ is selected from the group consisting of hydrogen atom, halogen, alkyl, alkoxy, haloalkyl, haloalkoxy, hydroxy and hydroxyalkyl, preferably selected from the group consisting of hydrogen atom, halogen and C₁₋₆ alkyl, and more preferably halogen.

16. The compound of formula (I) or the stereisomer, tautomer, mesomer, racemate, enantiomer, diastereomer thereof, or mixture thereof, or the pharmaceutically acceptable salt thereof according to any one of claims 1 to 15, wherein R⁴ is selected from the group consisting of hydrogen atom, halogen, alkyl, alkoxy, haloalkyl, haloalkoxy, hydroxy and hydroxyalkyl, preferably hydrogen atom or alkyl, and more preferably hydrogen atom or C₁₋₆ alkyl.

17. The compound of formula (I) or the tautomer, mesomer, racemate, enantiomer, diastereomer thereof, or mixture thereof, or the pharmaceutically acceptable salt thereof according to any one of claims 1 to 16, selected from the group consisting of:

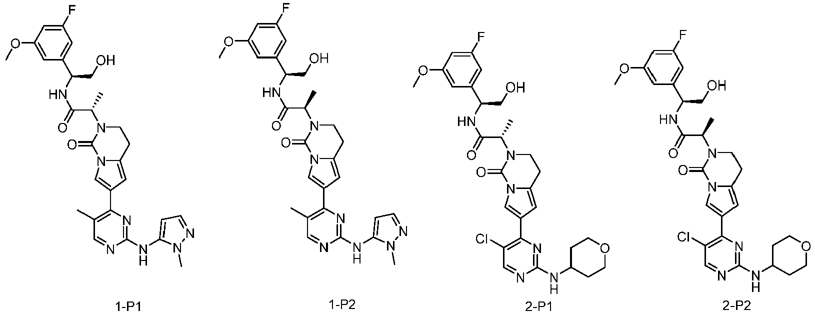

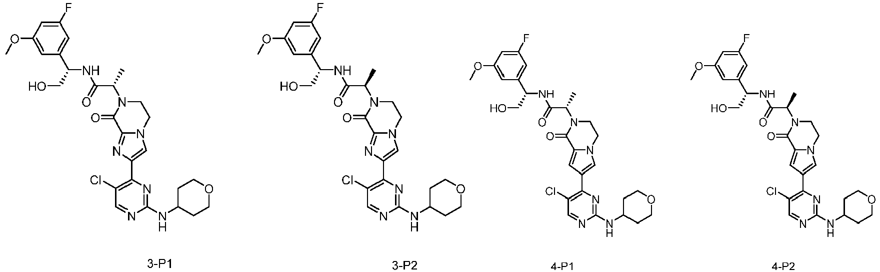

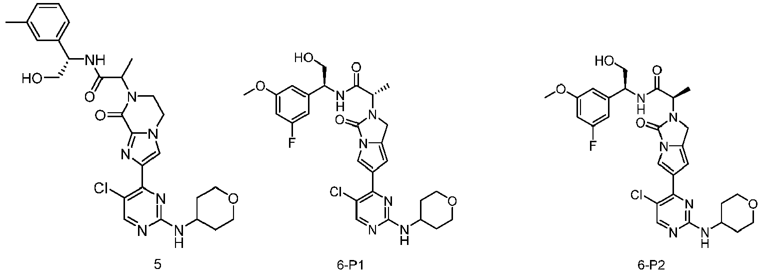

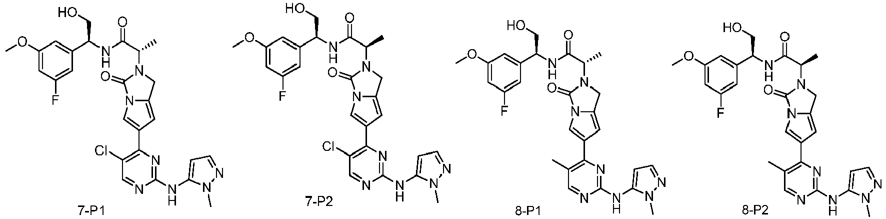

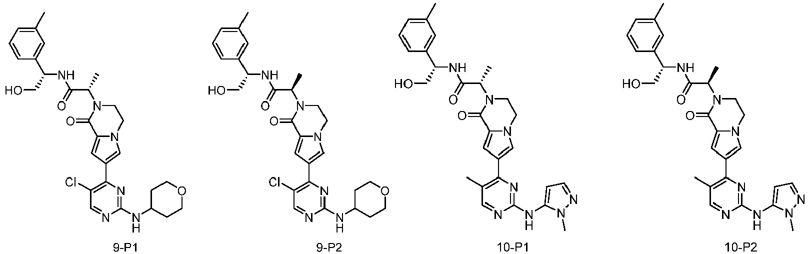

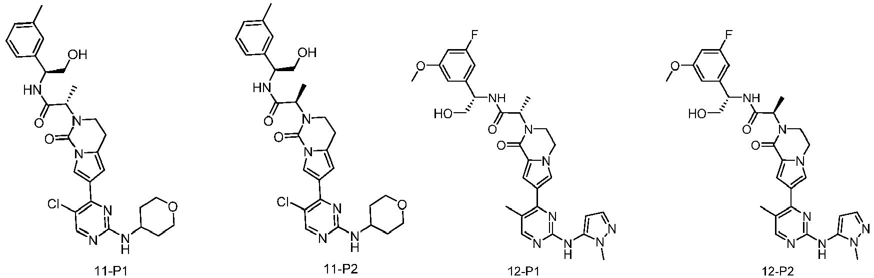

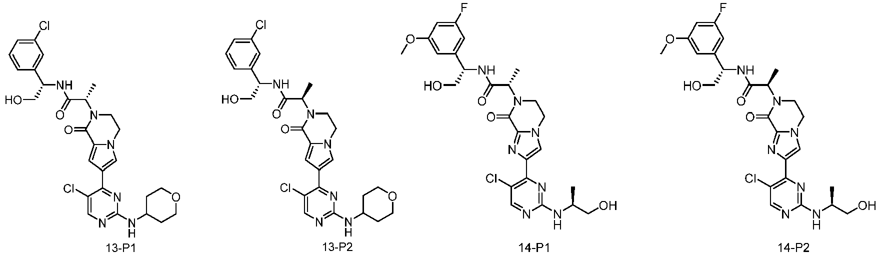

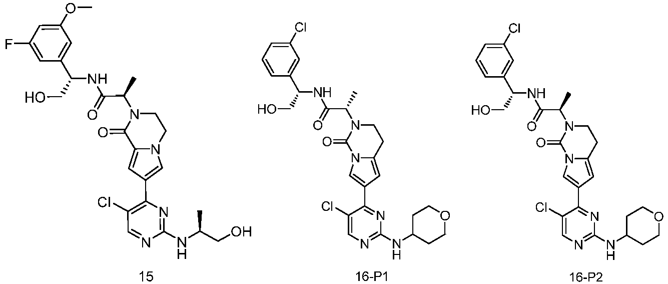

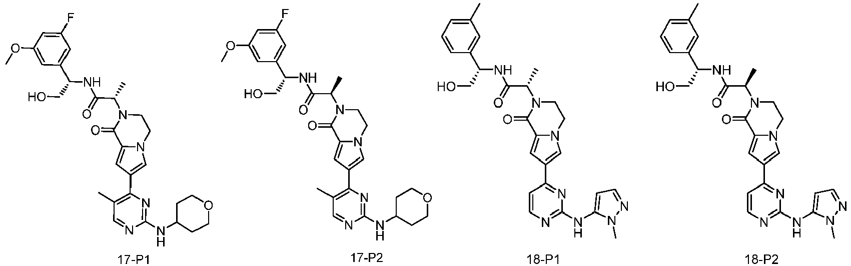

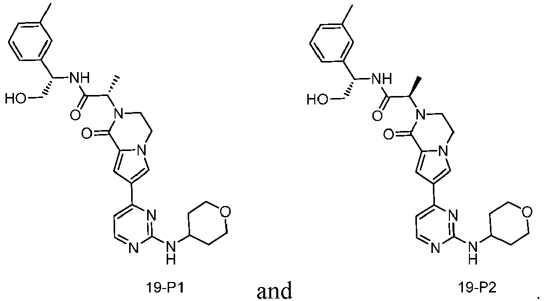

18. A compound of formula (IA) or a tautomer, mesomer, racemate, enantiomer, diastereomer thereof, or mixture thereof, or a pharmaceutically acceptable salt thereof: 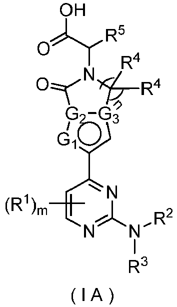 wherein:
G₁, G₂ and G₃ are identical or different and are each independently selected from the group consisting of CH, C and N;
each R¹ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R² is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl, wherein the alkyl, cycloalkyl, heterocyclyl, aryl and heteroaryl are each optionally further substituted by one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R³ is selected from the group consisting of hydrogen atom, halogen, alkyl, alkoxy, haloalkyl, haloalkoxy, hydroxy, hydroxyalkyl, cyano, amino, nitro, cycloalkyl and heterocyclyl;
each R⁴ is identical or different, and each is independently selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
R⁵ is selected from the group consisting of hydrogen atom, halogen, alkyl, alkoxy, haloalkyl, haloalkoxy, hydroxy, hydroxyalkyl, cyano, amino, nitro, cycloalkyl, heterocyclyl, aryl and heteroaryl;
n is 1, 2 or 3; and
m is 0, 1 or 2.

19. The compound of formula (IA) or the tautomer, mesomer, racemate, enantiomer, diastereomer thereof, or mixture thereof, or the pharmaceutically acceptable salt thereof according to claim 18, selected from the group consisting of: 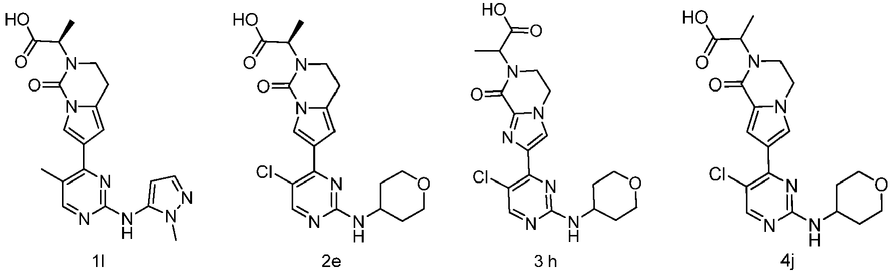 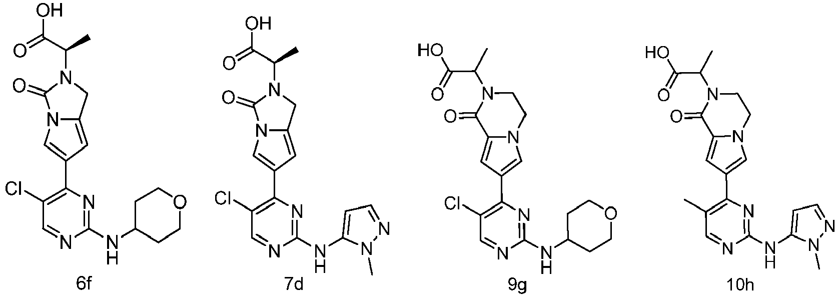 and

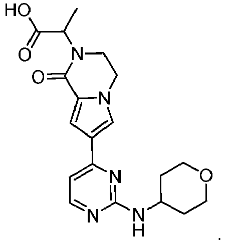

20. A method for preparing the compound of formula (I) or the tautomer, mesomer, racemate, enantiomer, diastereomer thereof, or mixture thereof, or the pharmaceutically acceptable salt thereof according to claim 1, comprising a step of: 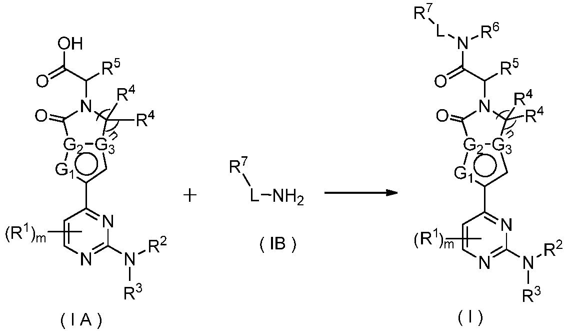 subjecting a compound of formula (IA) and a compound of formula (IB) to a condensation reaction under an alkaline condition to obtain the compound of formula (I) or the tautomer, mesomer, racemate, enantiomer, diastereomer thereof, or mixture thereof, or the pharmaceutically acceptable salt thereof, wherein the alkaline condition is preferably *N,N*-diisopropylethylamine; R⁶ is a hydrogen atom; and G₁, G₂, G₃, R¹ to R⁵, R⁷, L, m and n are as defined in claim 1.

21. A pharmaceutical composition comprising the compound of formula (I) or the tautomer, mesomer, racemate, enantiomer, diastereomer thereof, or mixture thereof, or the pharmaceutically acceptable salt thereof according to any one of claims 1 to 17, and one or more pharmaceutically acceptable carriers, diluents or excipients.

22. Use of the compound of formula (I) or the stereisomer, tautomer, mesomer, racemate, enantiomer, diastereomer thereof, or mixture thereof, or the pharmaceutically acceptable salt thereof according to any one of claims 1 to 17 or the pharmaceutical composition according to claim 21 in the preparation of a medicament for inhibiting ERK

23. Use of the compound of formula (I) or the stereisomer, tautomer, mesomer, racemate, enantiomer, diastereomer thereof, or mixture thereof, or the pharmaceutically acceptable salt thereof according to any one of claims 1 to 17 or the pharmaceutical composition according to claim 21 in the preparation of a medicament for treating or preventing cancer, inflammation, or other proliferative diseases, preferably cancer; wherein the cancer is selected from the group consisting of melanoma, liver cancer, kidney cancer, lung cancer, nasopharyngeal cancer, colorectal cancer, colon cancer, rectal cancer, pancreatic cancer, cervical cancer, ovarian cancer, breast cancer, bladder cancer, prostate cancer, leukemia, head and neck squamous cell carcinoma, carcinoma of uterine cervix, thyroid cancer, lymphoma, sarcoma, neuroblastoma, brain tumor, myeloma, astrocytoma and glioma.
